**Europäisches Patentamt**

**European Patent Office**

**Office européen des brevets**

(19)

(11)  **EP 1 026 240 A2**

(12)  **EUROPEAN PATENT APPLICATION**

(43) Date of publication:
    **09.08.2000  Bulletin 2000/32**

(51) Int. Cl.⁷: **C12N 15/10**,  C07K 1/107,
    C12N 15/62

(21) Application number: **00106289.2**

(22) Date of filing: **01.09.1989**

(84) Designated Contracting States:
    **AT BE CH DE FR GB IT LI LU NL SE**

(30) Priority: **02.09.1988 US 240160**

(62) Document number(s) of the earlier application(s) in
    accordance with Art. 76 EPC:
    **96112867.5 / 0 768 377**
    **89910702.3 / 0 436 597**

(71) Applicant: **Dyax Corp.**
    **Cambridge, MA 02139 (US)**

(72) Inventors:
    • **Ladner, Robert Charles**
      **Ijamsville, MD 21754 (US)**

    • **Gutermann, Sonia K.**
      **Belmont, MA 02178 (US)**

(74) Representative:
    **Plougmann, Vingtoft & Partners A/S**
    **Sankt Annae Plads 11,**
    **P.O. Box 3007**
    **1021 Copenhagen K (DK)**

Remarks:
    This application was filed on 23 - 03 - 2000 as a
    divisional application to the application mentioned
    under INID code 62.

(54)    **Generation and selection of recombinant varied binding proteins**

(57)    In order to obtain a novel binding protein against a chosen target, DNA molecules, each encoding a protein comprising one of a family of similar potential binding domains and a structural signal calling for the display of the protein on the outer surface of a chosen bacterial cell, bacterial spore or phage (genetic package) are introduced into a genetic package. The protein is expressed and the potential binding domain is displayed on the outer surface of the package. The cells or viruses bearing the binding domains which recognize the target molecule are isolated and amplified. The successful binding domains are then characterized. One or more of these successful binding domains is used as a model for the design of a new family of potential binding domains, and the process is repeated until a novel binding domain having a desired affinity for the target molecule is obtained. In one embodiment, the first family of potential binding domains is related to bovine pancreatic trypsin inhibitor, the genetic package is M 13 phage, and the protein includes the outer surface transport signal of the M 13 gene III protein.

FIG. 1.

EP 1 026 240 A2

**Description**

Field of the Invention

This, invention relates to development of novel binding proteins by an iterative process of mutagenesis, expression, chromatographic selection, and amplification.

Information Disclosure Statement

The amino acid sequence of a protein determines its three-dimensional (3D) structure, which in turn determines protein functioning (EPST63, ANFI73). The system of classification of protein structure of Schulz and Schirmer (SCHU79, ch 5) is adopted herein.

The 3D structure of a protein is essentially unaffected by the identity of the amino acids at some loci; at other loci only one or a few types of amino acid is allowed (SHOR8S, EISE8S, REID88). Generally, loci where wide variety is allowed have the amino acid side group directed toward the solvent. While limited variety is allowed where the side group is directed toward other parts of the protein. (See also SCHU79, p169-171 and CREI84, p239-245, 314-315).

The secondary structure (helices, sheets, turns, loops) of a protein is determined mostly by local sequence. Certain amino acids tend to be correlated with certain secondary structures and the commonly used Chou-Fasman (CHOU74, CHOU78a, CHOU78b) rules depend on these correlations. However, every amino acid type has been observed in helices and in both parallel and antiparallel sheets. Pentapeptides of identical sequence are found in different proteins; in some cases the conformations of the pentapeptides are very different (KABS84, ARGO87).

Turns and loops tolerate insertions and deletions more readily than do other secondary structures (RICH81, THOR88, SUTC87a); related proteins differ most in loops and turns.

Changing three residues in subtilisin from Bacillus amyloliquefaciens to be the same as the corresponding residues in subtilisin from B. licheniformis produced a protease that had nearly the same activity as the subtilisin from the latter organism; 82 differences remained in the sequences. The three residues changed were chosen because they were the only differences within 7 Angstroms (A) of the active site (WELL87a).

Schulz and Schirmer summarize many observations on the binding of proteins to other molecules (SCHU79, p98-105). For example, haemoglobin alpha chains bind very tightly to haemoglobin beta chains (delta G more negative than -11.0 Kcal/mole); antibodies bind tightly to antigens ($K_d$s range from $10^{-6}$ to $10^{-14}$ M, $K_d$ is the dissociation constant equal to [A][B]/[A:B]); basic bovine pancreatic trypsin inhibitor (BPTI) binds tightly to trypsin ($K_d$ = 6.0 x $10^{-14}$ M (TSCH87), delta G = -18.0 Kcal/mole); and avidin binds to biotin ($K_d$ = 1.3 x $10^{-15}$ M (CREI84, p362)). In each case the binding results from complementarity of the surfaces that come into contact: bumps fit into holes, unlike charges come together, dipoles align, and hydrophobic atoms contact other hydrophobic atoms. Although bulk water is excluded, individual water molecules are frequently found filling space, in intermolecular interfaces; these waters usually form hydrogen bonds to one or more atoms of the protein or to other bound water.

The factors affecting protein binding are known, (CHOT75, CHOT76, SCHU79, p98-107, and CREI84, Ch8), but designing new complementary surfaces has proved difficult. Although some rules have been developed for substituting side groups (SUTC87b), the side groups of proteins are floppy and it is difficult to predict what conformation a new side group will take. Further, the forces that bind proteins to other molecules are all relatively weak and it is difficult to predict the effects of these forces. Hence, it is difficult to design superior binding proteins based on theory alone (QUIO87).

Enzyme-substrate affinity, however, has fortuitously been increased by protein engineering (WILK84). A point mutant of tyrosyl tRNA synthetase of Bacillus stearothermophilus exhibits a 100-fold increase in affinity for ATP. Substitution of one amino acid for another at a surface locus may profoundly alter binding properties of the protein other than substrate binding, without affecting the tertiary structure of the protein. For example, in sickle-cell haemoglobin the change of the surface residue E6 to V in the beta chains causes deoxyhaemoglobin-S to form fibers through self binding (DICK83, p125-145); the tertiary and quaternary structure of the haemoglobin are not changed (PADL85, WISH75, WISH76).

Changing a single amino acid in BPTI greatly reduces its binding to trypsin, but some of the new molecules retain the parental characteristics of binding to and inhibiting chymotrypsin, while others exhibit new binding to elastase (TANK77; TSCH87). Changes of single amino acids on the surface of the lambda Cro repressor greatly reduce its affinity for the natural operator Or3, but greatly increase the binding of the mutant protein to a mutant operator (EISE85). Thus changing the surface of a binding protein may alter its specificity without abolishing binding activity.

The recently developed techniques of "reverse genetics" have been used to produce single specific mutations at precise base pair loci (OLIP86, OLIP87, and AUSU87). Mutations are generally detected by sequencing and in some cases by loss of wild-type function. These procedures allow researchers to analyze the function of each residue in a protein (MILL88) or of each base pair in a regulatory DNA sequence (CHEN88). In these analyses, the norm has been to strive for the classical goal of obtaining mutants carrying a single alteration (AUSU87).

Reverse genetics is often applied to coding regions to determine which residues are most important to protein structure and function; isolation of a single mutant at each residue of the protein gives an initial estimate of which residues play crucial roles.

Prior to the method of the present invention, two general approaches have been developed to create novel mutant proteins through reverse genetics. In one approach, dubbed "protein surgery" (DILL87), a specific substitution is introduced at a single protein residue to determine the effects on structure and function of specific substitutions (CRAI85) (RAOS87) (BASH87). However, many desirable protein alterations require multiple amino acid substitutions and thus are not accessible through single base changes or even through all possible amino acid substitutions at any one residue.

The other approach has been randomly to generate a variety of mutants at many loci within a cloned gene using mutagenic chemicals or radiation. The specific location and nature of the change are determined by DNA sequencing. (PAKU86) This approach is limited by the number of colonies that can be examined. Also, it does not take advantage of any knowledge of the protein structure and its relationship to binding activity.

Progress toward rules governing substitutions of amino acids (ULME83) has been greatly hampered by the extensive efforts involved in using either method and the practical limitations on the number of colonies that can be inspected (ROBE86).

The term "saturation mutagenesis" with reference to synthetic DNA is generally taken to mean generation of a population in which: a) every possible single-base change within a fragment of a gene of DNA regulatory region is represented, and b) most mutant genes contain only one mutation. Thus a set of all possible single mutations for a 6 base pair length of DNA comprises a population of 18 mutants. Oliphant et al. (OLIP86) and Oliphant and struhl (OLIP87) have demonstrated ligation and cloning of highly degenerate oligonucleotides and have applied saturation mutagenesis to the study of promoter sequence and function. They suggest that similar methods could be used to study genetic expression of proteins, but they do not say how to: a) choose protein residues to vary, or b) select or screen mutants with desirable properties.

Reidhaar-Olson and Sauer (REID88) have used synthetic degenerate oligo-nts to vary simultaneously two or three residues through all twenty amino acids in the dimer interface of cl repressor from bacteriophage lambda. They give no discussion of the limits on how many residues could be varied at once nor do they mention the problem of unequal abundance of DNA encoding different amino acids. They looked for proteins that either had wild-type dimerization or that did not dimerize. They did not seek proteins having novel binding properties and did not report any.

Several researchers have designed and synthesized proteins de novo. These designed proteins are small and most have been synthesized in vitro as polypeptides rather than genetically. Gutte and colleagues have made a polypeptide that binds DDT in 55% ethanol (MOSE83). Recently Moser et al. (MOSE87) reported genetic expression in E. coli both of the designed 24 residue DDT-binding protein and of fusions of the DDT-binding sequence to LacZ. They state that design of biologically active proteins is currently impossible.

Erickson et al. (ERIC86) have designed and synthesized a series of proteins that they have named betabellins, that are meant to have beta sheets. They suggest use of polypeptide synthesis with mixed reagents to produce several hundred analogous betabellins, and use of a column to recover analogues with high affinity for a chosen target compound bound to the column. They envision successive rounds of mixed synthesis of variant proteins and purification by specific binding. They do not discuss how residues should be chosen for variation. Because proteins cannot be amplified, the researchers must sequence the recovered protein to learn which substitutions improve binding. The researchers must limit the level of diversity so that each variety of protein will be present in sufficient quantity for the isolated fraction to be sequenced.

Methods have been developed to separate cells through their affinity to various substances. Methods applied to animal cells reveal common problems: a) non-specific interactions between cells and affinity supports, and b) irreversible binding of cells to affinity matrices (BONN85).

Ferenci and collaborators have published a series of papers on the chromatographic isolation of mutants of the maltose-transport protein LamB of E. coli (WAND79, FERE80a, FERE80b, FERE80c, FERE82a, FERE82b, FERE83, CLUN84, FERE86a, FERE86b, FERE86c, FERE87a, FERE87b, HEIN87, and HEIN88). The papers report that spontaneous and induced mutants at the lamB genetic locus can be isolated by chromatography over a column supporting immobilized maltose, maltodextrins, or starch. The reports speculate that other applications are possible, but specifically mention only the elucidation of the residues responsible for the selectivity of the maltodextrin pore or similar pore proteins. The mutant proteins were non-chimeric, and no attempt was made to obtain binding to a new target.

Both FERE86a and CLUN84 point up the difficulties of working with live bacteria that can metabolize chemicals and change their physiological behavior during the chromatographic experiment.

A fragment of a heterologous gene can be introduced into bacteriophage F1 gene III (SMIT85). If the inserted gene preserves the original reading frame, expression of the altered gene III causes an inserted domain to appear in the gene III protein. The resulting strain of f1 virions are adsorbed by an antibody against the protein encoded by the heterologous DNA. The phage were eluted at pH 2.2 and retained some infectivity. However, the single copy of f1 gene III was used for insertion of the heterologous gene so that all copies of gene III protein were affected; infectivity of the resultant phage was reduced 25-fold.

Smith presented his method as a way to isolate cloned genes using antibodies to the gene products. He made no mention of mutagenizing the inserted genetic material or of inducing novel binding properties in the inserted protein domain.

A fragment of the repeat region of the circumsporozoite protein from Plasmodium falciparum has been expressed on the surface of M13 as an insert in the gene III protein (CRUZ88). The recombinant phage were both antigenic and immunogenic in rabbits. The authors do not suggest mutagenesis of the inserted material.

Gene fragments coding for hepatitis B virus antigens have been fused to fragments of lamB, and if the fusion is in a region coding for exposed domains of LamB, the HBV antigens appear on the cell surface and are immunogenic (CHAR87). Charbit et al. (CHAR87) suggest use of these engineered strains for development of a live bacterial vaccine; they did not suggest mutagenesis of the fused heterologous gene fragments, nor development of binding capabilities.

Ladner, US Patent No. 4,704,692, "Computer Based System and Method for Determining and Displaying Possible Chemical Structures for Converting Double- or Multiple-Chain Polypeptides to Single-Chain Polypeptides" describes a design method for converting proteins composed of two or more chains into proteins of fewer polypeptide chains, but with essentially the same 3D structure. There is no mention of variegated DNA and no genetic selection. Ladner and Bird, WO88/01649 (Publ. March 10, 1988) disclose the specific application of computerized design of linker peptides to the preparation of single chain antibodies.

Ladner, Glick and Bird, WO88/06630 (publ. 7 Sept. 1988) (LGB) speculate that diverse single chain antibody domains may be screened for binding to a particular antigen by varying the DNA encoding the combining determining regions of a single chain antibody, subcloning the SCAD gene into the gpV gene of phage lambda so that a SCAD/gpV chimera is displayed on the outer surface of the phage, and selecting phage which bind to the antigen through affinity chromatography. The only antigen mentioned is bovine growth hormone. No other binding molecules, targets, carrier organisms, or outer surface proteins are discussed. Nor is there any mention of the method or degree of mutagenesis.

Ladner and Bird, WO88/06601 (publ. 7 September 1988) suggest that single chain "psuedodimeric" repressors (DNA-binding proteins) may be prepared by mutating a putative linker peptide followed by in vivo selection that mutation and selection may be used to create a dictionary of recognition elements for use in the design of asymmetric repressors. The repressors are not displayed on the outer surface of an organism.

No admission is made that any cited reference is prior art or pertinent prior art, and the dates given are those appearing on the reference and may not be identical to the actual publication date.

## SUMMARY OF THE INVENTION

This invention relates to the construction, expression, and selection of mutated genes that specify novel proteins with desirable binding properties, as well as these proteins themselves. The substances bound by these proteins, hereinafter referred to as "targets", may be, but need not be, proteins. Targets may include other biological or synthetic macromolecules as well as organic and inorganic molecules.

The novel binding proteins may be obtained: 1) by mutating a gene encoding a known binding protein within the subsequence encoding a known binding domain, or 2) by taking such a subsequence of the gene for a first protein and combining it with all or part of a gene for a second protein (which may or may not be itself a known binding protein), 3) by mutating a gene encoding a protein which, while not possessing a known binding activity, possesses a secondary or higher structure that lends itself to binding activity (clefts, grooves, etc.), or 4) by mutating a gene encoding a known binding protein but not in the subsequence known to cause the binding. The protein from which the novel binding protein is derived need not have any specific affinity for the target material.

In one embodiment, the invention relates to:

a) preparing a variegated population of replicable genetic packages, each package including a nucleic acid construct coding on expression for an outer-surface-displayed potential binding protein comprising (i) a structural signal directing the display of the protein on the outer surface of the package and (ii) a potential binding domain for binding said target, where a plurality of different potential binding domains are displayed by the individual packages,

b) causing the expression of said protein and the display of said protein on the outer surface of such packages,

c) contacting the packages with target material so that the potential binding domains of the proteins and the target material may interact, and separating packages bearing a potential binding domain that succeeds in binding the target material from packages that do not so bind,

d) recovering and replicating at least one package bearing a successful binding domain,

(e) determining the amino acid sequence of the successful binding domain of a genetic package which bound to the target material,

(f) preparing a new variegated population of replicable genetic packages according to step (a), the parental potential binding domain for the potential binding domains of said new packages being a successful binding domain whose sequence was determined in step (e), and repeating steps (b)-(e) with said new population, and, when a package bearing a binding domain of desired binding characteristics is obtained,

(g) abstracting the gene encoding the desired binding domain from the genetic package and placing it into a suitable expression system. (The binding domain may then be expressed as a unitary protein, or as a domain of a larger protein).

The invention further relates to a method of preparing a mixed population of replicable genetic packages in which each package includes a gene expressing a potential binding protein in such a manner that the protein is presented on the outer surface of the package. This method comprises:

i) preparing a variegated population of DNA inserts of each of which comprises a first sequence which codes on expression for a potential binding domain and, a second sequence encoding signal directing that the encoded protein be displayed on the outer surface of a chosen replicable genetic package, and

ii) incorporating the resulting population of DNA constructs into the chosen replicable genetic packages to produce a population of replicable genetic packages.

In a preferred embodiment, the potential-binding-protein-encoding inserts are incorporated into a gene encoding an outer-surface protein of the replicable genetic package.
The invention encompasses the design and synthesis of variegated DNA encoding a family of potential binding proteins characterized by constant and variable regions, said proteins being designed with a view toward obtaining a protein that binds a predetermined target.
For the purposes of this invention, the term "potential binding protein" refers to a protein encoded by one species of DNA molecule in a population of variegated DNA wherein the region of variation appears in one or more subsequences encoding one or more segments of the polypeptide having the potential of serving as a binding domain for the target substance.
From time to time, it may be helpful to speak of the "parent sequence" of the variegated DNA. When the novel binding domain sought is an analogue of a known binding domain, the parent sequence is the sequence that encodes the known binding domain. The variegated DNA will be identical with this parent sequence at most loci, but will diverge from it at chosen loci. When a potential binding domain is designed from first principles, the parent sequence is a sequence which encodes the amino acid sequence that has been predicted to form the desired binding domain, and the variegated DNA is a population of "daughter DNAs" that are related to that parent by a high degree of sequence similarity.
The fundamental principle of the invention is one of forced evolution. The efficiency of the forced evolution is greatly enhanced by careful choice of which residues are to be varied. The 3D structure of the potential binding domain is a key determinant in this choice. First a set of residues that can simultaneously contact one molecule of the target is identified. Then all or some of the codons encoding these residues are varied simultaneously to produce a variegated population of DNA. The variegated population of DNA is used to transform cells so that a variegated population of genetic packages is produced.
The mixed population of genetic packages containing genes encoding possible binding proteins is enriched for packages containing genes that express proteins that in fact bind to the target ("successful binding domains"). After one or more rounds of such enrichment, one or more of the chosen genes are examined and sequenced. If desired, new loci of variation are chosen. The selected daughter genes of one generation then become the parent sequences for the next generation of variegated DNA, beginning the next "variegation cycle." Such cycles are continued until a protein with the desired target affinity is obtained.
The appended claims are hereby incorporated by reference into this specification as an enumeration of the preferred embodiments.

BRIEF DESCRIPTION OF THE DRAWINGS

Figure 1 is a schematic showing the relationships between various types of Binding Domains (BD).

Figure 2 is a flow chart showing the major steps used to create a novel protein with affinity for a predetermined target.

Figure 3 is a schematic of a PBD contacting a molecule of target material.

Figure 4 is a schematic of the construction of pLG3 from M13mp18 and pBR322.

Figure 5 is a schematic of the construction of pLG7 from pLG3 and synthetic DNA.

DETAILED DESCRIPTION OF THE INVENTION

Sec. 0.1: Overview:

The present invention separates mutated genes that specify novel proteins with desirable binding properties from closely related genes that specify proteins with no or undesirable binding properties, by: 1) arranging that the product of each mutated gene be displayed on the outer surface of a replicable genetic package that contains the gene, and 2) using affinity separation incorporating a desirable target material to enrich the population of packages for those packages containing genes specifying proteins with improved binding to that target material.

Let $K_D(x,y)$ be a dissociation constant,

$$K_D(x,y) = \frac{[x]\,[y]}{[x:y]}.$$

For the purposes of the appended claims, a protein P is a binding protein if

(1) for one molecular, ionic or atomic species A, the dissociation constant $K_D(P,A) < 10^{-6}$ moles/liter, and

(2) for a different molecular, ionic or atomic species B, $K_D(P,B) > 10^{-1}$ moles/liter.

As a result of these two conditions, the protein P exhibits specificity for A over B, and a minimum degree of affinity (or avidity) for A.

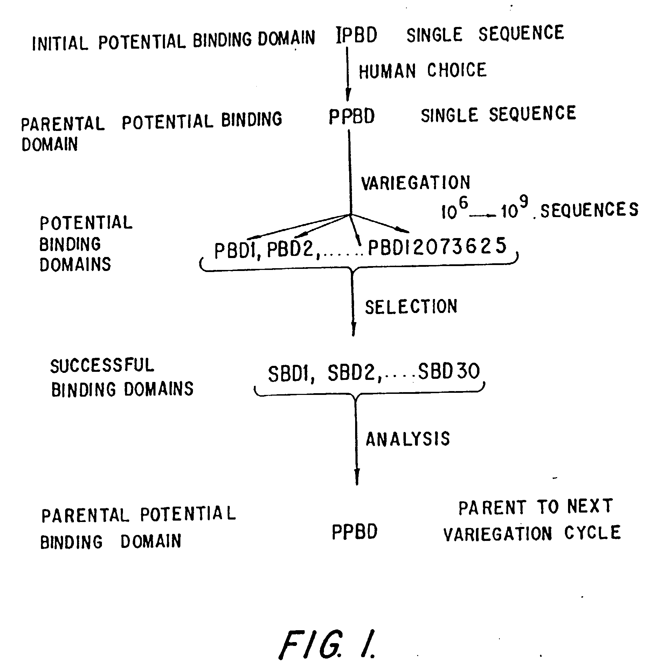

When a domain of a protein is primarily responsible for the protein's ability to specifically bind a chosen target, it is referred to herein as a "binding domain" (BD). We engineer the appearance of a stable protein domain, denoted as an "initial potential binding domain" (IPBD), on the surface of a genetic package. The present invention is concerned with the expression of numerous, diverse, variant "potential binding domains" (PBD), all related to a "parental potential binding domain" (PPBD) such as the binding domain of a known binding protein, and with selection and amplification of the genes encoding the most successful mutant PBDs. An IPBD is chosen as PPBD to the first round of variegation. Selection-through-binding isolates one or more "successful binding domains" (SBD). An SBD from one round of variegation and selection-through-binding is chosen to be the PPBD for the next round. The invention is not, however, limited to proteins with a single BD since the method may be applied to any or all of the BDs of the protein, sequentially or simultaneously. The relationships of the various BDs are illustrated in Figure 1.

The term "variegated DNA" refers to a population of molecules that have the same base sequence through most of their length, but that vary at a limited number of defined loci, preferably 5-10 codons. A molecule of variegated DNA can be introduced into a plasmid so that it constitutes part of a gene (OLIP86, OLIP87, AUSU87, REID88). When plasmids containing variegated DNA are used to transform bacteria, each cell makes a version of the original protein. Each colony of bacteria may produce a different version from any other colony. If the variegations of the DNA are concentrated at loci known to be on the surface of the protein or in a loop, a population of proteins will be generated, many members of which will fold into roughly the same 3D structure as the parent protein. The specific binding properties of each member, however, may be different from each other member. It remains to sort out the colonies containing genes for proteins with desirable binding properties from those that do not exhibit the desired affinities.

A "single-chain antibody" is a single chain polypeptide comprising at least 200 amino acids, said amino acids forming two antigen-binding regions connected by a peptide linker that allows the two regions to fold together to bind the antigen. Either the two antigen-binding regions must be variable domains of <u>known</u> antibodies, or they must (1) each fold into a beta barrel of nine strands that are spatially related in the same way as are the nine strands of known antibody variable light or heavy domains, and (2) fit together in the same way as do the variable domains of said known antibody. Generally speaking, this will require that, with the exception of the amino acids corresponding to the hypervariable region, there is at least 88% homology with the amino acids of the variable domain of a known antibody.

The term "affinity separation means" includes, but is not limited to: a) affinity column chromatography, b) batch elution from an affinity matrix material, c) batch elution from an affinity material attached to a plate, d) fluorescence activated cell sorting, and e) electrophoresis in the presence of target material. "Affinity material" is used to mean a material with affinity for the material to be purified, called the "analyte". In most cases, the association of the affinity material and the analyte is reversible so that the analyte can be freed from the affinity material once the impurities are washed away.

Affinity column chromatography, batch elution from an affinity matrix material held in some container, and batch elution from a plate are very similar and hereinafter will be treated under "affinity chromatography."

Fluorescent-activated cell sorting involves use of an affinity material that is fluorescent <u>per se</u> or is labeled with a fluorescent molecule. Current commercially available cell sorters require 800 to 1000 molecules of fluorescent dye, such as Texas red, bound to each cell. FACS can sort $10^3$ cells or viruses/sec.

Electrophoretic affinity separation involves electrophoresis of viruses or cells in the presence of target material, wherein the binding of said target material changes the net charge of the virus particles or cells. It has been used to separate bacteriophages on the basis of charge. (SERW87).

The present invention makes use of affinity separation of bacterial cells, or bacterial viruses (or other genetic packages) to enrich a population for those cells or viruses carrying genes that code for proteins with desirable binding properties.

In the present invention, the words "select" and "selection" are used exclusively in the genetic sense; <u>i.e.</u> a biological process whereby a phenotypic characteristic is used to enrich a population for those organisms displaying the desired phenotype.

The process of the present invention comprises three major parts:

I. design and production of a replicable genetic package (GP) that displays an IPBD on the surface of the GP, denoted GP(IPBD),

II. design and implementation of an affinity separation process that separates GP(IPBD)s that bind to a known affinity molecule from wild-type GPs or GP(IPBD⁻)s, neither of which binds the known affinity molecule, and

III. design and implementation of a genetic variegation method, denoted structure-directed mutagenesis, wherein a population of $10^6$ or more different GP(PBD)s, denoted GP(vgPBD), is produced.

One affinity separation is called a "separation cycle"; one pass of variegation followed by as many separation cycles as are needed to isolate an SBD, is called a "variegation cycle". The amino acid sequence of one SBD from one round becomes the PPBD to the next variegation cycle. We perform variegation cycles iteratively until the desired affinity and specificity of binding between an SBD and chosen target are achieved.

Part I is a strain construction in which we deal with a single IPBD sequence. Variability may be introduced into DNA subsequences adjacent to the <u>ipbd</u> subsequence and within the <u>osp-ipbd</u> gene so that the IPBD will appear on the GP surface. A molecule, such as an antibody, having high affinity for correctly folded IPBD is used to: a) detect IPBD on the GP surface, b) screen colonies for display of IPBD on the GP surface, or c) select GPs that display IPBD from a population, some members of which might display IPBD on the GP surface. In one preferred embodiment, Part I of the process involves:

1) choosing a GP such as a bacterial cell (Sec. 1.1.1), bacterial spore (1.2.1), or phage (1.3.1), having a suitable outer surface protein (Secs. 1.1.3, 1.2.3, and 1.3.3),

2) choosing a stable IPBD (Sec. 2),

3) designing an amino acid sequence that: a) includes the IPBD as a subsequence and b) will cause the IPBD to appear on the GP surface (Secs. 1.1.2, 1.2.2, 1.3.2, and 4),

4) engineering a gene, denoted osp-ipbd, that: a) codes for the designed animo acid sequence, b) provides the necessary genetic regulation, and c) introduces convenient sites for genetic manipulation (Secs. 4.1, 4.2, 4.3, 5.1, and 5.2),

5) cloning the osp-ipbd gene into the GP (Sec. 6.1), and

6) harvesting the transformed GPs (Sec. 7) and testing them for presence of IPBD on the GP surface (Sec. 8); this test is performed with an affinity molecule having high affinity for IPBD, denoted AfM(IPBD).

In another preferred embodiment, Part I of the process involves:

1) and 2) as above

3) designing a DNA sequence that: a) encodes the IPBD as a subsequence and b) contains suitable restriction sites so that random DNA may be operably linked to the ipbd gene fragment; and c) provides the necessary genetic regulations; this DNA sequence is called a "display probe", (Secs. 1.1.4, 1.2.4, 1.3.4 and 4),

4) constructing that display probe,

5) cloning the display probe into and amplifying it in a suitable host into the OCV,

6) cloning random or pseudorandom DNA into one of the restriction sites provided in the display probe, (Sec. 6.2), whereby the random or pseudorandom DNA functions as a potential osp, and

7) harvesting GPs (Sec. 7) screening colonies of the transformed GPs for presence of IPBD on the GP surface; this screening is performed with an affinity molecule having high affinity for IPBD, denoted AfM(IPBD), (Sec. 8); or, alternatively;

8) selecting GPs that display IPBD by use of an affinity separation using AfM(IPBD), (Sec. 8).

Once a GP(IPBD) is produced, it can be used many times as the starting point for developing different novel proteins that bind to a variety of different targets. The knowledge of how we engineer the appearance of one IPBD on the surface of a GP can be used to design and produce other GP(IPBD)s that display different IPBDs.

Although Part I deals with only a single IPBD, many preparations are made for Part III where we introduce numerous mutations into the potential binding domain. References to PBD or pbd in Part I are to indicate a preparatory intent.

In Part II we optimize separation of GP(IPBD) from wild-type GP, denoted wtGP, based on the affinity of IPBD for AfM(IPBD) and establish the sensitivity of the affinity separation process. In a preferred embodiment, Part II of the process of the present invention involves:

1) preparing affinity columns bearing AfM(IPBD) at various densities of AfM(IPBD)/(volume of matrix), (Sec. 10.1),

2) preparing GP(IPBD)s with various amounts of IPBD per GP,

3) picking a gradient regime for eluting the columns (Sec. 10.1),

4) determining which combination of: a) IPBD/GP, b) density of AfM(IPBD)/(volume of support), c) initial ionic strength, d) elution rate, and e) (amount of GP)/(volume of support) loaded, gives the best separation of GP(IPBD) from wtGP (Sec. 10.1),

5) determining the smallest amount of GP(IPBD) that can be isolated from a much larger amount of wtGP using the optimal condition, (Sec. 10.2), and

6) determining the efficiency of the affinity separation procedure (Sec. 10.3).

Part II optimizes separation of a single type of GP(IPBD) from a large excess of a single different GP. The optimum conditions will be used in Part III to separate GP(PBD)s that bind the target from GP(PBD)s that do not bind the target. The optimization will be at one or more specific temperatures and at one or more specific pHs. In Part III, the user must specify the conditions under which the selected SBD should bind the target. If the conditions of intended use differ markedly from the conditions for which affinity separation was optimized, the user must return to Part II and optimize the affinity separation for conditions similar to the conditions of intended use of the selected SBD.

In Part III, we choose a target material and a GP(IPBD) that was developed by the method of Part I and that is suitable to the target material. Using IPBD as the PPBD to the first cycle of variegation, we prepare a wide variety of osp-pbd genes that encode a wide variety of PBDs. We use an affinity separation, developed by the method of Part II, to enrich the population of GP(vgPBD)s for GPs that display PBDs with binding properties relative to the target that are superior to the binding properties of the PPBD. An SBD selected from one variegation cycle becomes the PPBD to the next variegation cycle. In a preferred embodiment, Part III of the process of the present invention involves:

1) picking a target molecule (Sec. 11),

2) picking a GP(IPBD) (Sec. 12),

3) picking a set of several residues in the PPBD to vary based on a) the 3D structure of the IPBD, b) sequences of homologous proteins, and c) computer or theoretical modeling that indicates which residues can tolerate different amino acids without disrupting the underlying structure (Sec. 13.1),

4) picking a subset of the residues to be varied simultaneously based on the number of different variants and which variants are within the detection capabilities of the affinity separation; (Sec. 13.2);

5) implementing the variegation by:

a) synthesizing the part of the osp-pbd gene that encodes the residues to be varied using a specific mixture of nucleotide substrates for some or all of the bases encoding residues slated for variation, thereby creating a population of DNA molecules, denoted vgDNA (Sec. 13.3),

b) ligating this vgDNA, by standard methods, into the operative cloning vector (OCV) (e.g. a plasmid or bacteriophage) (Sec. 14.1),

c) using the ligated DNA to transform cells, thereby producing a population of transformed cells (Sec. 14.2),

d) culturing (i.e. increasing in number) the population of transformed cells and harvesting the population of GP(PBD)s, said population being denoted as GP(vgPBD), (Sec. 14.3),

e) enriching the population for GPs that bind the target by using the affinity separation process developed in Part II, with the chosen target molecule as affinity molecule (Sec. 15),

f) repeating steps III.5.d and III.5.e until a GP(SBD) having improved binding to the target is isolated (Sec. 15), and

g) testing the isolated SBD or SBDs for affinity and specificity for the chosen target (Sec. 15.8),

6) repeating steps III.3, III.4, and III.5 until the desired degree of binding is obtained.

Part III is repeated for each new target material. Part I need be repeated only if no GP(IPBD) suitable to a chosen target is available. Part II need be repeated for each newly-developed GP(IPBD) and for previously-developed GP(IPBD)s if the intended conditions of use of a novel binding protein differ significantly from the conditions of previous optimizations.

Sec. 0.2: Abbreviations:

The following abbreviations will be used throughout the present invention:

| Abbreviation | Meaning |
|---|---|
| GP | Genetic Package, e.g. a bacteriophage |
| X | Any protein |
| x | The gene for protein X |
| IPBD | Initial Potential Binding Domain, e.g. BPTI |
| PBD | Potential Binding Domain, e.g. a derivative of BPTI |
| SBD | Successful Binding Domain, e.g. a derivative of BPTI selected for binding to a target |
| PPBD | Parental Potential Binding Domain, i.e. an IPBD or an SBD from a previous selection |
| OSP | Outer Surface Protein, e.g. coat protein of a phage or LamB from E. coli |
| OSP-PBD | Fusion of an OSP and a PBD, order of fusion not specified |
| OSTS | Outer Surface Transport Signal |
| GP(x) | A genetic package containing the x gene |
| GP(X) | A genetic package that displays X on its outer surface |
| (Q) | An affinity matrix supporting "Q" e.g. (T4 lysozyme) is T4 lysozyme attached to an affinity matrix |
| AfM(W) | A molecule having affinity for "W", e.g. trypsin is an AfM(BPTI) |
| XINDUCE | A chemical that can induce expression of a gene, e.g. IPTG for the lacUV5 promoter |
| OCV | Operative Cloning Vector |
| $K_T$ | $K_T = [T][SBD]/[T{:}SBD]$ (T is a target) |
| $K_N$ | $K_N = [N][SBD]/[N{:}SBD]$ (N is a non-target) |
| DoAMoM | Density of AfM(W) on affinity matrix |
| Abun(x) | Abundance of DNA molecules encoding amino acid x |
| OMP | Outer membrane protein |
| nt | nucleotide |
| $K_d$ | A bimolecular dissociation constant, $K_d = [A][B]/[A{:}B]$ |
| $S_{err}$ | Error level in synthesizing vgDNA |

Sec. 0.3: Standard sequencing method:

The present invention is not limited to a single method of determining the sequence of nucleotides (nts) in DNA subsequences. Sequencing reactions, agarose gel electrophoresis, and polyacrylamide gel electrophoresis (PAGE) are performed by standard procedures (AUSU87).

The present invention is not limited to a single method of determining protein sequences, and reference in the appended claims to determining the amino acid sequence of a domain is intended to include any practical method or combination of methods, whether direct or indirect. The preferred method, in most cases, is to determine the sequence of the DNA that encodes the protein and then to infer the amino acid sequence. In some cases, standard methods of protein-sequence determination may be needed to detect post-translational processing.

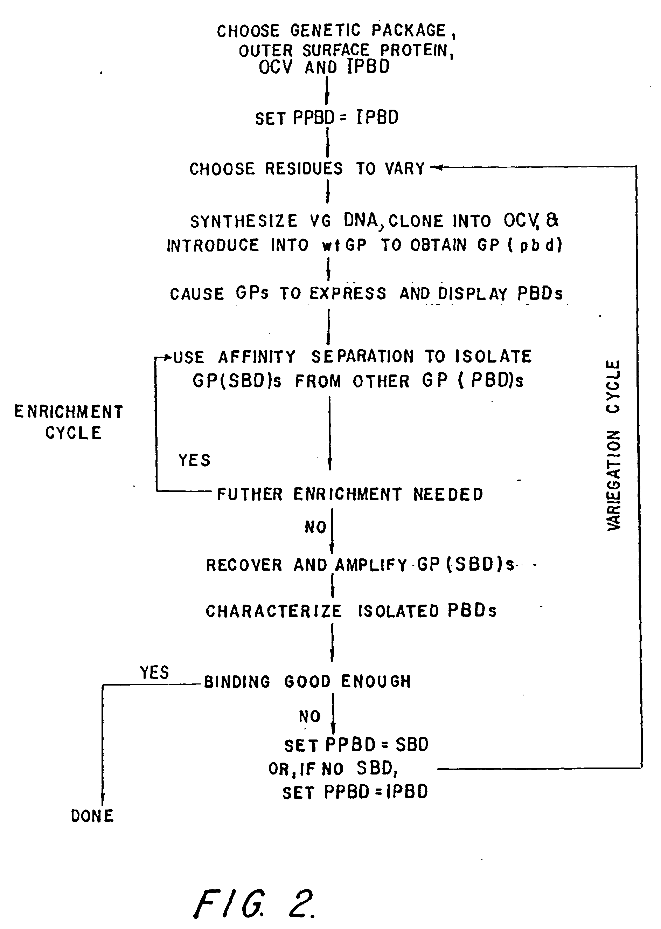

The major steps in the process of making and isolating a novel binding protein with affinity for a chosen target material are illustrated in Figure 2.

Sec. 1: Specification of Genetic Package and Means for Displaying a Heterologous Binding Domain On Its Outer Surface:

Sec. 1.0: General Requirements for Genetic Packages

It is emphasized that the GP on which selection-through-binding will be practiced must be capable, after the selection, either of growth in some suitable environment or of in vitro amplification and recovery of the encapsulated genetic message. During at least part of the growth, the increase in number must be approximately exponential with respect to time. The component of a population that exhibits the desired binding properties may be quite small, for example, one in $10^6$ or less. Once this component of the population is separated from the non-binding components, it must be possible to amplify it. Culturing viable cells is the most powerful amplification of genetic material known and is preferred. Genetic messages can also be amplified in vitro, but this is not preferred.

A GP may typically be a vegetative bacterial cell, a bacterial spore or a bacterial DNA virus. A strain of any living cell or virus is potentially useful if the strain can be:

1) maintained in culture,

2) affinity separated and retain its viability,

3) genetically altered with reasonable facility, and

4) manipulated to display the potential binding protein domain where it can interact with the target material during affinity separation.

DNA encoding the IPBD sequence may be operably linked to DNA encoding at least the outer surface transport signal of an outer surface protein (OSP) native to the GP so that the IPBD is displayed on the outer surface of the GP. It should be possible to cause a genetic package to display the IPBD or PBD on its outer surface without adversely affecting the viability of the GP or the binding characteristics of the IPBD or PBD, if the fusion is near domain boundaries (BECK83, CRAW87, TOTH86, SMIT85, MANO86; and cf. ROSS81, HOLL83).

Those characteristics of a protein that are recognized by a cell and that cause it to be transported out of the cytoplasm and displayed on the cell surface will be termed "outer-surface transport signals".

The replicable genetic entity (phage or plasmid) that carries the osp-pbd genes (derived from the osp-ipbd gene) through the selection-through-binding process, see Sec. 14, is referred to hereinafter as the operative cloning vector (OCV). When the OCV is a phage, it may also serve as the genetic package. The choice of a GP is dependent in part on the availability of a suitable OCV and suitable OSP.

Preferably, the GP is readily stored, for example, by freezing. If the GP is a cell, it should have a short doubling time, such as 20-40 minutes. If the GP is a virus, it should be prolific, e.g., a burst size of at least 100/infected cell. GPs which are finicky or expensive to culture are disfavored. The GP should be easy to harvest, preferably by centrifugation. The GP is preferably stable for a temperature range of -70 to 42°C (stable at 4°C for several days or weeks); resistant to shear forces found in HPLC; insensitive to UV; tolerant of desiccation; and resistant to a pH of 2.0 to 10.0, surface active agents such as SDS or Triton, chaotropes such as 4M urea or 2M guanidinium HCl, common ions such as $K^+$, $Na^+$, and $SO_4^{--}$, common organic solvents such as ether and acetone, and degradative enzymes. Finally, there must be a suitable OCV (see Sec. 3).

Preferably, the 3 D structure of the asp, and the sequence of the OSP gene p. 47 are known. If the 3D structure is not known, there is preferably knowledge of which residues are exposed on the cell surface, the location of the domain boundaries within the OSP, and/or of successful fusions of the OSP and a foreign insert. The OSP preferably appears in numerous copies on the outer surface of the GP, and preferably serves a non-essential function. It is desirable that the OSP not be post translationally processed, or at least that this processing be understood.

The preferred GP, OCV and OSP are those for which the fewest serious obstacles can be seen, rather than the one that scores highest on any one criterion.

Next, we consider general answers to the questions posed in this step for the cases of: a) vegetatively growing bacterial cells (Sec. 1.1), b) bacterial spores (Sec. 1.2), and c) (Sec. 1.3). Preferred OSPs for several GPs are given in Table 2.

Sec. 1.1: Bacterial Cells as Genetic Packages:

One may choose any well-characterized bacterial strain which may be grown in culture. The important questions in this case are: a) do we know enough about mechanisms that localize proteins on the outside of the cell, b) will the IPBD fold in the environment of the outer membrane, and c) will cells change expression of osp-pbd, derived from osp-ipbd, during affinity separation? Some IPBDs may need large or insoluble prosthetic groups, such as an $Fe_4S_4$ cluster, that are available within the cell, but not in the medium. The formation of $Fe_4S_4$ clusters found in some ferrodoxins is catalyzed by enzymes found in the cell (BONO85). IPBDs that require such prosthetic groups may fail to fold or function if displayed on bacterial cells.

Sec. 1.1.1: Preferred Bacterial Cells as GP :

In view of the extensive knowledge of E. coli, a strain of E. coli, defective in recombination, is the strongest candidate as a bacterial GP. Other preferred candidates are Salmonella typhimurium, Bacillus subtilis, and Pseudomonas aeruginosa.

Sec. 1.1.2: Preferred Outer Surface Proteins for Displaying IPBDs on Bacterial Cells:

Gram-negative bacteria have outer-membrane proteins (OMP), that form a subset of OSPs. Many OMPs span the membrane one or more times. The signals that cause OMPs to localize in the outer membrane are encoded in the amino acid sequence of the mature protein. Fusions of fragments of omp genes with fragments of an x gene have led to X appearing on the outer membrane (BENS84, CLEM81). If no fusion data are available, then we fuse an ipbd fragment to various fragments of the osp gene and obtain GPs that display the osp-ipbd fusion on the cell outer surface by screening or selection for the display-of-IPBD phenotype.

Oliver has reviewed mechanisms of protein secretion in bacteria (OLIV85 and OLIV87). Nikaido and Vaara (NIKA87) have reviewed mechanisms by which proteins become localized to the outer membrane of Gram-negative bacteria. For example, the LamB protein of E. coli is synthesized with a typical signal-sequence which is subsequently removed. Benson et al. (BENS84) showed that LamB-LacZ fusion proteins would be deposited in the outer membrane of E. coli when residues 1-49 of the mature LamB protein are included in the fusion, but that residues 1-43 are insufficient.

LamB of E. coli is a porin for maltose and maltodextrin transport, and serves as the receptor for adsorption of bacteriophages lambda and K10. This protein has been purified to homogeneity (ENDE78) and shown to function as a trimer (PALV79). Mutations to phage resistance have been used to define the parts of the LamB protein that adsorb each phage (ROAM80, CLEM81, CLEM83, GEHR87).

Topological models have been developed that describe the function of phage receptor and maltodextrin transport. The models describe these domains and their locations with respect to the surfaces of the outer membrane (CLEM81, CLEM83, CHAR84, HEIN88).

LamB is transported to the outer membrane if a functional N-terminal sequence is present; further, the first 49 amino acids of the mature sequence are required for successful transport (BENS84). Homology between parts of LamB protein and other outer membrane proteins OmpC, OmpF and PhoE has been detected (NIKA84), including homology between LamB amino acids 39-49 and sequences of the other proteins. These subsequences may label the proteins for transport to the outer membrane. Further, monoclonal antibodies derived from mice immunized with purified LamB, have been used to characterize tour distinct topological and functional regions, two of which are concerned with maltose transport (GABA82).

Sec. 1.1.3 Choice of Insertion site for IPBD in Bacterial Cell OSP:

For fusions of the phoA into the coding sequence for an integral membrane protein, the PhoA domain is localized according to where in the integral membrane protein the phoA gene was inserted (BECK83 and MANO86) That is, if phoA is inserted after an amino acid which normally is found in the cytoplasm, then PhoA appears in the cytoplasm. If phoA is inserted after an amino acid normally found in the periplasm, however, then the PhoA domain is localized on the periplasmic side of the membrane, and anchored in it. Beckwith and colleagues (BECK88) have extended these observations to the lacZ gene that can be inserted into genes for integral membrane proteins such that the LacZ domain appears in either the cytoplasm or the periplasm according to where the lacZ gene was inserted.

OSP-IPBD fusion proteins need not fill a structural role in the outer membranes of Gram-negative bacteria because parts of the outer membranes are not highly ordered. For large OSPs there is likely to be one or more sites at which osp can be truncated and fused to ipbd such that cells expressing the fusion will display IPBDs on the cell surface. If fusions between fragments of osp and x have been shown to display X on the cell surface, we can design an osp-ipbd gene by substituting ipbd for x in the DNA sequence. Otherwise, successful OMP-IPBD fusion is preferably sought by fusing fragments of the best omp to an ipbd, expressing the fused gene, and testing the resultant GPs for display-of-IPBD phenotype. We use the available data about OMP to pick the point or points of fusion between omp and ipbd to maximize the likelihood that IPBD will be displayed. Alternatively, we truncate osp at several sites or in a manner that produces osp fragments of variable length and fuse the osp fragments to ipbd; cells expressing the fusion are screened or selected which display IPBDs on the cell surface. An additional alternative is to include short segments of random DNA in the fusion of omp fragments to ipbd and then screen or select the resulting variegated population for members exhibiting the display-of-IPBD phenotype.

The promoter for the osp-ipbd gene, preferably, is subject to regulation by a small chemical inducer, such as isopropyl thiogalactoside (IPTG) (lac UV5 promoter). It need not come from a natural osp gene; any regulatable bacterial promoter can be used (MANI82).

Once a genetic packaging system employing vegetative bacterial cells has been designed, it is time to choose an IPBD (Sec. 2).

Sec. 1.1.4: In Vivo Selection for Pseudo-osp Gene From Random DNA Inserts in Bacterial Cells:

As an alternative to choosing a natural OSP and an insertion site in the OSP, we can construct a gene comprising: a) a regulatable promoter (e.g. lacUV5), b) a Shine-Dalgarno sequence, c) a periplasmic transport signal sequence, d) a fusion of the ipbd gene with a segment of random DNA (as in Kaiser et al. (KAIS87)), e) a stop codon, and f) a transcriptional terminator. The random DNA, which preferably comprises 90-300 bases, encode numerous potential OSTS. (EF. KAIS87) The fusion of ipbd and the random DNA could be in either order, but ipbd upstream is slightly preferred. Isolates from the population generated in this way can be screened for display of the IPBD. Preferably, a version of selection-through-binding is used to select GPs that display IPBD on the GP surface, and thus contain a DNA insert encoding a functional OSTS. Alternatively, clonal isolates of GPs may be screened for the display-of-IPBD phenotype.

The preference for ipbd upstream of the random DNA arises from consideration of the manner in which the successful GP(IPBD) will be used. In Part III, we will introduce numerous mutations into the pbd region of the osp-pbd gene, some of which might include gratuitous stop codons. If pbd precedes the random DNA, then gratuitous stop codons in pbd lead to no OSP-PBD protein appearing on the cell surface. If pbd follows the random DNA, then gratuitous stop codons in pbd might lead to incomplete OSP-PBD proteins appearing on the cell surface. Incomplete proteins often are non-specifically sticky so that GPs displaying incomplete PBDs are easily removed from the population.

Sec. 1.2: Displaying IPBD on bacterial spores:

Bacterial spores have desirable properties as GP candidates. Bacillus spores neither actively metabolize nor alter the proteins on their surface. However, spores are much more resistant than vegetative bacterial cells or phage to chemical and physical agents. Spores have the disadvantage that the molecular mechanisms that trigger sporulation are less well worked out than is the formation of M13 or the export of protein to the outer membrane of E. coli.

Sec. 1.2.1.: Preferred Bacterial Snores for Use as GPs:

Bacteria of the genus Bacillus form endospores that are extremely resistant to damage by heat, radiation, desiccation, and toxic chemicals (reviewed by Losick et al. (LOSI86)). These spores have complex structure and morphogenesis that is species-specific and only partially elucidated. The following observations are relevant to the use of Bacillus spores as genetic packages.

Plasmid DNA is commonly included in spores. Plasmid encoded proteins have been observed on the surface of Bacillus spores (DEBR86). Sporulation involves complex temporal regulation that is moderately well understood (LOSI86). The sequences of several sporulation promoters are known; coding sequences operatively linked to such promoters are expressed only during sporulation (RAYC87).

Donovan et al. have identified several polypeptide components of B. subtilis spore coat (DONO87); the sequences of two complete coat proteins and amino-terminal fragments of two others have been determined. Some components of the spore are synthesized in the forespore, e.g. small acid-soluble spore proteins (ERRI88), while other components are synthesized in the mother cell and appear in the spore (e.g. the coat proteins). This spatial organization of synthesis is controlled at the transcriptional level.

Spores self-assemble, but the signals that cause various proteins to localize in different parts of the spore are not well understood; presumably, the signals controlling deposition of the coat proteins from the cytoplasm of the mother cell onto the spore coat are embedded in the polypeptide sequence. Some, but not all, of the coat proteins are synthesized as precursors and are then processed by specific proteases before deposition in the spore coat (DONO87). Viable spores that differ only slightly from wild-type are produced in B. subtilis even if any one of four coat proteins is missing (DONO87). Disulfide bonds form within the spore (thiol reducing agents are needed to solubilize several of the proteins of the coat). The 12kd coat protein, CotD, contains 5 cysteines. CotD also contains an unusually high number of histidines (16) and prolines (7). The 11kd coat protein, CotC, contains only one cysteine and one methionine. CotC has a very unusual amino-acid sequence with 19 lysines (K) appearing as 9 K-K dipeptides and one isolated K. There are also 20 tyrosines (Y) of which 10 appear as 5 Y-Y dipeptides. Peptides rich in Y and K are known to become crosslinked in oxidizing environments (DEVO78, WAIT83, WAIT86). CotC contains 16 D and E amino acids that nearly equals the 19 Ks. There are no A, F, R, I, L, N, P, Q, S, or W amino acids in CotC. Neither CotC nor CotD is post-translationally cleaved. The proteins CotA and CotB are post-translationally cleaved.

Endospores from the genus Bacillus are more stable than are exospores from Streptomyces. Bacillus subtilis forms spores in 4 to 6 hours, but Streptomyces species may require days or weeks to sporulate. In addition, genetic knowledge and manipulation is much more developed for B. subtilis than for other spore-forming bacteria. Thus Bacillus spores are preferred over Streptomyces spores. Bacteria of the genus Clostridium also form very durable endospores, but clostridia, being strict anaerobes, are not convenient to culture. The choice of a species of Bacillus is governed by knowledge and availability of cloning systems and by how easily sporulation can be controlled. A particular strain is chosen by the criteria listed in Sec. 1.0. Many vegetative biochemical pathways are shut down when sporulation begins so that prosthetic groups might not be available.

Sec. 1.2.2 Preferred outer-surface proteins for Displaying IPBD on Bacterial Spores:

If a spore is chosen as GP, the promoter is the most important part of the osp gene, because the promoter of a spore coat protein is most active: a) when spore coat protein is being synthesized and deposited onto the spore and b) in the specific place that spore coat proteins are being made. In B. subtilis, some of the spore coat proteins are post-translationally processed by specific proteases. It is valuable to know the sequences of precursors and mature coat proteins so that we can avoid incorporating the recognition sequence of the specific protease into our construction of an OSP-IPBD fusion. The sequence of a mature spore coat protein contains information that causes the protein to be deposited in the spore coat; thus gene fusions that include some or all of a mature coat protein sequence are preferred for screening or selection for the display-of-IPBD phenotype.

Fusions of ipbd fragments to cotC or cotD fragments are likely to cause IPBD to appear on the spore surface. The genes cotC and cotD are preferred osp genes because CotC and CotD are not post-translationally cleaved. Subsequences from cotA or cotB could also be used to cause an IPBD to appear on the surface of B. subtilis spores, but we must take the post-translational cleavage of these proteins into account. DNA encoding IPBD could be fused to a fragment of cotA or cotB at either end of the coding region or at sites interior to the coding region. Spores could then be screened or selected for the display-of-IPBD phenotype.

To date, no Bacillus sporulation promoter has been shown to be inducible by an exogenous chemical inducer as the lac promoter of E. coli. Nevertheless, the quantity of protein produced from a sporulation promoter can be controlled by other factors, such as the DNA sequence around the Shine-Dalgarno sequence or codon usage.

Sec. 1.2.3: Choice of Insertion site for IPBD in OSP of Bacterial Spore:

The considerations governing insertion site in the spore OSP are the same as those given in Section 1.1.3.

Sec. 1.2.4: In Vivo Selection for Pseudo-osp Genes From Random DNA Inserts in Bacterial Spores:

Although the considerations for spores are nearly identical to the considerations for vegetative bacterial cells (Sec. 1.1), the available information on the mechanisms that cause proteins to appear on spores is meager so that use of the random-DNA approach becomes a more attractive option.

We can use the approach described above at 1.1.4 for attaching an IPBD to an E. coli cell, except that: a) a sporulation promoter is used, and b) no periplasmic signal sequence should be present.

Sec. 1.3: Displaying IPBD on Outer Surface of Phages:

Sec. 1.3.1: Preferred Phages for Use as GPs:

Unlike bacterial cells and spores, choice of a phage depends strongly on knowledge of the 3D structure of an OSP and how it interacts with other proteins in the capsid. The size of the phage genome and the packaging mechanism are also important because the phage genome itself is the cloning vector. The osp-ipbd gene must be inserted into the phage genome; therefore:

1) the virion must be capable of accepting the insertion or substitution of genetic material, and

2) the genome of the phage must be small enough to allow convenient manipulation.

Additional considerations in choosing phage are: 1) the morphogenetic pathway of the phage determines the environment in which the IPBD will have opportunity to fold, 2) IPBDs containing essential disulfides may not fold within a cell, 3) IPBDs needing large or insoluble prosthetic groups may not fold if secreted because the prosthetic group is lacking, and 4) when variegation is introduced in Part III, multiple infections could generate hybrid GPs that carry the gene for one PBD but have at least some copies of a different PBD on their surfaces; it is preferable to minimize this possibility.

Bacteriophages are excellent candidates for GPs because there is little or no enzymatic activity associated with intact mature phage, and because the genes are inactive outside a bacterial host, rendering the mature phage particles metabolically inert. The filamentous phage M13 and bacteriophage PhiX174 are of particular interest.

Filamentous phage :

The entire life cycle of the filamentous phage M13, a common cloning and sequencing vector, is well understood. M13 and f1 are so closely related that we consider the properties of each relevant to both (RASC86); any differentiation is for historical accuracy. The genetic structure (the complete sequence (SCHA78), the identity and function of the ten genes, and the order of transcription and location of the promoters) of M13 is well known as is the physical structure of the virion (BANN81, BOEK80, CHAN79, ITOK79, KAPL78, KUHN85b, KUHN87, MAKO80, MARV78, MESS78, OHKA81, RASC86, RUSS81, SCHA78, SMIT85, WEBS78, and ZIMM82); see RASC86 for a recent review of the structure and function of the coat proteins.

Relevant facts about M13 are disclosed in Example I.

Bacteriophage PhiX174 :

The bacteriophage PhiX174 is a very small icosahedral virus which has been thoroughly studied by genetics, biochemistry, and electron microscopy (See The Single-Stranded DNA Phages (DENH78)). To date, no proteins from PhiX174 have been studied by X-ray diffraction. PhiX174 is not used as a cloning vector because PhiX174 can accept almost no additional DNA; the virus is so tightly constrained that several of its genes overlap. Chambers et al. (CHAM82) showed that mutants in gene G are rescued by the wild-type G gene carried on a plasmid so that the host supplies this protein.

Three gene products of PhiX174 are present on the outside of the mature virion: F (capsid), G (major spike protein, 60 copies per virion), and H (minor spike protein, 12 copies per virion). The G protein comprises 175 amino acids, while H comprises 328 amino acids. The F protein interacts with the single-stranded DNA of the virus. The proteins F, G, and H are translated from a single mRNA in the viral infected cells.

Large DNA Phages

Phage such as lambda or T4 have much larger genomes than do M13 or PhiX174. Large genomes are less conveniently manipulated than small genomes. A phage with a large genome, however, could be used if genetic manipulation is sufficiently convenient. Phage such as lambda and T4 have more complicated 3D capsid structures than M13 or PhiX174, with more OSPs to choose from. Phage lambda virions and phage T4 virions form intracellularly, so that IPBDs requiring large or insoluble prosthetic groups might fold on the surfaces of these phage. Phage lambda and phage T4 are not preferred, however, derivatives of these phages could be constructed to overcome these disadvantages.

RNA Phages

RNA phage, such as Qbeta, are not preferred because manipulation of RNA is much less convenient than is the manipulation of DNA. Although competent RNA bacteriophage are not preferred, useful genetically altered RNA-containing particles could be derived from RNA phage, such as MS2.

To use MS2 as a GP, we would need to eliminate most of the natural viral genome so that an osp-ipbd gene could fit into the protein capsid. It is known that the A protein binds sequence-specifically to a site at the 5' end of the + RNA strand triggering formation of RNA-containing particles if coat protein is present. If a message containing the A protein binding site and the gene for a chimera of coat protein and a PBD were produced in a cell that also contained A protein and wild-type coat protein (both produced from regulated genes on a plasmid), then the RNA coding for the chimeric protein would get packaged. A package comprising RNA encapsulated by proteins encoded by that RNA satisfies the major criterion that the genetic message inside the package specifies something on the outside. The particles by themselves are not viable.

After isolating the packages that carry an SBD, we would need to:

1) separate the RNA from the protein capsid,

2) reverse transcribe the RNA into DNA, using AMV or MMTV reverse transcriptase, and

3) amplify the DNA by several cycles of polymerase chain reaction (PCR) until there is enough to subclone the recovered genetic message into a plasmid for sequencing and further work.

Alternatively, helper phage could be used to rescue the isolated phage.

Sec. 1.3.2: Preferred Outer-Surface Proteins for Displaying IPBDs on Phages:

For a given bacteriophage, the preferred OSP is usually one that is present on the phage surface in the largest number of copies, as this allows the greatest flexibility in varying the ratio of OSP-IPBD to wild type OSP and also gives the highest likelihood of obtaining satisfactory affinity separation. Moreover, a protein present in only one or a few copies usually performs an essential function in morphogenesis or infection; mutating such a protein by addition or insertion is likely to result in reduction in viability of the GP.

It is preferred that the wild-type osp gene be preserved. The ipbd gene fragment may be inserted either into a second copy of the recipient osp gene or into a novel engineered osp gene. The preferred OSP for use when the GP is M13 is the gene III protein (see Example 1).

Sec. 1.3.3: Choice of Insertion site for IPBD in OSP:

The user must choose a site in the candidate OSP gene for inserting a ipbd gene fragment. The coats of most bacteriophage are highly ordered. Thus in bacteriophage, unlike the cases of bacteria and spores, it is important to retain most or all of the residues of the parental OSP in engineered OSP-IPBD fusion proteins. A preferred site for insertion of the ipbd gene into the phage osp gene is one in which: a) the IPBD folds into its original shape, b) the OSP domains fold into their original shapes, and c) there is no interference between the two domains.

If there is a 3D model of the phage that indicates that either the amino or carboxy terminus of an OSP is exposed to solvent, then the exposed terminus of that mature OSP becomes the prime candidate for insertion of the ipbd gene. A low resolution 3D model suffices.

In the absence of a 3D structure, the amino and carboxy termini of the mature OSP are the best candidates for insertion of the ipbd gene. A functional fusion may require additional residues between the IPBD and OSP domains to avoid unwanted interactions between the domains. Random-sequence DNA or DNA coding for a specific sequence of a protein homologous to the IPBD or OSP, can be inserted between the osp fragment and the ipbd fragment if needed.

Fusion at a domain boundary within the OSP is also a good approach for obtaining a functional fusion. Smith exploited such a boundary when subcloning heterologous DNA into gene III of f1 (SMIT85).

There are several methods of identifying domains. Methods that rely on atomic coordinates have been reviewed by Janin and Chothia (JANI85) see also ROSE85, RASH84, VITA84, PABO79, POTE83, and SCOT87.

If the only structural information available is the amino acid sequence of the candidate OSP, we use the sequence to predict turns and loops. There is a high probability that some of the loops and turns will be correctly predicted (cf. Chou and Fasman, (CHOU72)); these locations are also candidates for insertion of the ipbd gene fragment.

Sec. 1.3.4: In Vivo Selection for Pseudo-OSP Gene from Random DNA Inserts in Bacterial Spores:

Alternatively, a functional insertion site may be determined by generating a number of recombinant constructions and selecting the functional strain by phenotypic characteristics. Because the OSP-IPBD must fulfill a structural role in the phage coat, it is unlikely that any particular random DNA sequence coupled to the ipbd gene will produce a fusion protein that fits into the coat in a functional way. Nevertheless, random DNA inserted between large fragments of a coat protein gene and the ipbd gene will produce a population that is likely to contain one or more members that display the IPBD on the outside of a viable phage. A display probe, similar to that defined in 1.1.4, is constructed and random DNA sequences cloned into appropriate sites.

Sec. 2: Choice of IPBD :

An IPBD may be chosen from naturally occurring proteins or domains of naturally occurring proteins, or may be designed from first principles. A designed protein may have advantages over natural proteins if: a) the designed protein is more stable, b) the designed protein is smaller, and c) the charge distribution of the designed protein can be specified more freely.

A candidate IPBD must meet the following criteria: 1) stablility under the conditions of its intended use (the domain may comprise the entire protein that will be inserted, _e.g._ BPTI), 2) knowledge of the amino acid sequence is obtainable, 3) identification of the residues on the outer surface, and their spatial relationships, and 4) availability of a molecule, AfM(IPBD) having high specific affinity for the IPBD.

Preferably, the IPBD is no larger than necessary because it is easier to arrange restriction sites in smaller amino-acid sequences. The usefulness of candidate IPBDs that meet all of these requirements depends on the availability of the information discussed below.

Information used to judge IPBD suitability includes: 1) a 3D structure (knowledge strongly preferred), 2) one or more sequences homologous to the IPBD (the more homologous sequences known, the better), 3) the pI of the IPBD (knowledge necessary in some cases), 4) the stability and solubility as a function of temperature, pH and ionic strength (preferably known to be stable over a wide range and soluble in conditions of intended use), 5) ability to bind metal ions such as $Ca^{++}$ or $Mg^{++}$ (knowledge preferred; binding _per se_, no preference), 6) enzymatic activities, if any (knowledge preferred, activity _per se_ has uses but may cause problems), 7) binding properties, if any (knowledge preferred, specific binding also preferred), 8) availability of a molecule having specific and strong affinity ( $K_d < 10^{-11}$ M) for the IPBD (preferred), 9) availability of a molecule having specific and medium affinity ( $10^{-8}$ M $< K_d < 10^{-6}$ M) for the IPBD (preferred), 10) the sequence of a mutant of IPBD that does not bind to the affinity molecule(s) (preferred), and 11) absorption spectrum in visible, UV, NMR, _etc._ (characteristic absorption preferred).

If only one species of molecule having affinity for IPBD (AfM(IPBD)) is available, it will be used to: a) detect the IPBD on the GP surface, b) optimize expression level and density of the affinity molecule on the matrix (Sec. 10.1), and c) determine the efficiency and sensitivity of the affinity separation (Secs. 10.2 and 10.3). As noted above, however, one would prefer to have available two species of AfM(IPBD), one with high and one with moderate affinity for the IPBD. The species with high affinity would be used in initial detection and in determining efficiency and sensitivity (10.2 and 10.3), and the species with moderate affinity would be used in optimization (10.1).

For at least 20 candidate IPBDs the above information is available or is practical to obtain, for example, bovine pancreatic trypsin inhibitor (BPTI, 58 residues), crambin (46 residues), third domain of ovomucoid (56 residues), T4 lysozyme (164 residues), and azurin (128 residues).

Most of the PBDs derived from a PPBD according to the process of the present invention affect residues having side groups directed toward the solvent. Exposed residues can accept a wide range of amino acids, while buried residues are more limited in this regard (REID88). Surface mutations typically have only small effects on melting temperature of the PBD, but may reduce the stability of the PBD. Hence the chosen IPBD should have a high melting temperature (60°C acceptable, the higher the better) and be stable over a wide pH range (8.0 to 3.0 acceptable; 11.0 to 2.0 preferred), so that the SBDs derived from the chosen IPBD by mutation and selection-through-binding will retain sufficient stability. Preferably, the substitutions in the IPBD yielding the various PBDs do not reduce the melting point of the domain below 50°C.

Two general characteristics of the target molecule, size and charge, make certain classes of IPBDs more likely than other classes to yield derivatives that will bind specifically to the target. Because these are very general characteristics, one can divide all targets into six classes: a) large positive, b) large neutral, c) large negative, d) small positive, e) small neutral, and f) small negative. A small collection of IPBDs, one or a few corresponding to each class of target, will contain a preferred candidate IPBD for any chosen target.

Alternatively, the user may elect to engineer a GP(IPBD) for a particular target; Sec 2.1 gives criteria that relate target size and charge to the choice of IPBD.

Sec. 2.1: Influence of target size on choice of IPBD:

If the target is a protein or other macromolecule a preferred embodiment of the IPBD is a small protein such as BPTI from <u>Bos</u> <u>taurus</u> (58 residues), crambin from rape seed (46 residues), or the third domain of ovomucoid from <u>Coturnix</u> <u>coturnix</u> <u>Japonica</u> (Japanese quail) (56 residues) (PAPA82), because targets from this class have clefts and grooves that can accommodate small proteins in highly specific ways. If the target is a macromolecule lacking a compact structure, such as starch, it should be treated as if it were a small molecule. Extended macromolecules with defined 3D structure, such as collagen, should be treated as large molecules.

If the target is a small molecule, such as a steroid, a preferred embodiment of the IPBD is a protein the size of ribonuclease from <u>Bos</u> <u>taurus</u> (124 residues), ribonuclease from <u>Aspergillus</u> <u>oryzae</u> (104 residues), hen egg white lysozyme from <u>Gallus</u> <u>gallus</u> (129 residues), azurin from <u>Pseudomonas</u> <u>aeruginosa</u> (128 residues), or T4 lysozyme (164 residues), because such proteins have clefts and grooves into which the small target molecules can fit. The Brookhaven Protein Data Bank contains 3D structures for these proteins. Genes encoding proteins as large as T4 lysozyme can be manipulated by standard techniques for the purposes of this invention.

If the target is a mineral, insoluble in water, one must consider the nature of the mineral's molecular surface.

Smooth surfaces, (such as crystalline silicon) require medium to large proteins (such as ribonuclease) as IPBD in order to have sufficient contact area and specificity. Rough, grooved surfaces (zeolites), could be bound either by small proteins (BPTI) or larger proteins (T4 lysozyme).

Sec. 2.2: Influence of target charge on choice of IPBD:

Electrostatic repulsion between molecules of like charge can prevent molecules with highly complementary surfaces from binding. Therefore, it is preferred that, under the conditions of intended use, the IPBD and the target molecule either have opposite charge or that one of them is neutral. Inclusion of counter ions can reduce or eliminate electrostatic repulsion.

Sec. 2.3: Other aspects of choice of IPBD:

If the chosen IPBD is an enzyme, it may be necessary to change one or more residues in the active site to inactivate enzyme function. For example, if the IPBD were T4 lysozyme and the GP were E. coli cells or M13, we would inactivate the lysozyme lest it lyse the cells. If, on the other hand, the GP were PhiX174, then inactivation of lysozyme may not be needed because T4 lysozyme can be overproduced inside E. coli cells without detrimental effects and PhiX174 forms intracellularly. It is preferred to inactivate enzyme IPBDs that might be harmful to the GP or its host by substituting mutant amino acids at one or more residues of the active site. It is permitted to vary one or more of the residues that were changed to abolish the original enzymatic activity of the IPBD. Those GPs that receive osp-pbd genes encoding an active enzyme may die, but the majority of sequences will not be deleterious.

Sec. 3: Choice at OCV:

The OCV is preferably small, e.g., less than 10 KB. It is desirable that cassette mutagenesis be practical in the OCV; preferably, at least 25 restriction enzymes are available that do not cut the OCV. It is likewise desirable that single-stranded mutagenesis be practical. Finally, the OCV preferably carries a selectable marker. A suitable OCV is obtained or is engineered by manipulation of available vectors. Plasmids are preferred over the bacterial chromosome because genes on plasmids are much more easily constructed and mutated than are chromosomal genes. When bacteriophage are to be used, the osp-ipbd gene must be inserted into the phage genome.
For phage such as M13, an antibiotic resistance gene is engineered into the genome (HINE80). More virulent phage, such as PhiX174, make discernable plaques that can be picked, in which case a resistance gene is not essential; furthermore, there is no room in the PhiX174 virion to add any new genetic material. Inability to include an antibiotic resistance gene is a disadvantage because it limits the number of GPs that can be screened.
It is preferred that GP(IPBD) carry a selectable marker not carried by wtGP. It is also preferred that wtGP carry a selectable marker not carried by GP(IPBD).

Sec. 4: Designing the osp-ipbd gene insert:

We design an amino acid sequence that will cause the IPBD to appear on the GP surface when it is expressed. This amino acid sequence may determine the entire coding region of the osp-ipbd gene, or it may contain only the ipbd sequence adjoining restriction sites into which random DNA will be cloned (Sec. 6.2).
The actual gene may be produced by any means. The pbd segment, derived from the ipbd segment, must be easily genetically manipulated in the ways described in Part III. Synthetic ipbd segments are preferred because they allow greatest control over placement of restriction sites.

Sec. 4.1 Genetic regulation of the osp-ipbd gene:

Regarding regulation of the osp-ipbd gene, the two important questions are: a) how much OSP-IPBD do we need on each GP, and b) how accurately must we regulate the amount?
The essential function of the affinity separation is to separate GPs that bear PBDs (derived from IPBD) having high affinity for the target from GPs bearing PBDs having low affinity for the target. If a gradient of some solute, such as increasing salt, changes the conditions, then all weakly-binding PBDs will cease to bind before any strongly-binding PBDs cease to bind. Regulation of the osp-pbd gene must be such that all packages display sufficient PBD to effect a good separation in Sec 15. If the amount of PBD/GP had an effect on the elution volume of the GP from the affinity matrix, then we would need to regulate the amount of PBD/GP accurately. The following analysis shows that there is no strong linear effect of IPBD/GP on elution volume and assumes only: a) that all GPs are the same size, b) that interactions between the PBDs and the affinity matrix dominate differential elution of GPs, c) that the system is at equilibrium, and d) that all PBDs on any one GP are identical.

If $N_p$ identical PBDs on a GP each have access to target molecules, and each PBD has a free-energy of binding to the target of delta $G_b$, then the total free energy of binding is

$$\text{delta } G_b{}^{tot} = N_p * \text{delta } G_b .$$

Delta $G_b$ is a function of parameters of the solvent, such as: 1) concentration of ions, 2) pH, 3) temperature, 4) concentration of neutral solutes such as sucrose, glucose, ethanol, etc., 5) specific ions, such as, calcium, acetate, benzoate, nicotinate, etc. If conditions are altered during affinity separation so that delta $G_b$ approaches zero, delta $G_b{}^{tot}$ approaches zero Np times faster. As delta $G_b{}^{tot}$ goes to or above zero, the packages will dissociate from the immobilized target molecules and be eluted.

GPs bearing more PBDs have a sharper transition between bound and unbound than packages with fewer of the same PBDs. For equilibrium conditions, the mid-point of the transition is determined only by the solution conditions that bring the individual interactions to zero free-energy. The number of PBDs/GP determines the sharpness of the transition.

It should also be noted that the number of PBDs/GP is usually influenced by physiological conditions so that a sample of genetically identical GP(PBD)s may contain GPs having different numbers of PBDs on the GP surface. In a population of GP(vgPBD)s each PBD sequence will appear on more that one GP, and the actual number of PBDs/GP will vary from GP to GP within some range. Within a variegated population of PBDs, let $PBD_x$ be the PBD with maximum affinity for the target. If there is a linear effect on elution volume of number of PBDs/GP, then the GPs having the greatest number of $PBD_x$ will be most retarded on the column. When we culture the enriched population the GP($PBD_x$) will be amplified and give rise to new GP($PBD_x$)s having varying numbers of $PBD_x$/GP. Thus the affinity separation process of the present invention could tolerate a linear effect of number of PBDs/GP on the elution volume of the GP(PBD) unless strong binding to target fortuitously causes the PBD to be displayed on the GP only in low number.

Since there is no linear effect on elution volume from the number of IPBDs/GP, need for highly accurate regulation of IPBD/GP is not anticipated. Reproducible gene expression is more easily controlled using regulated rather than constitutive genetic elements. The analysis above assumes that GP(IPBD)s are in equilibrium between solution in buffer and bound to the affinity matrix. Rate of elution may be an important parameter in column affinity chromatography. In batch elution from an affinity matrix or elution from an affinity plate, the time that each buffer is in contact with the affinity material may be an important variable. The density of affinity molecules on the matrix is an important variable in optimizing the affinity separation. Because the analysis above is qualitative, in Sec. 10 of the preferred embodiment we experimentally optimize: 1) the density of IPBD on the GP surface, 2) the density of affinity molecules on the affinity matrix, 3) the initial ionic strength, 4) the elution rate, and 5) the quantity of GP/(volume of matrix) to be loaded on the column.

Transcriptional regulation of gene expression is best understood and most effective, so we focus our attention on the promoter. A number of promoters are known that can be controlled by specific chemicals added to the culture medium. For example, the lacUV5 promoter is induced if isopropylthiogalactoside is added to the culture medium, for example, at between 1.0 uM and 10.0 mM. Hereinafter, we use "XINDUCE" as a generic term for a chemical that induces expression of a gene. If transcription of the osp-ipbd gene is controlled by XINDUCE, then the number of OSP-IPBDs per GP increases for increasing concentrations of XINDUCE until a fall-off in the number of viable packages is observed or until sufficient IPBD is observed on the surface of harvested GP(IPBD)s.

The attributes that affect the maximum number of OSP-IPBDs per GP are primarily structural in nature. There may be steric hindrance or other unwanted interactions between IPBDs if OSP-IPBD is substituted for every wild-type OSP. Excessive levels of OSP-IPBD may also adversely affect the solubility or morphogenesis of the GP. For cellular and viral GPs, as few as five copies of a protein having affinity for another immobilized molecule have resulted in successful affinity separations (FERE82a, FERE82b, and SMIT85).

Another consideration of promoter regulation is that it is useful later to know the range of regulation of the osp-ipbd. (Sec. 8) In particular, one should determine how nearly the absence of XINDUCE leads to the absence of IPBD on the GP surface; a non-leaky promoter is preferred. Non-leakiness is useful: a) to show that affinity of GP(osp-ipbd)s for AfM(IPBD) is due to the osp-ipbd gene, and b) to allow growth of 5 GP(osp-pbd) in the absence of XINDUCE if the expression of osp-pbd is disadvantageous. The lacUV5 promoter in conjunction with the LacI$^q$ repressor is a preferred example.

Sec. 4.2: DNA sequence design:

The present invention is not limited to a single method of gene design. The following procedure is an example of one method of gene design that fills the needs of the present invention.

If the amino-acid sequence of OSP-IPBD is a definite sequence, then the entire gene will be constructed (Sec. 6.1). If random DNA is to be fused to ipbd, then a "display probe" is constructed first; the random DNA is then inserted to complete the population of putative osp-ipbd genes (Sec. 6.2) from which a functional osp-ipbd gene is identified by in vivo selection or kindred techniques.

One may use any genetic engineering method to produce the correct gene fusion, so long as one can easily and accurately direct mutations to specific sites in the pbd DNA subsequence (Sec. 14.1). For the methods of mutagenesis considered here, however, the DNA sequence for the osp-ipbd gene must be different from any other DNA in the OCV. The degree and nature of difference needed is determined by the method of mutagenesis. One replaces subsequences coding for the PBD with vgDNA, then subsequences to be mutagenized must be bounded by restriction sites that are unique within the OCV. If single-stranded-oligonucleotide-directed mutagenesis is to be used, then the DNA sequence of the subsequence coding for the IPBD must be unique within the OCV.

Regulatory elements include: a) promoters, b) Shine-Dalgarno sequences, and c) transcriptional terminators, and may be isolated from nature or designed from knowledge of consensus sequences of natural regulatory regions.

The coding portions of genes to be synthesized are designed at the protein level and then encoded in DNA. The amino acid sequences are chosen to achieve various goals, including: a) display of a IPBD on the surface of a GP, b) change of charge on a IPBD, and c) generation of a population of PBDs from which to select an SBD. The ambiguity in the genetic code is exploited to allow optimal placement of restriction sites and to create various distributions of amino acids at variegated codons.

Sec. 4.3: Specific DNA sequence assignment:

A computer program may be used to identify all possible ambiguous DNA sequences coding for an amino-acid sequence given by the user and to identify places where recognition sites for site-specific restriction enzymes could be provided without altering the amino-acid sequence.

Restriction sites are positioned within the osp-ipbd gene so that the longest segment between sites is as short as possible. Enzymes the produce cohesive ends are preferred. The codon preferences of the intended host and the secondary structure of the messenger RNA are also considered.

Sec. 5.1: Organization of gene synthesis:

An established strategy for gene synthesis is to synthesize both strands of the entire gene in overlapping segments of 20 to 50 nucleotides (nts) (THER88). We prefer an alternative method that is more suitable for synthesis of vgDNA. Our method differs from previous methods (OLIP86, OLIP87, AUSU87) in that we: a) use two synthetic strands, and b) do not cut the extended DNA in the middle. Our goals are: a) to produce longer pieces of dsDNA than can be synthesized as ssDNA on commercial DNA synthesizers, and b) to produce strands complementary to single-stranded vgDNA. By using two synthetic strands, we remove the requirement for a palindromic sequence at the 3' end.

DNA synthesizers can produce oligo-nts of up to 100 nts in reasonable yield, $M_{DNA} = 100$. The parameters $N_w$ (the length of overlap needed to obtain efficient annealing) and $N_s$ (the number of spacer bases needed so that a restriction enzyme can cut near the end of blunt-ended dsDNA) are determined by DNA and enzyme chemistry. $N_w = 10$ and $N_s = 5$ are reasonable values.

We divide the DNA sequence to be synthesized into two nearly equal parts, each 5-8 bases longer than half the total length, so that there is an overlap between the two parts of 10 to 16 bp (Nw) containing no variegated bases. The overlap preferably, is not palindromic and has high GC content. We synthesize the overlap portion and the 5' extension of each strand. When these strands are annealed and completed with Klenow enzyme and all four NTPs, we obtain the desired sequence as blunt-ended dsDNA. If the DNA is to be ligated to other DNA having cohesive ends, five to ten (Ns) bases are added to that end. The synthetic dsDNA can then be cut efficiently with an appropriate restriction enzyme (OLIP87).

Because $M_{DNA}$ is not rigidly fixed at 100, the current limits of 190 (= $2 M_{DNA} - N_w$) nts overall and 100 in each fragment are not rigid, but can be exceeded by 5 or 10 nts. Going beyond the limits of 190 and 100 will lead to lower yields, but these may be acceptable in certain cases.

Sec. 5.2: DNA synthesis and purification methods :

The present invention is not limited to any particular method of DNA synthesis or construction.

In the preferred embodiment, DNA is synthesized by standard means on a Milligen 7500 DNA synthesizer. The Milligen 7500 has seven vials from which phosphoramidites may be taken. Normally, the first four contain A, C, T, and G. The other three vials may contain unusual bases such as inosine or mixtures of bases, the so-called "dirty bottle". The standard software allows programmed mixing of two, three, or four bases in equimolar quantities.

The present invention is not limited to any particular method of purifying DNA for genetic engineering. Agarose gel electrophoresis and electroelution on an IBI device (International Biotechnologies, Inc., New Haven, CT) is, preferably, used to purify large dsDNA fragments. For oligo-nts, PAGE and electroelution with an Epigene device (Epigene Corp., Baltimore, MD) are an alternative to HPLC.

Sec. 6.1: Cloning of Known OSP-ipbd gene into OCV:

In the preferred method, the synthetic gene is constructed using plasmids that are transformed into bacterial cells by standard methods (MANI82, p250) or slightly modified standard methods. Alternatively, DNA fragments derived from nature are operably linked to other fragments of DNA derived from nature or to synthetic DNA fragments. In most cases of the preferred method, gene synthesis involves construction of a series of plasmids containing larger and larger segments of the complete gene.

Sec. 6.2 Cloning of Random DNA (Potential osp) Into Display Probe:

If random DNA and phenotypic selection or screening are used to obtain a GP(IPBD), then we clone random DNA into one of the restriction sites that was designed into the display probe.

The random DNA may be obtained in a variety of ways. Degenerate synthetic DNA is one possibility. Alternatively, pseudorandom DNA may be taken from nature. If, for example, an Sph I site (GCATG/C) has been designed into the display probe at one end of the ipbd fragment, then we would use Nla III (CATG/) to partially digest DNA that contains a wide variety of sequences, generating a wide variety of fragments with CATG 3' overhangs. Preferably, the display probe has different restriction sites at each end of the ipbd gene so that random DNA can be cloned at either end.

A plasmid carrying the display probe is digested with the appropriate restriction enzyme and the fragmented, random DNA is annealed and ligated by standard methods. The ligated plasmids are used to transform cells that are grown and selected for expression of the antibiotic-resistance gene. Plasmid-bearing GPs are then selected for the display-of-IPBD phenotype by the procedure given in Sec. 15 of the present invention using AfM(IPBD) as if it were the target. Sec. 15 is designed to isolate GP(PBD)s that bind to a target from a large population that do not bind.

Sec. 7: Harvest of GPs :

Cells are transformed with ligated OCVs and selected for uptake of OCV after an appropriate incubation with an agent appropriate to the selectable markers on the OCV. GPs are harvested by methods appropriate to the GP at hand, generally, centrifugation to pelletize GPs and resuspension of the pellets in sterile medium (cells) or buffer (spores or phage).

Sec. 8: Verification of Display Strategy:

The harvested packages are now tested for display of IPBD on the surface; any ions or cofactors known to be essential for the stability of IPBD or AfM(IPBD) must be included at appropriate levels. The tests can be done: a) by affinity labeling, b) enzymatically, c) spectrophotometrically, d) by affinity separation, or e) by affinity precipitation. The AfM(IPBD) in this step is one picked to have strong affinity (preferably, $K_d < 10^{-11}$ M) for the IPBD molecule and little or no affinity for the wtGP. For example, if BPTI were the IPBD, trypsin, anhydrotrypsin, or antibodies to BPTI could be used as the AfM(BPTI) to test for the presence of BPTI. Anhydrotrypsin, a trypsin derivative with serine 195 converted to dehydroalanine, has no proteolytic activity but retains its affinity for BPTI (AKOH72 and HUBE77).

Preferably, the presence of the IPBD on the surface of the GP is demonstrated through the use of a soluble, labeled derivative of a AfM(IPBD) with high affinity for IPBD. The labeled derivative of AfM(IPBD) is denoted as AfM(IPBD)*.

If random DNA has been used, then the procedures of Sec. 15 are used to obtain a clonal isolate that has the display-of-IPBD phenotype. Alternatively, clonal isolates may be screened for the display-of-IPBD phenotype. The tests of this step are applied to one or more of these clonal isolates.

If no isolates that bind to the affinity molecule are obtained we take corrective action as disclosed in Sec. 9.

If one or more of the tests indicates that the IPBD is displayed on the GP surface, we verify that the binding of molecules having known affinity for IPBD is due to the chimeric osp-ipbd gene through the use of standard genetic and biochemical techniques, such as:

    1) transferring the osp-ipbd gene into the parent GP to verify that osp-ipbd confers binding,

2) deleting the osp-ipbd gene from the isolated GP to verify that loss of osp-ipbd causes loss of binding,

3) showing that binding of GPs to AfM(IPBD) correlates with [XINDUCE] (in those cases that expression of osp-ipbd is controlled by [XINDUCE]), and

4) showing that binding of GPs to AfM(IPBD) is specific to the immobilized AfM(IPBD) and not to the support matrix.

Presence of IPBD on the GP surface is indicated by a strong correlation between [XINDUCE] and the reactions that are linear in the amount of IPBD (such as: a) binding of GPs by soluble AfM(IPBD)*, b) absorption caused by IPBD, and c) biochemical reactions of IPBD). The demonstration (4) that binding is to AfM(IPBD) and the genetic tests (1) and (2) are important; the test with XINDUCE (3) is less so.

We sequence the relevant ipbd gene fragment from each of several clonal isolates to determine the construction.

We establish the maximum salt concentration and pH range for which the GP(IPBD) binds the chosen AfM(IPBD).

If the IPBD is displayed on the outside of the GP, and if that display is clearly caused by the introduced osp-ipbd gene, we proceed to Part II, otherwise we must analyze the result and adopt appropriate corrective measures.

Sec. 9: Perfecting the Display System:

If we have attempted to fuse an ipbd fragment to a natural osp fragment, our options are :

1) pick a different fusion to the same osp by

    a) using opposite end of osp,
    b) keeping more or fewer residues from osp in the fusion; for example, in increments of 3 or 4 residues,
    c) trying a known or predicted domain boundary,
    d) trying a predicted loop or turn position,

2) pick a different osp, or

3) switch to random DNA method.

If we have just tried the random DNA method unsuccessfully, our options are :

1) choose a different relationship between ipbd fragment and random DNA (ipbd first, random DNA second or vice versa),

2) try a different degree of partial digestion, a different enzyme for partial digestion, a different degree of shearing or a different source of natural DNA, or

3) switch to the natural OSP method.

If all reasonable OSPs of the current GP have been tried and the random DNA method has been tried, both without success, we pick a new GP.

Part II

Sec. 10.0: Affinity Separation Means:

In Part II we optimize an affinity separation system that will be used in Part III to enrich a population of GP(vgPBD)s for those GP(PBD)s that display PBDs with increased affinity for the target.

Affinity chromatography is the preferred means, but FACS, electrophoresis, or other means may also be used.

Sec. 10.1: Optimization of Affinity Chromatography Separation:

Changes in eluant concentration cause GPs to elute from the column. Elution volume, however, is more easily measured and specified. It is to be understood that the eluant concentration is the agent causing GP release and that an eluant concentration can be calculated from an elution volume and the specified gradient.

Using a specified elution regime, we compare the elution volumes of GP(IPBD)s with the elution volumes of wtGP on affinity columns supporting AfM(IPBD). Comparisons are made at various: a) amounts of IPBD/GP, b) densities of AfM(IPBD)/(volume of matrix) (DoAMoM), c) initial ionic strengths, d) elution rates, e) amounts of GP/(volume of support), f) pHs, and g) temperatures, because these are the parameters most likely to affect the sensitivity and efficiency of the separation. We then pick those conditions giving the best separation.

We do not optimize pH or temperature; rather we record optimal values for the other parameters for one or more values of pH and temperature. The conditions of intended use, specified by the user (Sec. 11), may include a specification of pH or temperature. If pH is specified, then pH will not be varied in eluting the column (Sec. 15.3). Decreasing pH may be used to liberate bound GPs from the matrix. If the intended use specifies a temperature, we will hold the affinity column at the specified temperature during elution, but we might vary the temperature during recovery.

The AFM (IPBD) is preferably one known to have moderate affinity for the IPBD ($K_d$ in the range $10^{-6}$ M to $10^{-8}$ M). When populations of GP(vgPBD)s are fractionated, there will be roughly three subpopulations: a) those with no binding, b) those that have some binding but can be washed off with high salt or low pH, and c) those that bind very tightly and must be rescued in situ. We optimize the parameters to separate (a) from (b) rather than (b) from (c). Let $PBD_w$ be a PBD having weak binding to the target and $PBD_s$ be a PBD having strong binding. Higher DoAMoM might, for example, favor retention of GP($PBD_w$) but also make it very difficult to elute viable GP($PBD_s$). We will optimize the affinity separation to retain GP($PBD_w$) rather than to allow release of GP($PBD_s$) because a tightly bound GP($PBD_s$) can be rescued by in situ growth. If we find that DoAMoM strongly affects the elution volume, then in part III we may reduce the amount of target on the affinity column when an SBD has been found with moderately strong affinity ($K_d$ on the order of $10^{-7}$ M) for the target.

In this step, we measure elution volumes of genetically pure GPs that elute from the affinity matrix as sharp bands that can be detected by UV absorption. Samples from effluent fractions are plated on suitable medium (cells or spores) or on sensitive cells (phage) and colonies or plaques counted.

Several values of IPBD/GP, DoAMoM, elution rates, initial ionic strengths, and loadings should be examined. We anticipate that optimal values of IPBD/GP and DoAMoM will be correlated and therefore should be optimized together. The effects of initial ionic strength, elution rate, and amount of GP/(matrix volume) are unlikely to be strongly correlated, and so they can be optimized independently.

For each set of parameters to be tested, the column is eluted in a specified manner. For example, we may use a regime called Elution Regime 1: a KCl gradient runs from 10mM to maximum allowed for the GP(IPBD) viability in 100 fractions of 0.05 $V_V$ (void volume), followed by 20 fractions of 0.05 $V_V$ at maximum allowed KCl; pH of the buffer is maintained at the specified value with a convenient buffer such as Tris. It is important that the conditions of this optimization be similar to the conditions that are used in Part III for selection for binding to target (Sec. 15.3) and recovery of GPs from the chromatographic system (Sec. 15.4).

When the osp-ipbd gene is regulated by [XINDUCE], IPBD/GP can be controlled by varying [XINDUCE]. Appropriate values of [XINDUCE] depend on the identity of [XINDUCE] and the promoter; if, for example, XINDUCE is isopropyithiogalactoside (IPTG) and the promoter is lacUV5, then [IPTG] = 0, 0.1 uM, 1.0 uM, 10.0 uM, 100.0 uM, and 1.0 mM are appropriate levels to test. The range of variation of [XINDUCE] is extended until an optimum is found or an acceptable level of expression is obtained.

DoAMoM is varied from the maximum that the matrix material can bind to 1% or 0.1% of this level in appropriate steps. We anticipate that the efficiency of separation will be a smooth function of DoAMoM so that it is appropriate to cover a wide range of values for DoAMoM with a coarse grid and then explore the neighborhood of the approximate optimum with a finer grid.

Several values of initial ionic strength are tested, such as 1.0 mM, 5.0 mM, 10.0 mM and 20.0 mM.

The elution rate is varied, by successive factors of 1/2, from the maximum attainable rate to 1/16 of this value. The fastest elution rate giving the good separation is optimal.

The goal of the optimization is to obtain a sharp transition between bound and unbound GPs, triggered by increasing salt or decreasing pH or a combination of both. This optimization need be performed only: a) for each temperature to be used, b) for each pH to be used, and c) when a new GP(IPBD) is created.

Regulatable promoters are available for all genetic packages except, possibly, bacterial spores. A promoter functional in bacterial spores might be prepared by constructing a hybrid of a sporulation promoter and a regulatable bacterial promoter (e.g., lac), or by saturation mutagenesis of a sporulation promoter followed by screening for regulatable promoter activity (cf. OLIP86, OLIP87). When the promoter of the osp-ipbd gene is not regulatable, we optimize DoAMoM, the elution rate, and the amount of GP/volume of matrix. If the optimized affinity separation is not acceptable, we must develop a means to alter the amount of IPBD per GP.

Sec. 10.2: Measuring the sensitivity of affinity separation:

We determine the sensitivity of the affinity separation ($C_{sensi}$) by measuring the minimum quantity of GP(IPBD) that can be detected in the presence of a large excess of wtGP. The user chooses a number of separation cycles, denoted $N_{chrom}$, that will be performed before an enrichment is abandoned; preferably, $N_{chrom}$ is in the range 6 to 10 and $N_{chrom}$ must be greater than 4. Enrichment can be terminated by isolation of a desired GP(SBD) before $N_{chrom}$ passes.

The measurement of sensitivity is significantly expedited if GP(IPBD) and wtGP carry different selectable markers.

Mixtures of GP(IPBD) and wtGP are prepared in the ratios of 1:$V_{lim}$, where $V_{lim}$ ranges by an appropriate factor (e.g. 1/10) over an appropriate range, typically $10^{11}$ through $10^4$. Large values of $V_{lim}$ are tested first; once a positive result is obtained for one value of $V_{lim}$, no smaller values of $V_{lim}$ need be tested. Each mixture is applied to a column supporting, at the optimal DoAMoM, an AfM(IPBD) having high affinity for IPBD and the column is eluted by the specified elution regime. The last fraction that contains viable GPs and an inoculum of the column matrix material are cultured. If GP(IPBD) and wtGP have different selectable markers, then transfer onto selection plates identifies each colony. Otherwise, a number (e.g. 32) of GP clonal isolates are tested for presence of IPBD by the techniques discussed in Sec. 8.

If IPBD is not detected on the surface of any of the isolated GPs, then GPs are pooled from: a) the last few (e.g. 3 to 5) fractions that contain viable GPs, and b) an inoculum taken from the column matrix. The pooled GPS are cultured and passed over the same column and enriched for GP(IPBD) in the manner described. This process is repeated until $N_{chrom}$ passes have been performed, or until the IPBD has been detected on the GPs. If GP(IPBD) is not detected after $N_{chrom}$ passes, $V_{lim}$ is decreased and the process is repeated.

$C_{sensi}$ equals the highest value of $V_{lim}$ for which the user can recover GP(IPBD) within $N_{chrom}$ passes. The number of chromatographic cycles ($K_{cyc}$) that were needed to isolate GP(IPBD) gives a rough estimate of $C_{eff}$; $C_{eff}$ is approximately the $K_{cyc}$th root of Vlim:

$$C_{eff} = (approx.) \exp( \log_e(V_{lim})/K_{cyc} )$$

For example, if $V_{lim}$ were $4.0 \times 10^8$ and three separation cycles were needed to isolate GP(IPBD), then $C_{eff}$ = (approx.) 736.

Sec. 10.3: Measuring the efficiency of separation :

To determine $C_{eff}$ more accurately, we determine the ratio of GP(IPBD)/wtGP loaded onto an AfM(IPBD) column that yields approximately equal amounts of GP(IPBD) and wtGP after elution.

Sec. 10.4: Other Separation Means

Other separation means are optimized in a manner parallel to the used for affinity chromatography.

FACS (e.g. FACStar from Beckton-Dickinson, Mountain View, CA) is most appropriate for bacterial cells and spores because the sensitivity of the machines requires approximately 1000 molecules of fluorescent label bound to each GP to accomplish a separation. To optimize FACS separation of GPs, we use a derivative of Afm(IPBD) that is labeled with a fluorescent molecule, denoted Afm (IPBD)*. The variables that must be optimized include: a) amount of IPBD/GP, b) concentration of Afm(IPBD)*, c) ionic strength, d) concentration of GPs, and e) parameters pertaining to operation of the FACS machine. Because Afm(IPBD)* and GPs interact in solution, the binding will be linear in both [Afm(IPBD)*] and [displayed IPBD]. Preferably, these two parameters are varied together. The other parameters can be optimized independently.

Electrophoresis is most appropriate to bacteriophage because of their small size (SERW87). Electrophoresis is a preferred separation means if the target is so small that chemically attaching it to a column or to a fluorescent label would essentially change the entire target. For example, chloroacetate ions contain only seven atoms and would be essentially altered by any linkage. GPs that bind chloroacetate would become more negatively charged than GPs that do not bind the ion and so these classes of GPs could be separated.

The parameters to optimize for electrophoresis include: a) IPBD/GP, b) concentration of gel material, e.g. agarose, c) concentration of Afm (IPBD), d) ionic strength, e) size, shape, and cooling capacity of the electrophoresis apparatus, f) voltages and currents, and f) concentration of GPs. Preferably, IPBD/GP and [Afm(IPBD)] are varied at the same time and other parameters are optimized independently.

Part III

Sec. 11.0: Choice of target material :

Any material may be chosen as target material, subject only to the following restrictions:

If affinity chromatography is to be used, then:

1) the molecules of the target material must be of sufficient size and chemical reactivity to be applied to a solid support suitable for affinity separation,

2) after application to a matrix, the target material must not react with water,

3) after application to a matrix, the target material must not bind or degrade proteins in a non-specific way, and

4) the molecules of the target material must be sufficiently large that attaching the material to a matrix allows enough unaltered surface area (generally at least 500 Å$^2$, excluding the atom that is connected to the linker) for protein binding.

If FACS is to be used as the affinity separation means, then:

1) the molecules of the target material must be of sufficient size and chemical reactivity to be conjugated to a suitable fluorescent dye or the target must itself be fluorescent,

2) after any necessary fluorescent labeling, the target must not react with water,

3) after any necessary fluorescent labeling, the target material must not bind or degrade proteins in a non-specific way, and

4) the molecules of the target material must be sufficiently large that attaching the material to a suitable dye allows enough unaltered surface area (generally at least 500 A$^2$, excluding the atom that is connected to the linker) for protein binding.

If affinity electrophoresis is to be used, then:

1) the target must either be charged or of such a nature that its binding to a protein will change the charge of the protein,

2) the target material must not react with water,

3) the target material must not bind or degrade proteins in a non-specific way, and

4) the target must be compatible with a suitable gel material.

Possible target materials include, but are not limited to: a) soluble proteins (such as horse heart myoglobin, human neutrophil elastase, activated (blood clotting) factor X, alpha-fetoprotein, alpha interferon, melittin, Bordetella pertussis adenylate cyclase toxin, any retroviral pol protease or any retroviral gag protease), b) lipoproteins (such as human low density lipoprotein), c) glycoproteins (such as a monoclonal antibody), d) lipopolysaccharides (such as O-antigen of Salmonella enteritidis), e) nucleic acids (such as tRNAs, ribosomal RNAs, messenger RNAs dsDNA or ssDNA, possibly with sequence specificity); f) soluble organic molecules (such as cholesterol , aspartame, bilirubin, morphine, codeine, dichlorodiphenyltrichlorethane (DDT), benzo(a)pyrene, prostaglandin PGE2, protoporphyrin IX, or actinomycin D), g) organometallic complexes (such as iron haem or cobolt haem), h) organic polymers (such as cellulose or chitin), i) insoluble minerals (such as asbestos, zeolites, or hydroxylapatite), j) viral and phage coat proteins (such as influenza haemaggutinin or phage lambda capsid), and k) bacterial membrane or outer membrane proteins (such as LamB from E. coli or flagella proteins).

A supply of several milligrams of pure target material is desired. Impure target material could be used, but one might obtain a protein that binds to a contaminant instead of to the target.

The following information about the target material is highly desirable:

1) stability as a function of temperature, pH, and ionic strength,

2) stability with respect to chaotropes such as urea or guanidinium Cl,

3) pI,

4) molecular weight,

5) requirements for prosthetic groups or ions, such as haem or $Ca^{+2}$, and

6) proteolytic activity, if any.

In addition to this most desirable information, it is useful to know: 1) the target's sequence, if the target is a macromolecule, 2) the 3D structure of the target, 3) enzymatic activity, if any, and 4) toxicity, if any.

The user of the present invention specifies certain parameters of the intended use of the binding protein:

1) the acceptable temperature range,

2) the acceptable pH range,

3) the acceptable concentrations of ions and neutral solutes,

4) the maximum acceptable dissociation constant for the target and the SBD:

$$K_T = [\text{Target}][\text{SBD}]/[\text{Target:SBD}]$$

In some cases, the user may require discrimination between T, the target, and N, some non-target. Let

$$K_T = [T][\text{SBD}]/[T:\text{SBD}] \text{ , and}$$

$$K_N = [N][\text{SBD}]/[N:\text{SBD}] \text{ ,}$$

then $K_T/K_N = ([T][N:\text{SBD}])/([N][T:\text{SBD}])$ .

The user then specifies a maximum acceptable value for the ratio $K_T/K_N$.

If the target material is a general protease, one must consider the following points:

1) a highly specific protease can be treated like any other target,

2) a general protease, such as subtilisin, may degrade the OSPs of the GP including OSP-PBDs; there are several alternative ways of dealing with general proteases, including: a) a chemical inhibitor may be used to prevent proteolysis (e.g. phenylmethylfluorosulfate (PMFS) that inhibits serine proteases), b) one or more active-site residues may be mutated to create an inactive protein (e.g. a serine protease in which the active serine is mutated to alanine), or c) one or more active-site amino-acids of the protein may be chemically modified to destroy the catalytic activity (e.g. a serine protease in which the active serine is converted to anhydroserine),

3) SBDs selected for binding to a protease need not be inhibitors; SBDs that happen to inhibit the protease target are a fairly small subset of SBDs that bind to the protease target,

4) the more we modify the target protease, the less like we are to obtain an SBD that inhibits the target protease, and

5) if the user requires that the SBD inhibit the target protease, then the active site of the target protease must not be modified any more than necessary; inactivation by mutation or chemical modification are preferred methods of inactivation and a protein protease inhibitor becomes a prime candidate for IPBD. For example, BPTI could be mutated, by the methods of the present invention, to bind to proteases other than trypsin (TANK77 and TSCH87).

Sec. 12.0: Choice of GP(IPBD) :

The user must pick a GP(IPBD) that is suitable to the chosen target according to the criteria of Sec. 2. It is anticipated that a small collection of a GP(IPBD)s can be assembled such that, for any chosen target, at least one member of the collection will be a suitable starting point for engineering a protein that binds to the chosen target by the methods of the present invention. The user should optimize the affinity separation for conditions appropriate to the intended use by the methods described in Part II.

Sec. 13.0: Identification of Family of PBDs, Related to PPBD, to Be Generated

Sec. 13.1: Choosing residues on IPBD (or other PPBD) to vary:

We choose residues in the IPBD to vary through consideration of several factors, including: a) the 3D structure of the IPBD, b) sequences homologous to IPBD, and c) modeling of the IPBD and mutants of the IPBD. Because the number of residues that could strongly influence binding is always greater than the number that can be varied simultaneously, the user must pick a subset of those residues to vary at one time. The user must also pick trial levels of variegation and calculate the abundances of various sequences. The list of varied residues and the level of variegation at each varied residue are adjusted until the composite variegation is commensurate with $C_{sensi}$ and $M_{ntv}$.

A key concept is that only structured proteins exhibit specific binding, i.e. can bind to a particular chemical entity to the exclusion of most others. Thus the residues to be varied are chosen with an eye to preserving the underlying IPBD structure. Substitutions that prevent the PBD from folding will cause GPs carrying those genes to bind indiscriminately so that they can easily be removed from the population.

Burial of hydrophobic surfaces so that bulk water is excluded is one of the strongest forces driving the binding of proteins to other molecules. Bulk water can be excluded from the region between two molecules only if the surfaces are complementary. We must test as many surfaces as possible to find one that is complementary to the target. The selection-through-binding isolates those proteins that are more nearly complementary to some surface on the target. The effective diversity of a variegated population is measured by the number of different surfaces, rather than the number of protein sequences. Thus we should maximize the number of surfaces generated in our population, rather than the number of protein sequences.

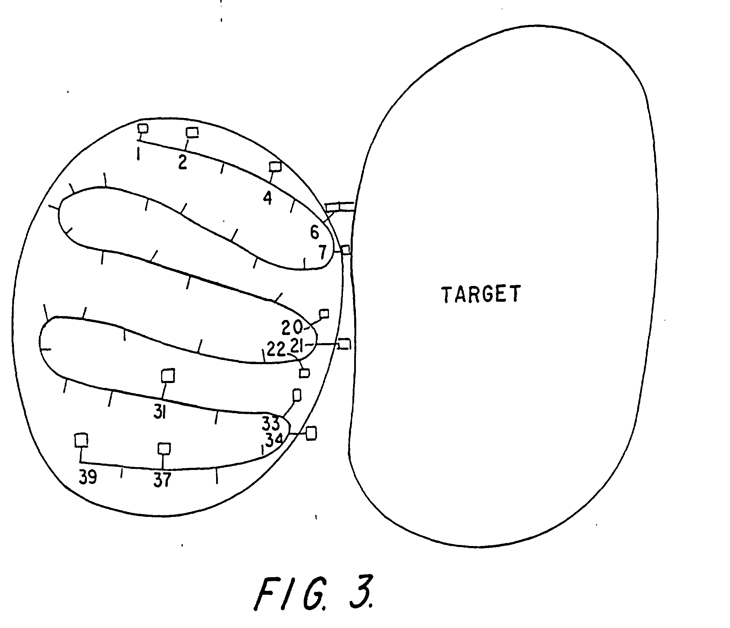

In hypothetical example 1, we consider a hypothetical PBD, shown in Figure 3 binding to a hypothetical target. Figure 3 is a 2D schematic of 3D objects; by hypothesis, residues 1, 2, 4, 6, 7, 13, 14, 15, 20, 21, 22, 27, 29, 31, 33, 34, 36, 37, 38, and 39 of the IPBD are on the 3D surface of the IPBD, even though shown well inside the circle. Proteins do not have distinct, countable faces. Therefore we define an "interaction set" to be a set of residues such that all members of the set can simultaneously touch one molecule of the target material without any atom of the target coming closer than van der Waals distance to any main-chain atom of the IPBD. The concept of a residue "touching" a molecule of the target is discussed below. One hypothetical interaction set, Set A, in Figure 3 comprises residues 6, 7, 20, 21, 22, 33, and 34, represented by squares. Another hypothetical interaction set, Set B, comprises residues 1, 2, 4, 6, 31, 37, and 39, represented by circles.

If we vary one residue, number 21 for example, through all twenty amino acids, we obtain 20 protein sequences and 20 different surfaces for interaction set A. Note that residue 6 is in two interaction sets and variation of residue 6 through all 20 amino acids yields 20 versions of interaction set A and 20 versions of interaction set B.

Now consider varying two residues, each through all twenty amino acids, generating 400 protein sequences. If the two residues varied were, for example, number 1 and number 21, then there would be only 40 different surfaces because interaction set A does not depend on residue 1 and interaction set B does not depend on residue 21. If the two residues varied, however, were number 7 and number 21, then 400 surfaces would be generated.

If N spatially separated residues are varied at one time, 20 x N surfaces are generated. Variation of N residues in the same interaction set yields $20^N$ surfaces. For example, if N = 7, variation of separated residues yields 140 surfaces while variation of interacting residues yields $20^7 = 1.28 \times 10^9$ surfaces. Thus, to maximize the number of surfaces generated when N residues are varied, all residues should be in the same interaction set.

The amount of surface area buried in strong protein-protein interactions ranges from 1000 Å$^2$ to 2000 Å$^2$ (SCHU79, p103ff). Individual amino acids have total surface areas that depend mostly on type of amino acid and weakly on conformation. These areas range from about 180 Å$^2$ for glycine to about 360 Å$^2$ for tryptophan. From amino-acid solvent exposures of published protein structures, we calculate that 1000Å$^2$ on a protein surface comprises between 4 and 30 amino-acid residues. Varied amino acid sequences, as found in actual proteins, involve between 10 and 25 residues in forming 1000 Å$^2$ of protein surface. Schulz and Schirmer estimate that 100 Å$^2$ of protein surface can exhibit as many as 1000 different specific patterns (SCHU79, p105). The number of surface patterns rises exponentially with the area that can be varied independently. One of the BPTI structures recorded in the Brookhaven Protein Data Bank (6PTI), for example, has a total exposed surface area of 3997 Å$^2$ (using the method of Lee and Richards (LEEB71) and a solvent radius of 1.4 Å and atomic radii as shown in Table 7). If we could vary this surface freely and if 100 Å$^2$ can produce 1000 patterns, we could construct $10^{120}$ different patterns by varying the surface of BPTI! This calculation is intended only to suggest the huge number of possible surface patterns based on a common protein backbone.

One protein framework cannot, however, display all possible patterns over any one particular 100 Å$^2$ of surface merely by replacement of the side groups of surface residues. The protein backbone holds the varied side groups in approximately constant locations so that the variations are not independent. We can, nevertheless, generate a vast collection of different protein surfaces by varying those protein residues that face the outside of the protein.

Examination of a model of BPTI in contact with myoglobin shows that residues 3, 7, 8, 10, 13, 39, 41, and 42 can all simultaneously contact a molecule the size and shape of myoglobin. Residue 49 cannot touch a single myoglobin molecule simultaneously with any of the first set even though all are on the surface of BPTI. It is not the intent of the present invention, however, to use models to determine which part of the target molecule will actually be the site of binding by a PBD.

For cassette mutagenesis, the protein residues to be varied are, preferably, close enough in sequence that the variegated DNA (vgDNA) encoding all of them can be made in one piece. The present invention is not limited to a particular length of vgDNA that can be synthesized. With current technology, a stretch of 60 amino acids (180 DNA bases) can be spanned.

One can use other mutational means, such as single-stranded-oligonucleotide-directed mutagenesis (BOTS85) using two or more mutating primers to mutate widely separated residues.

Alternatively, to vary residues separated by more than sixty residues, two cassettes may be mutated. A first cassette is mutagenized to produce a population having, for example, up to 30,000 members. Using variegated OCV, we mutagenize a second cassette to produce a second variegated population having the desired diversity.

The composite level of variation must not exceed the prevailing capabilities to a) produce very large numbers of independently transformed cells or b) detect small components in a highly varied population. The limits on the level of variegation are discussed in Sec. 13.2.

We assemble the data about the IPBD and the target that are useful in deciding which residues to vary 1) 3D structure, or at least a list of residues on the surface of the IPBD, 2) list of sequences homologous to IPBD, and 3) model of the target molecule or a stand-in for the target.

These data and an understanding of the behavior of different amino acids in proteins will be used to answer two questions:

1) which residues of the IPBD are on the outside and close enough together in space to touch the target simultaneously?

2) which residues of the IPBD can be varied with high probability of retaining the underlying IPBD structure?

Although an atomic model of the target material from X-ray crystallography, NMR, etc. is preferred in such examination, it is not necessary. For example, if the target were a protein of unknown 3D structure, it would be sufficient to know the molecular weight of the protein and whether it were a soluble globular protein, a fibrous protein, or a membrane protein. One can then choose a protein of known structure of the same class and similar size and shape to use as a molecular stand-in and yardstick. At low resolution, all proteins of a given size and class look much the same. The specific volumes are the same, all are more or less spherical and therefore all proteins of the same size and class have about the same radius of curvature. The radii of curvature of the two molecules determine how much of the two molecules can come into contact.

The most appropriate method of picking the residues of the protein chain at which the amino acids should be varied is by viewing, with interactive computer graphics, a model of the IPBD. A stick-figure representation of molecules is preferred. A suitable set of hardware is an Evans & Sutherland PS390 graphics terminal (Evans & Sutherland Corporation, Salt Lake City, UT) and a MicroVAX II supermicro computer (Digital Equipment Corp., Maynard, MA). Suitable programs for viewing and manipulating protein models include: a) PS-FRODO, written by T. A. Jones (JONE85) and distributed by the Biochemistry Department of Rice University, Houston, TX; and b) PROTEUS, developed by Dayringer, Tramantano, and Fletterick (DAYR86).

Theoretical calculations, such as dynamic simulations of proteins, are used to estimate the effect of substitution at a particular residue of a particular amino-acid type on the 3D structure of the parent protein. Such calculations might also indicate whether a particular substitution will greatly affect the flexibility of the protein.

Sec. 13.1.1: The principal set:

Using the knowledge of which residues are on the surface of the IPBD, we pick residues that are close enough together on the surface of the IPBD to touch a molecule of the target simultaneously without having any IPBD main-chain atom come closer than van der Waals distance (viz. 4.0 to 5.0 A) from any target atom. A residue of the IPBD "touches" the target if: a) a main-chain atom is within van der Waals distance, viz. 4.0 to 5.0 Å of any atom of the target molecule, or b) the $C_{beta}$ is within $D_{cutoff}$ of any atom of the target molecule so that a side-group atom could make contact with that atom. Because side groups differ in size (cf. Table 35), some judgment is required in picking $D_{cutoff}$. In the preferred embodiment, we will use $D_{cutoff}$ = 8.0 Å, but other values in the range 6.0 Å to 10.0 Å could be used. If IPBD has G at a residue, we construct a pseudo $C_{beta}$ with the correct bond distance and angles and judge the ability of the residue to touch the target from this pseudo $C_{beta}$.

Alternatively, we choose a set of residues on the surface of the IPBD such that the curvature of the surface defined by the residues in the set is not so great that it would prevent contact between all residues in the set and a molecule of the target. This method is appropriate if the target is a macromolecule, such as a protein, because the PBDs derived from the IPBD will contact only a part of the macromolecular surface.

We prefer that there be some indication that the underlying IPBD structure will tolerate substitutions at each residue in the principal set of residues. Indications could come from various sources, including: a) homologous sequences, b) static computer modeling, or c) dynamic computer simulations.

The residues in the principal set need not be contiguous in the protein sequence. We require only that the amino acids in the residues to be varied all be capable of touching a molecule of the target material simultaneously without having atoms overlap. If the target were, for example, horse heart myoglobin, and if the IPBD were BPTI, any set of residues in one interaction set of BPTI defined in Table 34 could be picked.

Preferably, the principal set contains eight to sixteen residues. This number of residues allows sufficient variability that a surface that is complementary to the target can be found, but is small enough that a significant fraction of the surface can be varied at one time.

Sec. 13.1.2: The secondary set:

The secondary set comprises residues that touch residues in the primary set, and are excluded from the primary set because the residue: a) is internal, b) is highly conserved, or c) is on the surface, but the curvature of the IPBD surface prevents the residue from being in contact with the target at the same time as one or more residues in the primary set.

Internal residues, although frequently conserved and may tolerate some conservative changes such as I to L or F to Y. These changes affect the detail placement and dynamics of adjacent protein residues and such variation may be useful once an SBD is found.

Surface residues in the secondary set are most often located on the periphery of the principal set, which do not make direct contact with the target simultaneously with all other residues of the principal set. The charge on the amino acid in one of these residues could, however, have a strong effect on binding. It is appropriate to vary the charge of some or all of these residues to improve an SBD. For example, the variegated codon containing equimolar A and G at base 1, equimolar C and A at base 2, and A at base 3 yields amino acids T, A, K, and E with equal probability.

Sec. 13.1.3: Choice of residues to vary initially:

The allowed level of variegation that assures progressively determines how many residues can be varied at once; geometry determines which ones.

The user picks residues to vary in many ways; the following is a preferred manner. Pairs of residues are picked that are diametrically opposed across the face of the principal set. Two such pairs are used to delimit the surface, up/down and right/left. Alternatively, three residues that form an inscribed triangle, having as large an area as possible, on the surface are picked. One to three other residues are picked in a checkerboard fashion across the interaction surface. Choice of widely spaced residues to vary creates the possibility for high specificity because all the intervening residues must have acceptable complementarity before favorable interactions can occur at widely-separated residues.

The number of residues picked is coupled to the range through which each can be varied by the restrictions discussed in Sec. 13.2. In the first round, we do not assume any binding between IPBD and the target and so progressivity is not an issue. At the first round, the user may elect to produce a level of variegation such that each molecule of vgDNA is potentially different through, for example, unlimited variegation of 10 codons ($20^{10}$ approx. = $10^{13}$). One run of the DNA synthesizer produces approximately $10^{13}$ molecules of length 100 nts. Inefficiencies in ligation and transformation will reduce the number of proteins actually tested to between $10^7$ and $5 \times 10^8$. Multiple iterations of the process with such very high levels of variegation will not yield repeatable results; the user must decide whether this is important.

Sec. 13.2: Range of variation at Each Site of Mutation:

The total level of variegation is the product of the number of variants at each varied residue. Each varied residue can have a different scheme of variegation, producing 2 to 20 different possibilities. We require that the process be progressive, i.e. each variegation cycle produces a better starting point for the next variegation cycle than the previous cycle produced.

N.B.: Setting the level of variegation such that the ppbd and many sequences related to the ppbd sequence are present in detectable amounts insures that the process is progressive. If the level of variegation is so high that the ppbd sequence is present at such low levels that there is an appreciable chance that no transformant will display the PPBD, then the best SBD of the next round could be worse than the PPBD. At excessively high level of variegation, each round of mutagenesis is independent of previous rounds and there is no assurance of progressivity. This approach can lead to valuable binding proteins, but repetition of experiments with this level of variegation will not yield progressive results. Excessive variation is not preferred.

If the level of variegation is such that the parental sequence and each single amino-acid change is present for selection, then we know that a selected sequence is closer to optimal or the same as the parent. If, on the other hand, very high levels of variegation are used, a sequence may be selected, not because it is superior to the parental sequence, but because the parental and improved sequences are, by chance; absent.

Progressivity is not an all-or-nothing property. So long as most of the information obtained from previous variegation cycles is retained and many different surfaces that are related to the PPBD surface are produced, the process is progressive. If the level of variegation is so high that the ppbd gene may not be detected, the assurance of progressivity diminishes. If the probability of recovering PPBD is negligible, then the probability of progressive behavior is also negligible.

An opposing force in our design considerations is that PBDs are useful in the population only up to the amount that can be detected; any excess above the detectable amount is wasted. Thus we produce as many surfaces related to PPBD as possible within the constraint that the PPBD be detectable.

We defer specification of exactly how much variegation is allowed until we have: a) specified real nt distributions for a variegated codon, and b) examined the effects of discrepancies between specified nt distributions and actual nt distributions.

Sec. 13.3: Design of vgDNA Encoding PBD Family:

We must now decide how to distribute the variegation within the codons for the residues to be varied. These decisions are influenced by the nature of the genetic code. When vgDNA is synthesized, variation at the first base of a codon creates a population containing amino acids from the same column of the genetic code table (as shown in the Table 3-6 on p87 of WATS87); variation at the second base of the codon creates a population containing amino acids from the same row of the genetic code table; variation at the third base of the codon creates a population containing amino acids from the same box. If two or three bases in the same codon are varied, the pattern is more complicated. Work with 3D protein structural models may suggest definite sets of amino acids to substitute at a given residue, but the method of variation may require either more or fewer kinds of amino acids be included. For example, examination of a model might suggest substitution of N or Q at a given residue. Combinatorial variation of codons requires that mixing N and Q at one location also include K and H as possibilities at the same residue. One must choose to put: 1) N only, 2) Q only, or 3) a mixture of N, K, H, and Q. The present invention does not rely on accurate predictions of which amino acids should be placed at each residue, rather attention is focused on which residues should be varied.

There are many ways to generate diversity in a protein. (See RICH86, CARU85, and OLIP86.) One extreme case is that one or a few residues of the protein are varied as much as possible (inter alia see CARU85, CARU87, RICH86, and WHAR86). We will call this limit "Focused Mutagenesis". Focused Mutagenesis is appropriate when the IPBD or other PPBD shows little or no binding to the target, as at the beginning of the search for a protein to bind to a new target material. When there is no binding between the PPBD and the target, we preferably pick a set of five to seven residues and vary each through all 20 possibilities.

An alternative plan of mutagenesis ("Diffuse Mutagenesis") is to vary many more residues through a more limited set of choices (See Vershon et al., Ch15 of INOU86 and PAKU86). This can be accomplished by spiking each of the pure nts activated for DNA synthesis (e.g. nt-phosphoramidites) with a small amount of one or more of the other activated nts. Contrary to general practice, the present invention sets the level of spiking so that only a small percentage ( 1% to .00001%, for example ) of the final product contains the initial DNA sequence. Many single, double, triple, and higher mutations occur, but recovery of the basic sequence is a possible outcome. Let $N_b$ be the number of bases to be varied, and let Q be the fraction of all sequences that should have the parental sequence, then M, the fraction of the mixture that is the majority component, is

$$M = \exp\{ \log_e(Q)/N_b \} = 10 (\log_{10}(Q)/N_b).$$

If, for example, thirty base pairs on the DNA chain were to be varied and 1% of the product is to have the parental sequence, then each mixed nt substrate should contain 86% of the parental nt and 14% of other nts. Table 8 shows the fraction (fn) of DNA molecules having n non-parental bases when 30 bases are synthesized with reagents that contain fraction M of the majority component. When M=.63096, f24 and higher are less than $10^{-8}$. The entry "most" in Table 8 is the number of changes that has the highest probability. Note that substantial probability for multiple substitutions only occurs if the fraction of parental sequence (f0) is allowed to drop to around $10^{-6}$. Mutagenesis of this soft can be applied to any part of the protein at any time, but is most appropriate when some binding to the target has been established. The $N_b$ base pairs of the DNA chain that are synthesized with mixed reagents need not be contiguous. They are picked so that between $N_b/3$ and $N_b$ codons are affected to various degrees. The residues picked for mutation are picked with reference to the 3D structure of the IPBD, if known. For example, one might pick all or most of the residues in the principal and secondary set. We may impose restrictions on the extent of variation at each of these residues based on homologous sequences or other data. The mixture of non-parental nts need not be random, rather mixtures can be biased to give particular amino acid types specific probabilities of appearance at each codon. For example, one residue may contain a hydrophobic amino acid in all known homologous sequences; in such a case, the first and third base of that codon would be varied, but the second would be set to T. This diffuse structure-directed mutagenesis will reveal the subtle changes possible in protein backbone associated with conservative interior changes, such as V to I, as well as some not so subtle changes that require concomitant changes at two or more residues of the protein.

For Focused Mutagenesis, we now consider the distribution of nts that will be inserted at each variegated codon. Each codon could be programmed differently. If we have no information indicating that a particular amino acid or class of amino acid is appropriate, we strive to substitute all amino acids with equal probability because representation of one pbd above the detectable level is wasteful. Equal amounts of all four nts at each position in a codon yields the amino acid distribution in which each amino acid is present in proportion to the number of codons that code for it. This distribution has the disadvantage of giving two basic residues for every acidic residue. In addition, six times as much R, S, and L as W or M occur. If five codons are synthesized with this distribution, sequences encoding five Rs are 7776-times more abundant than sequences encoding five Ws. To have W-W-W-W-W present at detectable levels, we must have R-R-R-R-R present in 7776-fold excess.

Let Abun(x) be the abundance of DNA sequences coding for amino acid x, defined by the distribution of nts at each base of the codon. For any distribution, there will be a most-favored amino acid (mfaa) with abundance Abun(mfaa) and a least-favored amino acid (lfaa) with abundance Abun(lfaa). We seek the nt distribution that allows all twenty amino acids and that yields the largest ratio Abun(lfaa)/Abun(mfaa) subject to two constraints: equal abundances of acidic and basic amino acids and the least possible number of stop codons. Thus only nt distributions that yield  Abun(E)+Abun(D) = Abun(R)+Abun(K)  are considered, and the function maximized is:

$$\{(1-\text{Abun(stop)}) (\text{Abun(lfaa)/Abun(mfaa)})\}.$$

We have simplified the search for an optimal nt distribution by limiting the third base to T or G (C or G is equivalent). All amino acids are possible and the number of accessible stop codons is reduced because TGA and TAA codons are eliminated. The amino acids F, Y, C, H, N, I, and D require T at the third base while W, M, Q, K, and E require G. Thus we use an equimolar mixture of T and G at the third base.

A computer program, written as part of the present invention and named "Find Optimum vgCodon" (See Table 9), varies the composition at bases 1 and 2, in steps of 0.05, and reports the composition that gives the largest value of the quantity {(Abun(lfaa)/Abun(mfaa) (1-Abun(stop)))} . A vg codon is symbolically defined by the nt distribution at each base:

|  | T | C | A | G |
|---|---|---|---|---|
| base #1 = | t1 | c1 | a1 | g1 |
| base #2 = | t2 | c2 | a2 | g2 |
| base #3 = | t3 | c3 | a3 | g3 |

$$t1 + c1 + a1 + g1 = 1.0$$

$$t2 + c2 + a2 + g2 = 1.0$$

$$t3 = g3 = 0.5, c3 = a3 = 0.$$

The variation of the quantities t1, c1, a1, g1, t2, c2, a2, and g2 is subject to the constraint that Abun(E)+Abun(D) equals Abun(K)+Abun(R);

$$Abun(E)+Abun(D) = g1*a2$$

$$Abun(K)+Abun(R) = a1*a2/2 + c1*g2 + a1*g2/2$$

$$g1*a2 = a1*a2/2 + c1*g2 + a1*g2/2$$

Solving for g2, we obtain

$$g2 = (g1*a2 - 0.5*a1*a2)/(c1 + 0.5*a1) .$$

In addition,

$$t1 = 1 - a1 - c1 - g1$$

$$t2 = 1 - a2 - c2 - g2 .$$

We vary a1, c1, g1, a2, and c2 and then calculate t1, g2, and t2. Initially, variation is in steps of 5%. Once an approximately optimum distribution of nts is determined, the region is further explored with steps of 1%. The logic of this program is shown in Table 9. The optimum distribution is:

Optimum vgCodon

|         | T    | C    | A    | G    |
|---------|------|------|------|------|
| base #1 = | 0.26 | 0.18 | 0.26 | 0.30 |
| base #2 = | 0.22 | 0.16 | 0.40 | 0.22 |
| base #3 = | 0.5  | 0.0  | 0.0  | 0.5  |

and yields DNA molecules encoding each type amino acid with the abundances shown in Table 10.

The computer that controls a DNA synthesizer, such as the Milligen 7500, can be programmed to synthesize any base of an oligo-nt with any distribution of nts by taking some nt substrates (e.g. nt phosphoramidites) from each of two or more reservoirs. Alternatively, nt substrates can be mixed in any ratios and placed in one of the extra reservoir for so called "dirty bottle" synthesis.

The actual nt distribution obtained will differ from the specified nt distribution due to several causes, including: a) differential inherent reactivity of nt substrates, and b) differential deterioration of reagents. It is possible to compensate partially for these effects, but some residual error will occur. We denote the average discrepancy between specified and observed nt fraction as $S_{err}$,

$$S_{err} = \text{square root} ( \text{average}[ (f_{obs} - f_{spec})/f_{spec} ] )$$

were $f_{obs}$ is the amount of one type of nt found at a base and $f_{spec}$ is the amount of that type of nt that was specified at the same base. The average is over all specified types of nts and over a number (e.g. 10 or 20) different variegated bases. By hypothesis, the actual nt distribution at a variegated base will be within 5% of the specified distribution. Actual DNA synthesizers and DNA synthetic chemistry may have different error levels. It is the user's responsibility to deter-

mine $S_{err}$ for the DNA synthesizer and chemistry employed.

To determine the possible effects of errors in nt composition on the amino-acid distribution, we modified the program "Find optimum vgCodon" in four ways:

1) the fraction of each nt in the first two bases is allowed to vary from its optimum value times $(1 - S_{err})$ to the optimum value times $(1 + S_{err})$ in seven equal steps ($S_{err}$ is the hypothetical fractional error level entered by the user); the sum of nt fractions at one base always equals 1.0,

2) g2 is varied in the same manner as a2, i.e. we dropped the restriction that Abun(D)+Abun(E) = Abun(K)+Abun(R) ,

3) t3 and g3 are varied from 0.5 times $(1 - S_{err})$ to 0.5 times $(1 + S_{err})$ in three equal steps,

4) the smallest ratio Abun(lfaa)/Abun(mfaa) is sought.

In actual experiments, we will direct the synthesizer to produce the optimum DNA distribution "Optimum vgCodon" given above. Incomplete control over DNA chemistry may, however, cause us to actually obtain the following distribution that is the worst that can be obtained if all nt fractions are within 5% of the amounts specified in "Optimum vgCodon". A corresponding table can be calculated for any given $S_{err}$ using the program "Find worst vgCodon within Serr of given distribution." given in Table 11.

Optimum vgCodon, worst 5% errors

|  | T | C | A | G |
|---|---|---|---|---|
| base #1 = | 0.251 | 0.189 | 0.273 | 0.287 |
| base #2 = | 0.209 | 0.160 | 0.400 | 0.231 |
| base #3 = | 0.475 | 0.0 | 0.0 | 0.525 |

This distribution yields DNA encoding different amino acids at the abundances shown in Table 12.
If five codons are synthesized with reagents mixed so as to produce the nt-distribution "Optimum vgCodon", and it we actually obtained the nt-distribution "Optimum vgCodon, worst 5% errors", then DNA sequences encoding the mfaa at all of the five codons are about 277 times as likely as DNA sequences encoding the lfaa at all of the five codons; about 24% of the DNA sequences will have a stop codon in one or more of the five codons.
When five codons are synthesized using equimolar mixtures at bases 1 and 2, $(\text{Abun(mfaa)/Abun(lfaa)})^5 = 7776$. If we program the optimum nt distribution and come within 5%, then $(\text{Abun(mfaa)/Abun(lfaa)})^5 = 277$. The total number of different PBDs is unchanged, but the least-favored sequence is about 28 times more abundant. Detecting the least-favored amino-acid sequence when varying four residues with equimolar nts at each varied base requires as sensitive a separation system as does detecting the least-favored amino-acid sequence when varying five residues with the optimized nt distribution.
By hypothesis, the distribution "Optimal vgCodon" is used in the second version of the second variegation of hypothetical example 2. The abundance of the DNA encoding each type of amino acid is, however, taken from the Table 12. The abundance of DNA encoding the parental amino acid sequence is:

```
Amount(parental seq.)
        F24         G30         D34         E42         T47
    = Abun(F) * Abun(G) * Abun(D) * Abun(E) * Abun(T)
    =  .0249  x  .0663  x  .0545  x  .0602  x  .0437
    = 2.4 x 10⁻⁷
```

Therefore, DNA encoding the PPBD sequence as well as very many related sequences will be present in sufficient quantity to be detected and we are assured that the process will be progressive.

A level of variegation that allows recovery of the PPBD has two properties:

1) we cannot regress because the PPBD is available,

2) an enormous number of multiple changes related to the PPBD are available for selection and we are able to detect and benefit from these changes.

The user must adjust the list of residues to be varied and levels of variegation at each residue until the calculated variegation is within the bounds set by $M_{ntv}$ and $C_{sensi}$.

Preferably, we also consider the interactions between the sites of variegation and the surrounding DNA. If the method of mutagenesis to be used is replacement of a cassette, we consider whether the variegation will generate gratuitous restriction sites and whether they seriously interfere with the intended introduction of diversity. We reduce or eliminate gratuitous restriction sites by appropriate choice of variegation pattern and silent alteration of codons neighboring the sites of variegation. See the Detailed Example.

Sec. 14.1: Insertion of synthetic vgDNA into a Plasmids:

For cassette mutagenesis, restriction sites were designed and synthesized, and are used to introduce the synthetic vgDNA into the OCV. Restriction digestions and ligations are performed by standard methods (AUSU87). In the case of single-stranded-oligonucleotide-directed mutagenesis, synthetic vgDNA is used to create diversity in the vector (BOTS85).

Sec. 14.2: Transformation of cells:

The present invention is not limited to any one method of transforming cells with DNA. Standard methods, such as thos described in MANI82, may be optimized for the particular host cells and OCV. The goal is to produce a large number of independent transformants, preferably $10^7$ of more. It is not necessary to isolate transformed cells between transformation and affinity separation. We prefer to have transformed cells at high concentration so that they can be plated densely on relatively few plates.

Sec. 14.3: Growth of the GP(vgPBD) population:

The transformed cells are grown first under non-selective conditions that allow expression of plasmid genes and then selected to kill untransformed cells.

Transformed cells are then induced to express the osp-pbd gene at the appropriate level of induction, as determined in Sec. 10.1. The GPs carrying the IPBD are harvested by a method appropriate to the package.

A high level of diversity can be generated by in vitro variegated synthesis of DNA and this diversity can be maintained passively through several generations in an organism without positive selective pressure. Loss or reduction in frequency of deleterious mutations is advantageous for the purposes of the present invention. It is preferable that the selection is must be performed before more than a few generations elapse. Moreover, subdividing the variegated population before amplification in an organism by removing a small sample (less than 10%) for further work would result in loss of diversity; therefore, one should use all or most of the synthetic DNA and most or all of the transformed cells.

Sec. 15.: Isolation of GP(PBD)s with binding-to-target phenotypes :

The harvested packages are enriched for the binding-to-target phenotype by use of affinity separation involving target material immobilized on a matrix. Packages that fail to bind to target material are washed away. If the packages are bacteriophage or endospores, it may be desirable to include a bacteriocidal agent, such as azide, in the buffer to prevent bacterial growth.

Sec. 15.1: Attaching the target material to a column:

Affinity column chromatography is the preferred method of affinity separation, but other affinity separation methods may be used. A variety of commercially available support materials for affinity chromatography are used. These include derivatized beads to which the target material is covalently linked, or non-derivatized material to which the target material adheres irreversibly.

Suppliers of support material for affinity chromatography include: Applied Protein Technologies Cambridge, MA; Bio-Rad Laboratories, Rockville Center, NY; Pierce Chemical Company, Rockford, IL. Target materials are attached to the matrix in accord with the directions of the manufacturer of each matrix preparation with consideration of good presentation of the target.

Sec. 15.2: Reducing selection due to non-specific binding:

We reduce non-specific binding of GP(PBD)s to the matrix that bears the target in two ways:

1) we treat the column with blocking agents such as genetically defective GPs or a solution of protein before the population of GP(vgPBD)s is chromatographed, and

2) we pass the population of GP(vgPBD)s over a matrix containing no target or a different target from the same class as the actual target prior to affinity chromatography.

Step (1) above saturates any non-specific binding that the affinity matrix might show toward wild-type GPs or proteins in general; step (2) removes components of our population that exhibit non-specific binding to the matrix or to molecules of the same class as the target. If the target were horse heart myoglobin, for example, a column supporting bovine serum albumin could be used to trap GPs exhibiting PBDs with strong non-specific binding to proteins. If cholesterol were the target, then a hydrophobic compound, such as p-tertiarybutylbenzyl alcohol, could be used to remove GPs displaying PBDs having strong non-specific binding to hydrophobic compounds. It is anticipated that PBDs that fail to fold or that are prematurely terminated will be non-specifically sticky. The capacity of the initial column that removes indiscriminately adhesive PBDs should be greater (e.g. 5 fold greater) than the column that supports the target molecule.

Variation in the support material (polystyrene, glass, agarose, etc.) in analysis of clones carrying SBDs is used to eliminate enrichment for packages that bind to the support material rather than the target.

Sec. 15.3: Eluting the column:

The population of GPs is applied to an affinity matrix under conditions compatible with the intended use of the binding protein and the population is fractionated by passage of a gradient of some solute over the column. The process enriches for PBDs having affinity for the target and for which the affinity for the target is least affected by the eluants used.

Ions or cofactors needed for stability of PBDs (derived from IPBD) or target must be included in buffers at appropriate levels. We first remove GP(PBD)s that do not bind the target by washing the matrix with the volume of the initial buffer required to bring the optical density (at 260 nm or 280 nm) back to base line plus one to five void volumes ($V_V$). The column is then eluted with a gradient of increasing: a) salt, b) [H+] (decreasing pH), c) neutral solutes, d) temperature (increasing or decreasing), or e) some combination of these factors. Salt is the most preferred solute for gradient formation. Other solutes that generally weaken non-covalent interaction may also be used. "Salt" includes solutions containing any of the following ionic species:

| Na+ | K+ | Ca++ | Mg++ |
|---|---|---|---|
| $NH_4$+ | Li+ | Sr++ | Ba++ |
| Rb+ | Cs+ | Cl- | Br- |
| $SO_4$-- | $HSO_4$- | $PO_4$--- | $HPO_4$-- |
| $H_2PO_4$- | $CO_3$-- | $HCO_3$- | Acetate |
| Citrate | Standard l-Amino Acids | Standard nucleotides | Guanidinium Cl |

Other ionic or neutral solutes may be used. All solutes are subject to the necessity that they not kill the genetic packages. Neutral solutes, such as ethanol, acetone, ether, or urea, are frequently used in protein purification, however, many of these are very harmful to bacteria and bacteriophage above low concentrations. Bacterial spores, on the other hand, are impervious to most neutral solutes. Several passes may be made through the steps in Sec. 15. Different solutes may be used in different analyses, salt in one, pH in the next, etc.

Sec. 15.4: Recovery of packages:

Recovery of packages that display binding to an affinity column may be achieved in several ways, including from:

1) fractions eluted with a gradient as described above;
2) fractions eluted with soluble target material,
3) cells grown in situ on the matrix,
4) cells incubated with parts of the matrix,
5) fractions eluted after chemically or enzymatically degrading the linkage holding the target to the matrix, and
6) regeneration of GPs after degrading the packages and recovering OCV DNA.

It is possible to utilize combinations of these methods. It should be remembered that what we want to recover from the affinity matrix is not the GPs per se, but the information in them. Recovery of viable GPs is very strongly preferred, but recovery of genetic material is essential.

Inadvertent inactivation of the GPs is very deleterious. It is preferred that maximum limits for solutes that do not inactivate the GPs or denature the target or the column are determined. One may use conditions that denature the column to elute GPs; before the target is denatured, a portion of the affinity matrix should be removed for possible use as an inoculum. As the GPs are held together by protein-protein interactions and other non-covalent molecular interactions, there will be cases in which the molecular package will bind so tightly to the target molecules on the affinity matrix that the GPs can not be washed off in viable form. This will only occur when very tight binding has been obtained. In these cases, methods (3) through (5) above can be used to obtain the bound packages or the genetic messages from the affinity matrix.

It is possible, by manipulation of the elution conditions, to isolate SBDs that bind to the target at one pH ($pH_b$) but not at another pH ($pH_o$). The population is applied at $pH_b$ and the column is washed thoroughly at $pH_b$. The column is then eluted with buffer at $pH_o$ and GPs that come off at the new pH are collected and cultured. Similar procedures may be used for other solution parameters, such as temperature. For example, GP(vgPBD)s could be applied to a column supporting insulin. After eluting with salt to remove GPs with little or no binding to insulin, we elute with salt and glucose to liberate GPs that display PBDs that bind insulin or glucose in a competitive manner.

Sec. 15.5: Amplifying the Enriched Packages

Viable GPs having the selected binding trait are amplified by culture in a suitable medium, or, in the case of phage, infection into a host so cultivated. If the GPs have been inactivated by the chromatography, the OCV carrying the osp-pbd gene must be recovered from the GP, and introduced into a new, viable host.

Sec. 15.6: Determining whether further enrichment is needed:

The probability of isolating a GP with improved binding increases by $C_{eff}$ with each separation cycle. Let N be the number of distinct amino-acid sequences produced by the variegation. We want to perform K separation cycles before attempting to isolate an SBD, where K is such that the probability of isolating a single SBD is 0.10 or higher.

$$K = \text{the smallest integer} \geq \log_{10}(0.10\,N)/\log_{10}(C_{eff})$$

For example, if N were $1.0 \times 10^7$ and $C_{eff} = 6.31 \times 10^2$ then $\log_{10}(1.0 \times 10^6)/\log_{10}(6.31 \times 10^2) = 6.0000/2.8000 = 2.14$. Therefore we would attempt to isolate SBDs after the third separation cycle. After only two separation cycles, the probability of finding an SBD is $(6.31 \times 10^2)^2/(1.0 \times 10^7) = .04$ and attempting to isolate SBDs might be profitable.

Clonal isolates from the last fraction eluted in Sec. 15.3 containing any viable GPs, as well as clonal isolates obtained by culturing an inoculum taken from the affinity matrix, are cultured. If K separation cycles have been completed, samples from a number, e.g. 32, of these clonal isolates are tested for elution properties on the (target) column. If none of the isolated, genetically pure GPs show improved binding to target, or if K cycles have not yet been completed, then we pool and culture, in a manner similar to the manner set forth in Sec. 14.3, the GPs from the last few fractions eluted (see Sec. 15.4) that contained viable GPs and from the GPs obtained by culturing an inoculum taken from the column matrix. We then repeat the enrichment procedure described in Sec. 15. This cyclic enrichment may continue $N_{chrom}$ passes or until an SBD is isolated.

If one or more of the isolated GPs has improved retention on the (target) column, we determine whether the retention of the candidate SBDs is due to affinity for the target material. Target material is attached to a different support matrix at optimal density and the elution volumes of candidate GP(SBD)s are measured. We pick the candidate that either has the highest elution volume or that is retained on the column after elution. If none of the candidate GP(SBD)s has higher elution volume than GP(PPBD of this round), then we pool and culture the GPs from the last few fractions that contained viable GPs and the GPs obtained by culturing an inoculum taken from the column matrix. We then repeat the enrichment procedure of Sec. 15.

If all of the SBDs show binding that is superior to PPBD of this round, we pool and culture the GPs from the last fraction that contains viable GPs and from the inoculum taken from the column. This population is re-chromatographed at least one pass to fractionate further the GPs based on $K_d$.

If an RNA phage were used as GP, the RNA would either be cultured with the assistance of a helper phage or be reverse transcribed and the DNA amplified. The amplified DNA could then be sequenced or subcloned into suitable plasmids.

Sec. 15.7: Characterizing the Population:

We characterize members of the population showing desired binding properties by genetic biochemical methods. We obtain clonal isolates and test these strains by genetic and affinity methods to determine genotype and phenotype with respect to binding to target. For several genetically pure isolates that show binding, we demonstrate that the binding is caused by the artificial chimeric gene by excising the osp-sbd gene and crossing it into the parental GP. We also ligate the deleted backbone of each GP from which the osp-sbd is removed and demonstrate that each backbone alone cannot confer binding to the target on the GP. We sequence the osp-sbd gene from several clonal isolates.

Sec. 15.8: Testing of binding affinity:

For one or more clonal isolates, we subclone the sbd gene fragment, without the osp fragment, into an expression vector such that each SBD can be produced as a free protein. Each SBD protein is purified by normal means, including affinity chromatography. Physical measurements of the strength of binding are then made on each free SBD protein by one of the following methods: 1) alteration of the Stokes radius as a function of binding of the target material, measured by characteristics of elution from a molecular sizing column such as agarose, 2) retention of radiolabeled SBD on a spun affinity column to which has been affixed the target material, or 3) retention of radiolabeled target material on a spun affinity column to which has been affixed the SBD. The measurements of binding for each free SBD are compared to the corresponding measurements of binding for the PPBD.

In each assay, we measure the extent of binding as a function of concentration of each protein, and other relevant physical and chemical parameters.

In addition, the SBD with highest affinity for the target from each round is compared to the best SBD of the previous round (IPBD for the first round) and to the IPBD with respect to affinity for the target material. Successive rounds of mutagenesis and selection-through-binding yield increasing affinity until desired levels are achieved.

If binding is not yet sufficient, we must decide which residues to vary next (see Sec. 16.0).

Sec. 15.9: Other Affinity Separation Means:

FACs may be used to separate GPs that bind fluorescent labeled target with the optimized parameters determined in Part II. We discriminate against artifactual binding to the fluorescent lable by using two or more different dyes, chosen to be structurally different.

Electrophoretic affinity separation uses unaltered target so that only other ions in the buffer can give rise to artifactual binding. Artifactual binding to the gel material gives rise to retardation independent of field direction and so is easily eliminated. A variegated population of GPs will have a variety of charges.

First the variegated population of GPs is electrophoresed in a gel that contains no target material. The electrophoresis continues until the GPs are distributed along the length of the lane. The target-free lane in which the initial electrophoresis is conducted is separated by a removable baffle from a square of gel that contains target material. The baffle is removed and a second electrophoresis is conducted at right angles to the first. GPs that do not bind target migrate with unaltered mobility while GPs that do bind target will separate from the majority that do not bind target. A diagonal line of non-binding GPs will form. This line is excised and discarded. Other parts of the gel are dissolved and the GPs cultured.

Sec. 16.0: The Next Variegation Cycle:

Which residues of the PBD should be varied in the next variegation cycle? The general rule is to preserve as much accumulated information as possible. The amino acids just varied are the ones best determined. The environment of other residues has changed, so that it is appropriate to vary them again. Because there are always more residues in the principal and secondary sets than can be varied simultaneously, we start by picking residues that either have never been varied (highest priority) or that have not been varied for one or more cycles. If we find that varying all the residues except those varied in the previous cycle does not allow a high enough level of diversity, then residues varied in the previous cycle might be varied again. For example, if the number of independent transformants that can be produced and the sensitivity of the affinity separation were such that seven residues could be varied, and if the principal and secondary sets contained 13 residues, we would always vary seven residues, even though that implies varying some residue twice in a row. In such cases, we would pick the residues just varied that contain the amino acids of highest abundance in the variegated codons used.

It is the accumulation of information that allows the process to select those protein sequences that produce binding between the SBD and the target. Some interfaces between proteins and other molecules involve twenty or more residues. Complete variation of twenty residues would generate $10^{26}$ different proteins. By dividing the residues that lie close together in space into overlapping groups of five to seven residues, we can vary a large surface but never need to test more than $10^7$ to $10^9$ candidates at once, a savings of $10^{19}$ to $10^{17}$ fold.

Having picked the residues to vary, we again set the range of variegation for each residue according to the principles set forth in 13.2, design the vgDNA encoding the desired mutants (Sec. 13.3), clone the vgDNA into GPs (Sec. 14), and select-by-binding-to-target those GPs bearing SBDs (Sec. 15).

Sec. 17.0: OTHER CONSIDERATIONS:

Sec. 17.1: Joint selections:

One may modify the affinity separation of the method described to select a molecule that binds to material A but not to material B. One needs to prepare two selection columns, one with material A and the other with material B. The population of genetic packages is prepared in the manner described, but before applying the population to A, one passes the population over the B column so as to remove those members of the population that have high affinity for B. It may be necessary to amplify the population that does not bind to B before passing it over A. Amplification would most likely be needed if A and B were in some ways similar and the PPBD has been selected for having affinity for A.

For example, to obtain an SBD that binds A but not B, three columns could be connected in series: a) a column supporting some compound, neither A nor B, or only the matrix material, b) a column supporting B, and c) a column supporting A. A population of GP(vgPBD)s is applied to the series of columns and the columns are washed with the buffer of constant ionic strength that is used in the application. The columns are uncoupled, and the third column is eluted with a gradient to isolate GP(PBD)s that bind A but not B.

One can also generate molecules that bind to both A and B. In this case we use a 3D model and mutate one face of the molecule in question to get binding to A. We then mutate a different face to produce binding to B.

The materials A and B could be proteins that differ at only one or a few residues. For example, A could be a natural protein for which the gene has, been cloned and B could be a mutant of A that retains the overall 3D structure of A. SBDs selected to bind A but not B must bind to A near the residues that are mutated in B. If the mutations were picked to be in the active site of A (assuming A has an active site), then an SBD that binds A but not B will bind to the active site of A and is likely to be an inhibitor of A.

To obtain a protein that will bind to both A and B, we can, alternatively, first obtain an SBD that binds A and a different SBD that binds B. We can then combine the genes encoding these domains so that a two-domain single-polypeptide protein is produced. The fusion protein will have affinity for both A and B.

One can also generate binding proteins with affinity for both A and B, such that these materials compete for the same site on the binding protein. We guarantee competition by overlapping the sites for A and B. We first create a molecule that binds to target material A. We then vary a set of residues defined as: a) those residues that were varied to obtain binding to A, plus b) those residues close in 3D space to the residues of set (a) but that are internal and so are unlikely to bind directly to either A or B. Residues in set (b) are likely to make small changes in the positioning of the residues in set (a) such that the affinities for A and B will be changed by small amounts. Members of these populations are selected for affinity to both A and B.

Sec. 17.2: Selection for non-binding:

The method of the present invention can be used to select proteins that do <u>not</u> bind to selected targets. Consider a protein of pharmacological importance, such as streptokinase, that is antigenic to an undesirable extent. We can take the pharmacologically important protein as IPBD and antibodies against it as target. Residues on the surface of the pharmacologically important protein would be variegated and GP(PBD)s that do not bind to an antibody column would be collected several ways, including: a) from a 3D structure, b) from hydrophobicity considerations, or c) chemical labeling. The 3D structure of the pharmacologically important protein remains the preferred guide to picking residues to vary, except now we pick residues that are widely spaced so that we leave as little as possible of the original surface unaltered.

Destroying binding frequently requires only that a single amino acid in the binding interface be changed. If polyclonal antibodies are used, we face the problem that all or most of the strong epitopes must be altered in a single molecule. Preferably, one would have a set of monoclonal antibodies, or a narrow range of antibody species. If we had a series of monoclonal antibody columns, we could obtain one or more mutations that abolish binding to each monoclonal antibody. We could then combine some or all of these mutations in one molecule to produce a pharmacologically important protein recognized by none of the monoclonal antibodies. Such mutants must be tested to verify that the pharmacologically interesting properties have not be altered to an unacceptable degree by the mutations.

Typically, polyclonal antibodies display a range of binding constants for antigen. Even if we have only polyclonal antibodies that bind to the pharmacologically important protein, we may proceed as follows. We engineer the pharmacologically important protein to appear on the surface of a replicable GP. We introduce mutations into residues that are on the surface of the pharmacologically important protein or into residues thought to be on the surface of the pharmacologically important protein so that a population of GPs is obtained. Polyclonal antibodies are attached to a column and the population of GPs is applied to the column at low salt. The column is eluted with a salt gradient. The GPs that elute at the lowest concentration of salt are those which bear pharmacologically important proteins that have been mutated in a way that eliminates binding to the antibodies having maximum affinity for the pharmacologically important protein. The GPs eluting at the lowest salt are isolated and cultured. The isolated SBD becomes the PPBD to further rounds of variegation so that the antigenic determinants are successively eliminated.

Sec. 17.3: Selection of PBDs for retention of structure:

We can select for insertions or deletions that preserve the 3D structure of known binding proteins. Consider on GP that express BPTI on its surface. In the bpti-osp gene, we can replace the codons for K26 and A27 with five variegated codons ($3.2 \times 10^6$ sequences). K26 and A27 are in a turn and are far from the trypsin binding surface. We use selection-through-binding to isolate GPs expressing mutants of BPTI that retain high, specific affinity for trypsin.

Sec. 17.4: Created binding proteins not unique:

For each target, there are a large number of SBDs that may be found by the method of the present invention. To increase the probability that some PBD in the population will bind to the target, we generate as large a population as we can conveniently subject to selection-through-binding. Key questions in management of the method are "How many transformants can we produce?", and "How small a component can we find through selection-through-binding?". Geneticists routinely find mutations with frequencies of one in $10^{10}$ using simple, powerful selections. The optimum level of variegation is determined by the maximum number of transformants and the selection sensitivity, so that for any reasonable sensitivity we may use a progressive process to obtain a series of proteins with higher and higher affinity for the chosen target material. Enrichments of 1000-fold by a single pass of elution from an affinity plate have been demonstrated (SMIT85).

Use of different variation schemes can yield different binding proteins. For any given target, a large plurality of proteins will bind to it. Thus, if one binding protein turns out to be unsuitable for some reason (e.g. too antigenic), the procedure can be repeated with different variation parameters. For example, one might choose different residues to vary or pick a different nt distribution at variegated codons so that a new distribution of amino acids is tested at the same residues. Even if the same principal set of residues is used, one might obtain a different SBD if the order in which one picks subsets to be varied is altered.

Sec. 17.5: Other modes of mutagenesis possible:

The modes of creating diversity in the population of GPs discussed herein are not the only modes possible. Any method of mutagenesis that preserves at least a large fraction of the information obtained from one selection and then introduces other mutations in the same domain will work. The limiting factors are the number of independent transformants that can be produced and the amount of enrichment one can achieve through affinity separation. Therefore the preferred embodiment uses a method of mutagenesis that focuses mutations into those residues that are most likely to affect the binding properties of the PBD and are least likely to destroy the underlying structure of the IPBD.

Other modes of mutagenesis might allow other GPs to be considered. For example, the bacteriophage lambda is not a useful cloning vehicle for cassette mutagenesis because of the plethora of restriction sites. One can, however, use single-stranded-oligo-nt-directed mutagenesis on lambda without the need for unique restriction sites. No one has used single-stranded-oligo-nt-directed mutagenesis to introduce the high level of diversity called for in the present invention, but if it is possible, such a method would allow use of phage with large genomes.

Example 1

BPTI-Derived Binding Protein for HHMb; Displayed by M13 Phage

Presented below is a hypothetical example of a protocol for developing a new binding molecule derived from BPTI with affinity for horse heart myoglobin (HHMb) using the common E. coli bacteriophage M13 as genetic package. It will be understood that some further optimization, in accordance with the teachings herein, may be necessary to obtain the desired results. Possible modifications in the preferred method are discussed immediately following various steps of the hypothetical example.

By hypothesis, we set the following technical capabilities:

$Y_{DQ}$     500 ng/synthesis of ssDNA 100 bases long,
        10 ug/synthesis of ssDNA 60 bases long,
        1 mg/synthesis of ssDNA 20 bases long.

$M_{DNA}$     100 bases

$Y_{pl}$     1 mg/l

$L_{ef}$     0.1 % for blunt-blunt,
        4 % for sticky-blunt,
        11 % for sticky-sticky.

$M_{ntv}$     $5 \times 10^8$

$C_{eff}$     900-fold enrichment

$C_{sensi}$     1 in $4 \times 10^8$

$N_{chrom}$     10 passes

$S_{err}$     0.05

Example 1, Part I

In this example, we will use M13 as a replicable GP and BPTI as IPBD. In Part I, we are concerned only with getting BPTI displayed on the outer surface of an M13 derivative. Variable DNA may be introduced in the osp-ipbd gene, but not within the region that codes for the trypsin-binding region of BPTI. Once BPTI is displayed on the M13 outer surface of an M13 derivative, we proceed to Part II to optimize the affinity separation procedures.

For this example, we choose a filamentous bacteriophage of E. coli, M13. We prefer phage over vegetative bacterial cells because phage are much less metabolically active. We prefer phage over spores because the molecular mechanisms of the virion formation and 3D structure of the virion are much better understood than are the corresponding processes of spore formation and structures of spores.

M13 is a very well studied bacteriophage, widely used for DNA sequencing and as a genetic vector; it is a typical member of the class of filamentous phages. The relevant facts about M13 and other phages that will allow us to choose among phages are cited in Sec. 1.3.1.

Compared to other bacteriophage, filamentous phage in general are attractive and M13 in particular is especially attractive because:

1) the 3D structure of the virion is known,

2) the processing of the coat protein is well understood,

3) the genome is expandable,

4) the genome is small,

5) the sequence of the genome is known,

6) the virion is physically resistant to shear, heat, cold, guanidinium Cl, low pH, and high salt,

7) the phage is a sequencing vector so that sequencing is especially easy, and

8) antibiotic-resistance genes have been cloned into the genome with predictable results (HINE80).

Other criteria listed in Sec. 1.0 and 1.3 of the are also satisfied: M13 is easily cultured and stored (FRIT85), each infected cell yielding 100 to 1000 M13 progeny after infection. M13 has no unusual or expensive media requirements and is easily harvested and concentrated (SALI64, YAMA70, FRIT85). M13 is stable toward physical agents: temperature (10% of phage survive 30 minutes at 85°C), shear (Waring blender does not kill), desiccation (not applicable), radiation (not applicable), age (stable for years).

M13 is stable toward chemicals: pH (< 2.2 (SMIT85)), surface active agents: not applicable, chaotropes (guanidinium HCl = 6.0 M), ions (no specific sensitivities), organic solvents (ether and other organic solvents are lethal (MARV78)), proteases (not applicable, HHMb not a protease). M13 is not known to be sensitive to other enzymes.

M13 genome is 6423 b.p. and the sequence is known (SCHA78). Because the genome is small, cassette mutagenesis is practical on RF M13 (AUSU87), as is single-stranded oligo-nt directed mutagenesis (FRIT85). M13 is a plasmid and transformation system in itself, and an ideal sequencing vector. M13 can be grown on Rec⁻ strains of E. coli. The M13 genome is expandable (MESS78, FRIT85). M13 confers no advantage, but doesn't lyse cells. The sequence of gene VIII is known, and the amino acid sequence can be encoded on a synthetic gene, using lacUV5 promoter and used in conjunction with the LacI$^q$ repressor. The lacUV5 promoter is induced by IPTG. Gene VIII protein is secreted by a well studied process and is cleaved between A23 and A24. Residues 18, 21, 22, and 23 of gene VIII protein control cleavage. Mature gene VIII protein makes up the sheath around the circular ssDNA. The 3D structure of f1 virion is known at medium resolution; the amino terminus of gene VIII protein is on surface of the virion. No fusions to M13 gene VIII protein have been reported. The 2D structure of M13 coat protein is implicit in the 3D structure. Mature M13 gene VIII protein has only one domain. There are four minor proteins: gene III, VI, VII, and IX. Each of these minor proteins is present in about 5 copies per virion and is related to morphogenesis or infection. The major coat protein is present in more than 2500 copies per virion.

Although no fusions of M13 gene VIII to other genes have been reported, knowledge of the virion 3D structure (BANN810) makes attachment of IPBD to the amino terminus of mature M13 coat protein (M13 CP) quite attractive. Should direct fusion of BPTI to M13 CP fail to cause BPTI to be displayed on the surface of M13, we will vary part of the BPTI sequence and/or insert short random DNA sequences between BPTI and M13 CP.

Smith (SMIT85) and de la Cruz et al. (CRUZ88) have shown that insertions into gene III cause novel protein domains to appear on the virion outer surface. If BPTI can not be made to appear on the virion outer surface by fusing the bpti gene to the m13cp gene, we will fuse bpti to gene III either at the site used by Smith and by de la Cruz et al. or to one of the termini. We will use a second, synthetic copy of gene III so that some unaltered gene III protein will be present.

The gene VIII protein is chosen as OSP because it is present in many copies and because its location and orientation in the virion are known. Note that any uncertainty about the azimuth of the coat protein about its own alpha helical axis is unimportant.

The 3D model of f1 indicates strongly that fusing BPTI to the amino terminus of M13 CP is more likely to yield a functional protein than any other fusion site. (See Sec. 1.3.3).

The amino-acid sequence of M13 pre-coat (SCHA78), called AA_seg1, is

```
                     AA_seg1

           1    1    2  | |2    3    3    4    4    5
      5    0    5    0  \ /5    0    5    0    5    0
     MKKSLVLKASVAVATLVPMLSFAAEGDDPAKAAFNSLQASATEYIGYAWA

          5    6    6    7    7
          5    0    5    0    3
     MVVVIVGATIGIKLFKKFTSKAS
```

The single-letter codes for amino acids and the codes for ambiguous DNA are internationally recognized (GEOR87). The best site for inserting a novel protein domain into M13 CP is after A23 because SP-I cleaves the pre-coat protein after A23, as indicated by the arrow. Proteins that can be secreted will appear connected to mature M13 CP at its amino terminus. Because the amino terminus of mature M13 CP is located on the outer surface of the virion, the introduced domain will be displayed on the outside of the virion.

BPTI is chosen as IPBD of this example (See Sec. 2.1) because it meets or exceeds all the criteria: it is a small, very stable protein with a well known 3D structure. Marks et al. (MARK86) have shown that a fusion of the phoA signal peptide gene fragment and DNA coding for the mature form of BPTI caused native BPTI to appear in the periplasm of E. coli, demonstrating that there is nothing in the structure of BPTI to prevent its being secreted.

Marks et al. (MARK87) also showed that the structure of BPTI is stable even to the removal of one of the cystine bridges. They did this by replacing both C14 and C38 with either two alanines or two threonines. The C14/C38 cystine bridge that Marks et al. removed is the one very close to the scissile bond in BPTI; surprisingly, both mutant molecules functioned as trypsin inhibitors. This indicates that BPTI is redundantly stable and so is likely to fold into approximately the same structure despite numerous surface mutations. Using the knowledge of homologues, vide infra, we can infer which residues must not be varied if the basic BPTI structure is to be maintained.

The 3D structure of BPTI has been determined at high resolution by X-ray diffraction (HUBE77, MARQ83, WLOD84, WLOD87a, WLOD87b), neutron diffraction (WLOD84), and by NMR (WAGN87). In one of the X-ray structures deposited in the Brookhaven Protein Data Bank, "6PTI", there was no electron density for A58, indicating that A58 has no uniquely defined conformation. Thus we know that the carboxy group does not make any essential interaction in the folded structure. The amino terminus of BPTI is very near to the carboxy terminus. Goldenberg and Creighton reported on circularized BPTI and circularly permuted BPTI (GOLD83). Some proteins homologous to BPTI have more or fewer residues at either terminus.

BPTI has been called "the hydrogen atom of protein folding" and has been the subject of numerous experimental and theoretical studies (STAT87, SCHW87, GOLD83, CHAZ83).

BPTI has the added advantage that at least 32 homologous proteins are known, as shown in Table 13. A tally of ionizable groups is shown in Table 14 and the composite of amino acid types occurring at each residue is shown in Table 15.

BPTI is freely soluble and is not known to bind metal ions. BPTI has no known enzymatic activity. EPTI binds to trypsin, $K_d = 6.0 \times 10^{-14}$ M (TSCH87). BPTI is not toxic. If K15 of BPTI is changed to L, there is no measurable binding between the mutant BPTI and trypsin (TSCH87).

All of the conserved residues are buried; of the seven fully conserved residues only G37 has noticeable exposure. The solvent accessibility of each residue in BPTI is given in Table 16 which was calculated from the entry "6PTI" in the Brookhaven Protein Data Bank with a solvent radius of 1.4 A, the atomic radii given in Table 7, and the method of Lee and Richards (LEEB71). Each of the 51 non-conserved residues can accommodate two or more kinds of amino acids. By independently substituting at each residue only those amino acids already observed at that residue, we could obtain approximately $7 \times 10^{42}$ different amino acid sequences, most of which will fold into structures very similar to BPTI.

BPTI will be useful as a IPBD for macromolecules. (See Sec. 2.1.1) BPTI and BPTI homologues bind tightly and with high specificity to a number of enzymes.

BPTI is strongly positively charged except at very high pH, thus BPTI is useful as IPBD for targets that are not also strongly positive under the conditions of intended use (see Sec. 2.1.2). There exist homologues of BPTI, however, having quite different charges (viz. SCI-III from Bombyx mori at -7 and the trypsin inhibitor from bovine colostrum at -1). Once a derivative of M13 is found that displays BPTI on its surface, the sequence of the BPTI domain can be replaced by one of the homologous sequences to produce acidic or neutral IPBDs.

BPTI is not an enzyme (See Sec. 2.1.3). BPTI is quite small; if this should cause a pharmacological problem, two or more BPTI-derived domains may be joined as in the human BPTI homologue that has two domains.

A derivative of M13 is the preferred OCV. (See Sec. 3). A "phagemid" is a hybrid between a phage and a plasmid, and is used in this invention. Double-stranded plasmid DNA isolated from phagemid-bearing cells is denoted by the standard convention, e.g. pXY24. Phage prepared from these cells would be designated XY24. Phagemids such as Bluescript K/S (sold by Stratagene) are not suitable for our purposes because Bluescript does not contain the full genome of M13 and must be rescued by coinfection with helper phage. Such coinfections could lead to genetic recombination yielding heterogeneous phage unsuitable for the purposes of the present invention.

The bacteriophage M13 bla 61 (ATCC 37039) is derived from wild-type M13 through the insertion of the beta lactamase gene (HINE80). This phage contains 8.13 kb of DNA. M13 bla cat 1 (ATCC 37040) is derived from M13 bla 61 through the additional insertion of the chloramphenicol resistance gene (HINE80); M13 bla cat 1 contains 9.88 kb of DNA. Although neither of these variants of M13 contains the ColE1 origin of replication, either could be used as a starting point to construct a usable cloning vector for the present example.

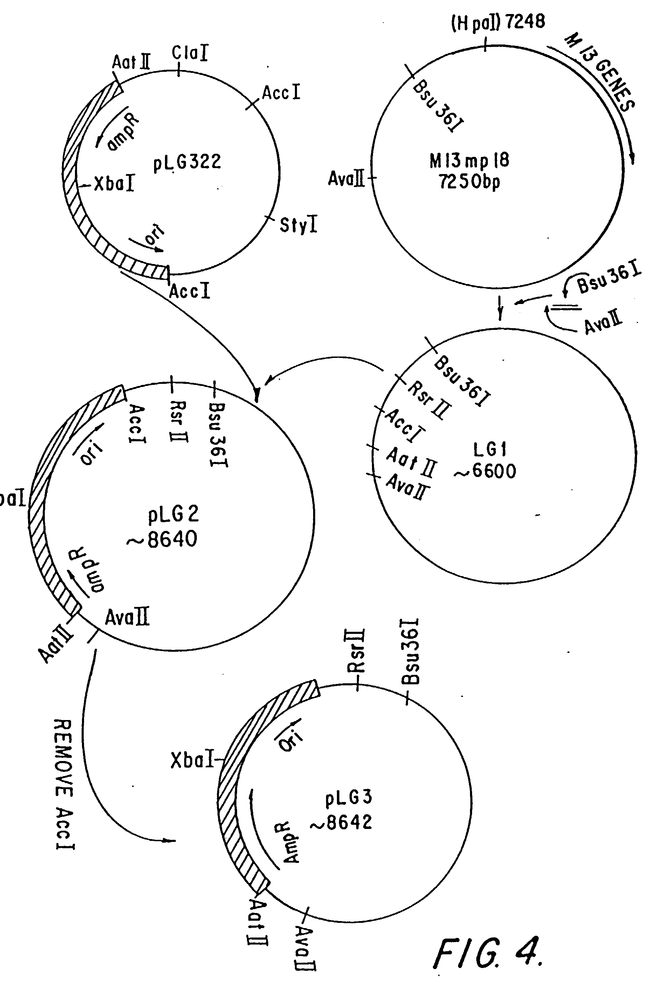

The OCV for the current example is constructed by a process illustrated in Figure 4. A brief description of all the plasmids and phagemids constructed for this Example is found in Table 17.

For ss oligo-nt site-directed mutagenesis, multiple primers lead to higher efficiency. Three non-mutagenic primers are used: bases 2326-2352 of wt M13, bases 4854-4875 of wt M13, and the complement of bases 3431-3451 of pBR322. Note that pLG2 and its derivatives carry the anti-sense strand of the amp$^R$ gene in the + DNA strand. The segments are picked to be high in GC content and to divide the pLG7 genome into several segments of approximately-equal length.

The genetic engineering procedures needed to construct the OCV are standard, using commercially available restriction enzymes under recommended conditions. All restriction fragments of DNA are purified by electrophoresis or HPLC. M13 and its engineered derivatives are infected into E. coli strain PE384 (F$^+$,Rec$^-$,Sup$^+$,Amp$^S$). Plasmid DNA of M13 derivatives is transformed into E. coli strain. PE383(F$^-$,Rec$^-$,Sup$^+$,Amp$^S$) so that we avoid multiple rounds of infection in the culture. Isolation of M13 phage is by the procedure of Salivar et al. (SALI64); isolation of replicative form (RF) M13 is by the procedure of Jazwinski et al. (JAZW73a and JAZW73b). Isolation of plasmids containing the ColE1 origin of replication is by the method of Maniatis (MANI82).

We pick the amp$^R$ gene from pBR322 as a convenient antibiotic resistance gene. Another resistance gene, such as kanamycin, could be used. The Acc I-to-Aat II fragment of pBR322 is a conveniently obtained source of any$^R$ and the Col E1 origin.

M13mp18 (New England BioLabs) contains neither Aat II nor Acc I sites. Therefore we insert an adaptor that allows us to insert the Aat II-to-Acc I fragment of pBR322 that carries the amp$^R$ gene and the ColE1 origin of replication into a desirable place in M13mp18. M13mp18 contains a lacUV5 promoter and a lacZ gene that are not useful to the purposes of the present invention. By cutting M13mp18 with AvaII and Bsu36I and discarding the approximately 600 intervening base pairs, we eliminate all recognition sites of several enzymes useful for engineering the bpti-gene VIII gene.

The following adaptor is synthesized,

```
5' GACCGACGTCtgcctcGTATACCGGACCGcatagctCC      3' olig#1
3'    GCTGCAGacggagCATATGGCCTGGCgtatcgaGGACT 5' olig#2
 AvaII|AatII|       |AccI|RsrII |         |Bsu36I
```

The annealed adaptor is ligated with RF M13mp18 that has been cut with both AvaII and Bsu36I and purified by PAGE or HPLC. Transformed cells are selected for plasmid uptake with ampicillin. The resulting construct is called pLG1.

DNA from pLG1 is cut with both Aat II and Acc I. AatII-to-AccI fragment of pBR322 is ligated to the backbone of LG1. The correct construct is named pLG2.

The Acc I restriction site is no longer needed for vector construction. To eliminate this site, RF pLG2 dsDNA is cut with Acc I, treated with Klenow fragment and dATP and dTTP to make it blunt and then religated. The cloning vector, named pLG3, is now ready for stepwise insertion of the osp-ipbd gene.

We are now ready to design a gene (See Sec. 4) that will cause BPTI-domains to appear on the outer surface of an M13 derivative: LG7.

To obtain a novel protein domain attached to the outside of M13, we insert DNA that codes for mature BPTI after A23 of the precoat protein of M13. Mature BPTI begins with an arginine residue, which is charged; cleavage by signal peptidase I is normal in such cases. Signal peptidase I (SP-I) cuts a chimera of M13 coat protein and BPTI after A23 leaving mature BPTI attached at its carboxy end to the amino terminus of M13 CP.

The following amino-acid sequence, called AA_seq2, is constructed, by inserting the sequence for mature BPTI (shown underscored) immediately after the signal sequence of M13 precoat protein (indicated by the arrow) and before the sequence for the M13 CP.

## AA_seq2

```
         1    1    2   ||2    3    3    4    4    5
    5    0    5    0   \/5    0    5    0    5    0
    MKKSLVLKASVAVATLVPMLSFARPDFCLEPPYTGPCKARIIRYFYNAKA


         5    6    6    7    7    8    8    9    9   10
    5    0    5    0    5    0    5    0    5    0
    GLCQTFVYGGCRAKRNNFKSAEDCMRTCGGAAEGDDPAKAAFNSLQASAT


        10   11   11   12   12   13
    5    0    5    0    5    0
    EYIGYAWAMVVVIVGATIGIKLFKKFTSKAS
```

Sequence numbers of fusion proteins refer to the fusion, as coded, unless otherwise noted. Thus the alanine that begins M13 CP is referred to as "number 82", "number 1 of M13 CP", or "number 59 of the mature BPTI-M13 CP fusion".

The osp-ipbd gene is regulated by the lacUV5 promoter and terminated by the trpA transcription terminator. The host strain of E. coli harbors the lacI^q gene. The osp-ipbd gene is expressed and processed in parallel with the wild-type gene VIII. The novel protein, that consists of BPTI tethered to a M13 CP domain, constitutes only a fraction of the coat. Affinity separation is able to separate phage carrying only five or six copies of a molecule that has high affinity for an affinity matrix (SMIT85); 1% incorporation of the chimeric protein results in about 30 copies of the protein exposed on the surface. If this is insufficient, additional copies may be provided by, for example, increasing IPTG.

A model comprising M13 coat, after the model for f1 of Marvin and colleagues (BANN81), and a BPTI domain, taken from the Brookhaven Protein Data Bank entry "6PTI", was constructed by standard model building methods that insure that covalent bond lengths and angles are close to acceptable values. The model shows that the fusion protein could fit into the supramolecular structure in a stereochemically acceptable fashion without disturbing the internal structure of either the M13 CP or BPTI domain.

The ambiguous DNA sequence coding for AA_seq2, is examined by a computer program for places where recognition sites for restriction enzymes could be created without altering the amino-acid sequence. (See Sec. 4.3). A master table of enzymes is compiled from the catalogues of enzyme suppliers. The enzymes that do not cut the OCV. (Preferably constructed as described above).

Using the procedure given in Sec. 4.3, we design a ipbd gene, such as that shown in Table 25. Some restriction enzymes (e.g. Ban I or Hph I) cut the OCV too often to be of value.

The entire DNA sequence of the m13cp-bpti fusion with annotation appears in Table 25 showing the useful restriction sites and biologically important features, viz. the lacUV5 promoter, the lacO operator, the Shine-Dalgarno sequence, the amino acid sequence, the stop codons, and the transcriptional terminator.

The ipbd gene is synthesized in several steps using the method described in Sec. 5.1, generating dsDNA fragments of 150 to 190 base pairs.

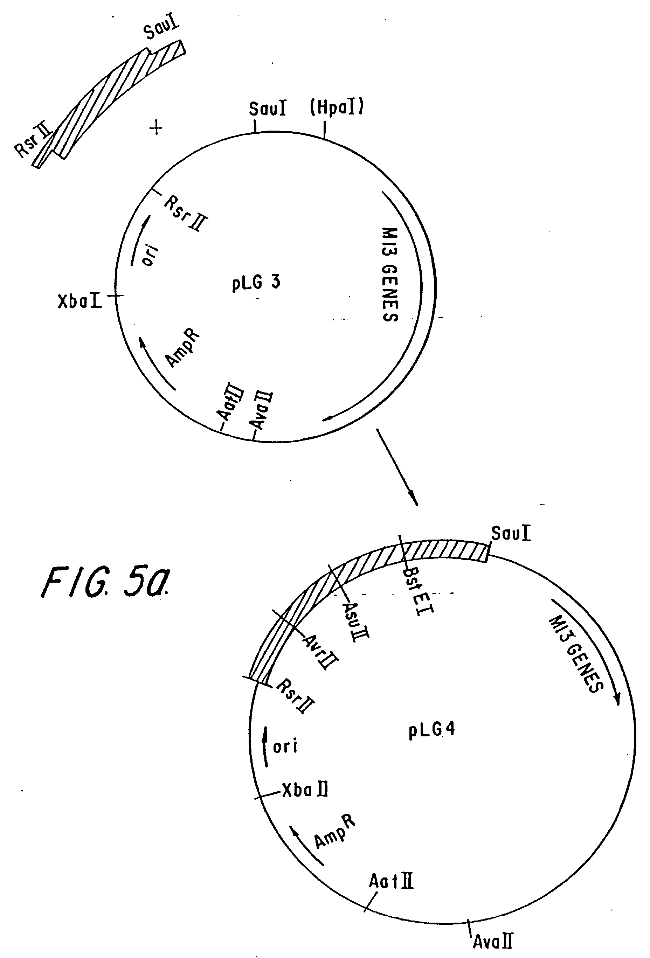

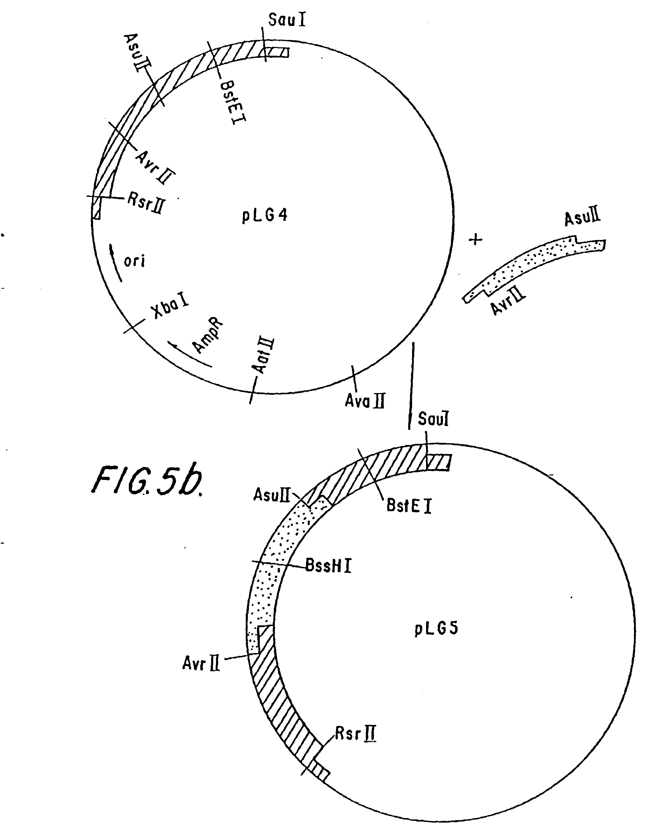

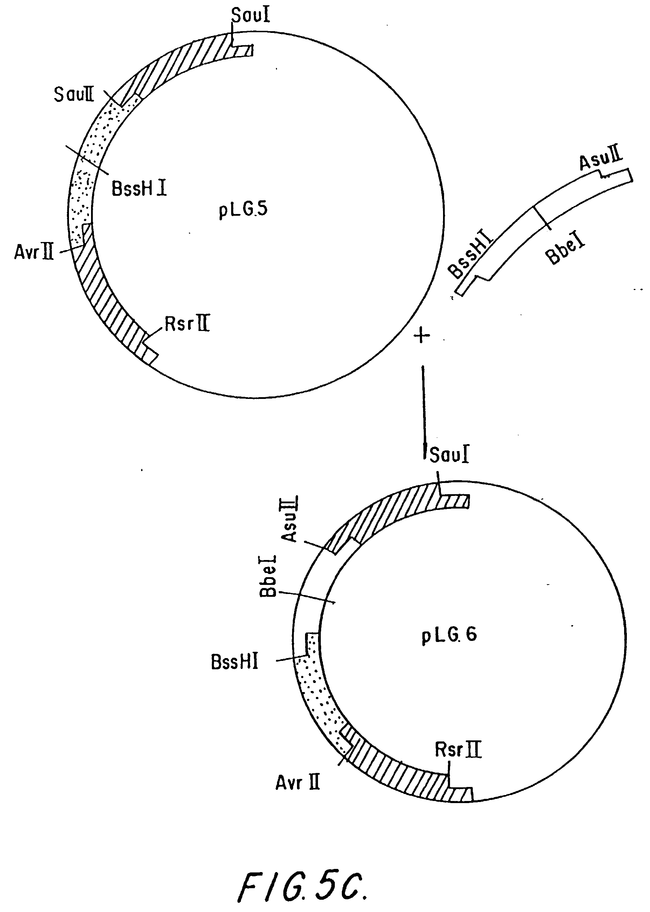

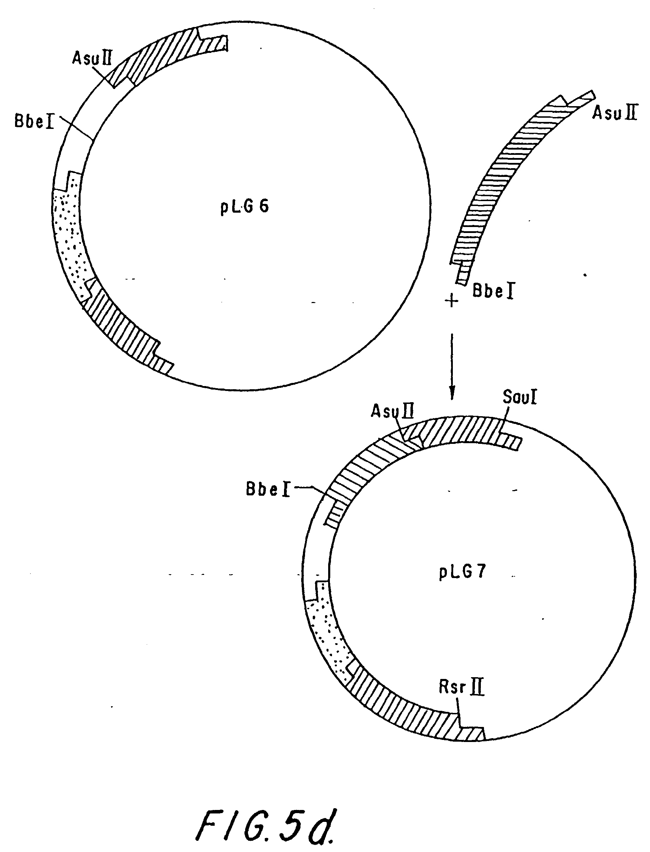

The four steps (See Sec. 6.1) by which we clone synthetic fragments of the m13cp-bpti gene (the osp-ipbd gene of the present example) into pLG3 and its derivatives are illustrated in Figure 5.

The sequence to be introduced into pLG3 comprises a) the segment from RsrII to AvrII (Table 25), b) a spacer sequence (gccgctcc), and c) the segment from AsuII to SauI. The segment is 158 bases long and is synthesized from two shorter synthetic oligo-nts as described in Sec. 5.1 of the generic specification.

Table 27 shows the antisense strand of the sequence to be inserted. The 99 base fragment shown in upper case letters and underscored (5'-CCGTCC....CCTTCG-3' = olig#3) is synthesized in the standard manner. Similarly, the 100 base long fragment of the sense strand shown in lower case (5'-cgctca....aattg-3' = olig#4) is synthesized. After annealing, the double-stranded region is extended with Klenow fragment by the procedure given above to make the entire 176 bases double stranded. The overlap region is 23 base pairs long and contains 14 CG pairs and 9 AT pairs. The DNA between AvrII and AsuII does not code for anything in the final pbd gene; it is there so that the DNA can be cut by both AvrII and AsuII at the same time in the next step. Eight bases have been added to the left of RsrII and nine bases have been added to the left of SauI (same specificity and cutting pattern as Bsu36I). These bases at the ends are not part of the final product; they must be present so that the restriction enzymes can bind and cut the synthetic DNA to produce specific sticky ends.

The synthetic DNA is cut with both SauI and RsrII and is ligated to similarly cut dsDNA of pLG3. The construct with the correct insert is called pLG4.

The second step of the construction of the OCV is illustrated in Table 28. As in the construction of pLG4, two pieces of single-stranded DNA are synthesized: a 99 base long fragment of the anti-sense strand ending with p25 and a 99 base long fragment (starting with p18). Both the synthetic dsDNA and dsRF pLG4 DNA are cut with both AvrII and AsuII and are ligated and used to transform E. coli. The construct carrying this second insert is called pLG5.

Construction of pLG6 proceeds similarly to the construction of pLG5. The sequence is shown in Table 30. The two single stranded segments (one from the anti-sense strand ending with N66 and the other from the sense strand starting with the third base of the codon for Y58) are synthesized, annealed, and extended with Klenow fragment. Both the synthetic DNA and RF pLG5 are cut with both BssHI and AsuII, purified, and the appropriate pieces are ligated and used to transform E. coli.

The construction of pLG7 is illustrated in Table 32 and proceeds similarly to the constructions of pLG4, pLG5, and pLG6. The two single stranded segments (one from the anti-sense strand ending with the first base of the codon for V110 and the other beginning with E101) are synthesized, annealed, and extended with Klenow fragment. Both the synthetic DNA and RF pLG6 are cut with both BbeI and AsuII, purified, and the appropriate pieces are ligated and used to transform E. coli. The construct with the correct fourth insert is called pLG7; the display of BPTI on the outer surface of LG7 is verified by the methods of Sec. 8.

M13am429 is an amber mutation of M13 used to reduce non-specific binding by the affinity matrix for phages derived from M13. M13am429 is derived by standard genetic methods (MILL72) from wtM13.

Phage LG7 is grown on E. coli strain PE384 in LB broth with various concentrations of IPTG added to the medium to induce the osp-ipbd gene. Phage LG7 is obtained from cells grown with 0.0, 0.1, 1.0, 10.0 or 100.0 uM, or 1.0 mM IPTG, harvested (See Sec. 7) by the method of Salivar (SALI64), and concentrated to obtain a titre of $10^{12}$ pfu/ml by the method of Messing (MESS83).

The preferred method of determining whether LG7 displays BPTI on its surface (See Sec. 8) is to determine whether these phage can retain a labeled derivative of trypsin (trp) or anhydrotrypsin (AHTrp) on a filter that allows passage of unbound trp or AHTrp. Trypsin contains 10 tyrosine residues and can be iodinated with $^{125}I$ by standard methods; we denote the labeled trypsin as "trp*". Labeled anhydrotrypsin is denoted as "AHTrp*". Other types of labels can be used on trp or AHTrp, e.g. biotin or a fluorescent label. AHTrp* or trp* is labeled to an activity of 0.3 uCi/ug. A sample of $10^{12}$ LG7(10 mM IPTG) is mixed with 1.0 ug of trp* or AHTrp* in 1.0 ml of a buffer of 10 mM KCl, adjusted to pH 8.0 with 1 mM $K_2HPO_4$ / $KH_2PO_4$. The mixture is passed through an Amicon MSP1 system fitted with a membrane filter that allows passage of proteins smaller that $M_r = 300,000$. Filters are soaked in buffer containing trp or AHTrp prior to the analysis. The filter is washed twice with 0.5 ml of buffer containing trp or AHTrp. The radioactivity retained on the filter is quantitated with a scintillation counter or other suitable device. If each virion displays one copy of BPTI, then .05 ug of protein can be bound that would give rise to $3 \times 10^4$ disintegrations / minute on the filter.

An alternative way to quantitate display of BPTI on the surface of LG7 is to use the stoichiometric binding between trypsin and BPTI to titrate the BPTI. A solution that titers $10^{12}$ pfu/ml of a phage is approximately $1.6 \times 10^{-9}$ M in phage if each virion is infective. The ratio of pfu to total phage can be determined spectrophotometrically using the molar extinction coefficients at 260 nm and 280 nm corrected for the increased length of LG7 as compared to wtM13.

For example, if a 1.0 ml solution that contains $10^{12}$ pfu of LG7 phage grown with 1.0 mM IPTG inhibits trypsin solutions up to $4.8 \times 10^{-7}$ M, we calculate that there are approximately 300 BPTIs/GP (i.e. ($4.8 \times 10^{-7}$ molecules of BPTI/l)/($1.6 \times 10^{-9}$ phage/l)). Inhibition of a specified concentration of trypsin is most easily measured spectrophotometrically using a peptide-linked dye, such as $N_{alpha}$-benzoyl-Arg-Nan (TSCH87).

Alternatively, binding to an affinity column may be used to demonstrate the presence of BPTI on the surface of phage LG7. An affinity column of 2.0 ml total volume having BioRad Affi-Gel 10$^{(TM)}$ matrix and 30 mg of AHTrp as affinity material is prepared by the method of BioRad. The void volume ($V_V$) of this column is, by hypothesis, 1.0 ml. This affinity column is denoted (AHTrp).

A sample of $10^{12}$ M13am429 is applied to (AHTrp) in 1.0 ml of 10 mM KCl buffered to pH 8.0 with $KH_2PO_4$ / $K_2HPO_4$. The column is then washed with the same buffer until the optical density at 280 nm of the effluent returns to base line or 4 x $V_V$ have been passed through the column, whichever comes first. Samples of LG7 or LG10 are then applied to the blocked (AHTrp) column at $10^{12}$ pfu/ml in 1.0 ml of the same buffer. The column is then washed again with the same buffer until the optical density at 280 nm of the effluent returns to base line or 4 x $V_V$ have been passed through, whichever comes first. Following this wash, a gradient of KCl from 10 mM to 2 M in 3 x $V_V$, buffered to pH 8.0 with phosphate is passed over the column. The first KCl gradient is followed by a KCl gradient running from 2 M to 5 M in 3 x $V_V$. The second KCl gradient is followed by a gradient of guanidinium Cl from 0.0 M to 2.0 M in 2 x $V_V$ in 5 M KCl and buffered to pH 8.0 with phosphate. Fractions of 50 ul are collected and assayed for phage by plating 4 ul of each fraction at suitable dilutions on sensitive cells. Retention of phage on the column is indicated by appearance of LG7 phage in fractions that elute significantly later from the column than control phage LG10 or wtM13. A successful isolate of LG7 that displays BPTI is identified, the bpti insert and junctions are sequenced, and this isolate is used for further work described below.

If vgDNA is used to obtain a functional fusion between a BPTI mutant and M13 CP (vide infra), then DNA from a clonal isolate is sequenced in the regions that were variegated. Then gratuitous restriction sites for useful restriction enzymes are removed if possible by silent codon changes. The sequence numbers of residues in OSP-IPBD will be changed by any insertions; hereinafter, we will, however, denote residues inserted after residue 23 as 23a, 23b, etc. Insertions after residue 81 will be denoted as 81a, 81b, etc. This preserves the numbering of residues between C5 and C55 of BPTI. Residue C5 of BPTI is always denoted as 28 in the fusion; residue C55 of BPTI is always denoted as 78 in the fusion, and the intervening residues have constant numbers.

Should LG7 phage from cells grown with 10 mM IPTG fail to display BPTI on its surface, we have several options. We might try to determine why the construction failed to work as expected. There are various possible modes of failure, including : a) BPTI is not cleaved from the M13 signal sequence, b) BPTI is cleaved from the M13 CP, and c) the chimeric protein is made and cleaved after the signal sequence, but the processed protein is not incorporated into the M13 coat. BPTI has been secreted from E. coli (MARK86); however the M13 coat-protein signal sequence was not used. Therefore problems stemming from the signal sequence are unlikely, but possible. We could determine whether BPTI was present in the periplasm or bound to the inner membrane of LG7-infected cells by assays using try* or Antry*.

Proteins in the periplasm can be freed through spheroplast formation using lysozyme and EDTA in a concentrated sucrose solution (BIRD67, MALA64). If BPTI were free in the periplasm, it would be found in the supernatant. Try* would be mixed with supernatant and passed over a non-denaturing molecular sizing column and the radioactive fractions collected. The radioactive fractions would then be analyzed by SDS-PAGE and examined for BPTI-sized bands by silver staining.

Spheroplast formation exposes proteins anchored in the inner membrane. Spheroplasts are mixed with AHTrp* and then either filtered or centrifuged to separate them from unbound AHTrp*. After washing with hypertonic buffer, the spheroplasts are analyzed for extent of AHTrp* binding alternatively, membrane proteins are analyzed by western blot analysis.

If BPTI is found free in the periplasm, then we would expect that the chimeric protein was being cleaved both between BPTI and the M13 mature coat sequence and between BPTI and the signal sequence. In that case, we should alter the BPTI/M13 CP junction by inserting vgDNA at codons for residues 78-82 of AA_seq2.

If BPTI is found attached to the inner membrane, then there are two likely explanations. The first is that the chimeric protein is being cut after the signal sequence, but is not being incorporated into LG7 virion; the treatment would also be to insert vgDNA between residues 78 and 82 of AA_seq2. The alternative hypothesis is that BPTI could fold and react with trypsin even if signal sequence is not cleaved. N-terminal amino acid sequencing of trypsin-binding material isolated from cell homogenate determines what processing is occurring. If signal sequence were being cleaved, we would use the procedure above to vary residues between C78 and A82; subsequent passes would add residues after residue 81. If signal sequence were not being cleaved, we would vary residues between 23 and 27 of AA_seq2. Subsequent passes through that process would add residues after 23.

If BPTI were found neither in the periplasm nor on the inner membrane, then we would expect that the fault was in the signal sequence or the signal-sequence-to-BPTI junction. The treatment in this case would be to vary residues between 23 and 27.

Several experiments that introduce variegation into the bpti-gene VIII fusion are possible, including:

1) 3 variegated codons between residues 78 and 82 using olig#12 and olig#13,

2) 3 variegated codons between residues 23 and 27 using olig#14 and olig#15,

3) 5 variegated codons between residues 78 and 82 using olig#13 and olig#12a,

4) 5 variegated codons between residues 23 and 27 using olig#15 and olig#14a,

5) 7 variegated codons between residues 78 and 82 using olig#13 and olig#12b, and

6) 7 variegated codons between residues 23 and 27 using olig#15 and olig#14b.

To alter the BPTI-M13 CP junction, we introduce DNA variegated at codons for residues between 78 and 82 into the Sph I and Sfi I sites of pLG7. The residues after the last cysteine are highly variable in amino acid sequences homologous to BPTI, both in composition and length; in Table 25 these residues are denoted as G79, G80, and A81. The first part of the M13 CP is denoted as A82, E83, and G84. One of the oligo-nts olig#12, olig#12a, or olig#12b and the primer olig#13 are synthesized by standard methods. The oligo-nts are:

```
residue   75  76  77  78  79  80  81  82  83
5' gc|gag|cGC|ATG|CGT|ACC|TGC|qfk|qfk|qfk|GCT|GAA|-

          84  85  86  87  88  89  90  91
         GGT|GAT|GAT|CCG|GCC|AAA|GCG|GCC|gcg|cc 3' olig#12


residue   75  76  77  78  79  80  81 81a 81b
5' gc|gag|cGC|ATG|CGT|ACC|TGC|qfk|qfk|qfk|qfk|qfk|-

          82  83  84  85  86  87
         GCT|GAA|GGT|GAT|GAT|CCG|-

                 88  89  90  91
                GCC|AAA|GCG|GCC|gcg|cc 3' olig#12a


residue   75  76  77  78  79  80  81 81a 81b
5' gc|gag|cGC|ATG|CGT|ACC|TGC|qfk|qfk|qfk|qfk|qfk|-

         81c 81d 82  83  84  85  86  87
         qfk|qfk|GCT|GAA|GGT|GAT|GAT|CCG|-

                 88  89  90  91
                GCC|AAA|GCG|GCC|gcg|cc 3' olig#12b


 residue   91  90  89  88  87  86
 5' gg|cgc|GGC|CGC|TTT|GGC|CGG|ATC 3'    olig#13
```

where q is a mixture of (0.26 T, 0.18C, 0.26 A, and 0.30 G), f is a mixture of (0.22 T, 0.16 C, 0.40 A, and 0.22 G), and k is a mixture of equal parts of T and G. The bases shown in lower case at either end are spacers and are not incorporated into the cloned gene. The primer is complementary to the 3' end of each of the longer oligo-nts. One of the variegated oligo-nts and the primer olig#13 are combined in equimolar amounts and annealed. The dsDNA is completed with all four (nt)TPs and Klenow fragment. The resulting dsDNA and RF pLG7 are cut with both Sfi I and Sph I, purified, mixed, and ligated. This ligation mixture goes through the process described in Sec. 15 in which we select a transformed clone that, when induced with IPTG, binds AHTrp.

To vary the junction between M13 signal sequence and BPTI, we introduce DNA variegated at codons for residues between 23 and 27 into the Kpn I and Xho I sites of pLG7. The first three residues are highly variable in amino acid sequences homologous to BPTI. Homologous sequences also vary in length at the amino terminus. One of the oligo-nts olig#14, olig#14a, or olig#14b and the primer olig#15 are synthesized by standard methods. The oligo-nts are:

```
residue :      17  18  19  20  21  22  23  24  25
5'  g|gcc|gcG|GTA|CCG|ATG|CTG|TCT|TTT|GCT|qfk|qfk|-

       26  27  28  29  30
      |qfk|TTC|TGT|CTC|GAG|cgc|ccg|cga|  3'  olig#14
```

```
residue     17  18  19  20  21  22  23  24  25  26
5'g|gcc|gcG|GTA|CCG|ATG|CTG|TCT|TTT|GCT|qfk|qfk|qfk|-

     26a 26b 27  28  29  30
    |qfk|qfk|TTC|TGT|CTC|GAG|cgc|ccg|cga|  3'  olig#14a,
```

```
residue     17  18  19  20  21  22  23  24  25  26
5'g|gcc|gcG|GTA|CCG|ATG|CTG|TCT|TTT|GCT|qfk|qfk|qfk|-

  26a 26b 26c 26d 27  28  29  30
 |qfk|qfk|qfk|qfk|TTC|TGT|CTC|GAG|cgc|ccg|cga|3'olig#14b
```

```
5'    |tcg|cgg|gcg|CTC|GAG|ACA|GAA|  3'  olig#15
```

where q is a mixture of (0.26 T, 0.18 C, 0.26 A, and 0.30 G), f is a mixture of (0.22 T, 0.16 C, 0.40 A, and 0.22 G), and k is a mixture of equal parts of T and G. The bases shown in lower case at either end are spacers. One of the variegated oligo-nts and the primer are combined in equimolar amounts and annealed. The ds DNA is completed with all four (nt)TPs and Klenow fragment. The resulting dsDNA and RF pLG7 are cut with both Kpn I and Xho I, purified, mixed, and ligated. This ligation mixture goes through the process described in Sec. 15 in which we select a transformed clone that, when induced with IPTG, binds AHTrp or trp.

If none of these approaches produces a working chimeric protein, we may try a different signal sequence, or a different OSP in M13 (e.g., the gene III protein for which there is fusion data (SMIT85, CRUZ88)), or another genetic package.

Example 1, Part II

BPTI binds very tightly to trypsin ($K_d$ = 6.0 x $10^{-14}$ M) and to anhydrotrypsin, so that these molecules are not preferred for optimizing the amount of BPTI to display on LG7 or the amount of affinity molecule to attach to the column. Tschesche et al. reported on the binding of several BPTI derivatives to various proteases:

Dissociation constants for BPTI derivatives, Molar.

| Residue #15 | Trypsin (bovine pancreas) | Chymotrypsin (bovine pancreas) | Elastase (porcine pancreas) | Elastase (human leukocytes) |
|---|---|---|---|---|
| lysine | 6.0 x $10^{-14}$ | 9.0 x $10^{-9}$ | - | 3.5 x $10^{-6}$ |
| glycine | - | - | + | 7.0 x $10^{-9}$ |
| alanine | + | - | 2.8 x $10^{-8}$ | 2.5 x $10^{-9}$ |

(continued)

| Residue #15 | Trypsin (bovine pancreas) | Chymotrypsin (bovine pancreas) | Elastase (porcine pancreas) | Elastase (human leukocytes) |
|---|---|---|---|---|
| valine | - | - | $5.7 \times 10^{-8}$ | $1.1 \times 10^{-10}$ |
| leucine | - | - | $1.9 \times 10^{-8}$ | $2.9 \times 10^{-9}$ |

From the report of Tschesche et al. we infer that molecular pairs marked "+" have $K_d$s greater than $3.5 \times 10^{-6}$ M and that molecular pairs marked "-" have $K_d$s much greater than $3.5 \times 10^{-6}$ M. Because of the wealth of data about the binding of BPTI and various mutants to trypsin and other proteases (TSCH87), we can proceed in various ways. (For other PBDs we can obtain two different monoclonal antibodies, one with a high affinity having $K_d$ of order $10^{-11}$ M, and one with a moderate affinity having $K_d$ on the, order of $10^{-6}$ M.) In this example, we may use: a) the moderate binding between BPTI and human leukocyte elastase (HuLEl), b) the moderately strong binding of porcine elastase to BPTI(V15), or c) the binding of BPTI(A15) (residue 38 in the pbd gene) for trypsin (weak but detectable) or for porcine pancreatic elastase.

We compare the retention of LG7 virions to the retention of wild-type M13 on {AHTrp}. M13 derivatives having more DNA than wild-type M13 have corresponding longer virions. Thus we will create pLG8 that differs from pLG7 only in having stop codons at codons 2 and 3, and an altered L codon at codon 7 of the osp-ipbd gene. Phage LG8 will have exactly as much DNA as LG7; therefore the LG8 virion is exactly as long as the LG7 virion. LG8 can not, however, display BPTI on its surface.

To expedite identification of different M13-derived phage, we replace the amp$^R$ gene of LG8 with the tet$^R$ gene from pBR322 by standard methods. The BSMI-to-AatII tet$^R$ bearing fragment of pBR322 is ligated into DNA from pLG8 cut with XbaI and AatII. The correct construction, having 9.2 kb, is easily distinguished from pBR322 and is called LG10.

The phage LG7 is grown at various levels of IPTG in the medium and harvested in the way previously described. An affinity column having bed volume of 2.0 ml and supporting an amount of HuLEl picked from the range 0.1 mg to 30.0 mg on 1 ml of BioRad Affi-Gel 10$^{(TM)}$ or Affi-Gel 15$^{(TM)}$ is designated {HuLEl}.

An appropriate set of densities of HuLEl on the column is (0.1 mg/ml, 0.5 mg/ml, 2.0 mg/ml, 8.0 mg/ml, 15.0 mg/ml, and 30.0 mg/ml). The $V_V$ of {HuLEl} is, by hypothesis, 1.0 ml. The elution of LG7 phage is compared to the elution of LG10 on {HuLEl} having varying amounts of HuLEl affixed. The columns are eluted in a standard way:

1) 10 mM KCl buffered to pH 8.0 with phosphate, until optical density at 280nm falls to base line or 4 x $V_V$, whichever is first,

2) a gradient of 10 mM to 2 M KCl in 3 x $V_V$, pH held at 8.0 with phosphate,

3) a gradient of 2 M to 5 M KCl in 3 x $V_V$, phosphate buffer to pH 8.0,

4) constant 5 M KCl plus 0 to 0.8 M guanidinium Cl in 2 x $V_V$, with phosphate buffer to pH 8.0.

The preferred level of induction (IPTG$_{optimal}$) and amount of affinity molecule on the matrix (DoAMoM$_{optimal}$) are those settings that give the sharpest LG7 elution peak that shows significant retardation as compared to LG8, which carries no BPTI. By hypothesis, the best separation occurs for the amount of BPTI/GP produced when the cells are induced with 10.0 uM IPTG and when 4.0 mg HuLEl/ml is applied to BioRad Affi-Gel 10$^{(TM)}$.

When the amount of BPTI/GP and the amount of HuLEl/volume of support have been optimized, we turn to optimization of elution rate, initial ionic strength, and the amount of GP/(volume of support). These parameters can be optimized separately.

Using optimal BPTI/GP and HuLEl/volume of support, we measure the elution volume of LG7 and LG8 for different elution rates, viz. 1, 1/2, 1/4, 1/8 and 1/16 times the maximum flow rate. By hypothesis, 1/4 of maximum elution rate is better than 1/2, but 1/8 is about the same as 1/4. Therefore 1/4 maximum elution rate will be used.

Elution volumes of LG7 obtained from cells grown on media that is 2.0 mM in IPTG are measured at optimal DoAMoM and elution rate for loadings of $10^9$, $10^{10}$, $10^{11}$, and $10^{12}$ pfu. By hypothesis, $10^{12}$ pfu of pure LG7 overloads the column and significant number of phage elute before their characteristic position in the KCl gradient. We also find that $10^{11}$ pfu overloads the column only slightly, and that $10^{10}$ pfu does not overload the column. Because the use of the affinity separation in Sec. 15 will involve a population in which no single member is more than one part in $10^4$, we conclude that $10^{12}$ pfu of a variegated population could be applied to a column of 1.0 ml matrix volume without overloading with respect any one species. The overloading of a 1.0 ml column by $10^{12}$ pfu also indicates that the initial column that captures indiscriminately adhesive phage should be 5 to 10 times as large as the column that supports the target material.

Elution volumes of LG7 and LG10 obtained from cells grown on media that is 2.0 mM in IPTG are measured at optimal conditions and for a loading of $10^{10}$ pfu for various initial ionic strengths: 1.0 mM, 5.0 mM, 10.0 mM, 20.0 mM, and 50.0 mM. We may find, for example, that LG10 is slightly retarded by the column when loaded at 1.0 mM KCl, but that LG7 always comes off the column at its characteristic place in the gradient. We use 10.0 mM as initial ionic strength in all remaining affinity separations.

To determine the sensitivity of chromatography of phage that display variants of BPTI on their surfaces (Sec. 10.1), we prepare artificial mixtures of two closely-related phage that differ only at one residue in the BPTI domain. One variety of phage has strong affinity for the column used in this step, while the other phage has no affinity for the column. We chromatograph these mixtures to discover how little of the phage that binds to the column can be detected within a large majority of phage that do not bind the column.

For these tests we choose AHTrp as AfM(BPTI). A column having 2 ml bed volume is prepared with (DOAMOM$_{optimal}$ mg of AHTrp)/(ml of Affi-Gel 10$^{(TM)}$). The column is called {AHTrp} and has $V_V$ = 1.0 ml.

A new phage, LG9, is prepared that displays BPTI(V15) as IPBD in contrast to LG7 that displays BPTI(K15, wild-type) as IPBD. Residue 15 of BPTI is residue 38 of the osp-ipbd gene. We introduce the change K38 to V by replacement of a short segment of the osp-ipbd gene between Apa I & Stu I. The correct construction is called pLG9. To expedite differentiation between LG7 and an LG9-derivative phage, we replace the amp$^R$ gene of LG9 with the tet$^R$ gene from pBR322. DNA from pBR322 between BsmI (1353, blunted) and AatII (1428) is ligated to dsDNA from pLG9 cut with XbaI (blunted) and AatII. The correct construction, having 9.2 kb, is easily distinguished from pBR322 and is called LG11. DNA from phage LG11 is sequenced in the vicinity the junctions of the newly inserted tet$^R$ gene to confirm the construction.

LG7 and LG11 are grown with optimum IPTG (2.0 mM) and harvested. Mixtures are prepared in the ratios

$$LG7:LG11 :: 1:V_{lim}$$

where $V_{lim}$ ranges from $10^{10}$ to $10^5$ by factors of 10. Large values of $V_{lim}$ are tested first; once a $V_{lim}$ is found that allows recovery of LG7, smaller values of $V_{lim}$ are not be tested.

The column {AHTrp} is first blocked by treatment with $10^{11}$ virions of M13am429 in 100 ul of 10 mM KCl buffered to pH 8.0 with phosphate; the column is washed with the same buffer until OD$_{260}$ returns to base line or 4 x $V_V$ have passed through the column, whichever comes first. One of the mixtures of LG7 and LG11 containing $10^{12}$ pfu in 1 ml of the same buffer is applied to {AHTrp}. The column is eluted in a standard way :

1) 10 mM KCl buffered to pH 8.0 with phosphate, until optical density at 280nm falls to base line or 4 x $V_V$, whichever is first, (discard effluent),

2) a gradient of 10 mM to 2 M KCl in 3 x $V_V$, pH held at 8.0 with phosphate, (30 x 100 ul fractions),

3) a gradient of 2 M to 5 M KCl in 3 x $V_V$, phosphate buffer to pH 8.0, (30 x 100 ul fractions),

4) constant 5 H KCl plus 0 to 0.8 M guanidinium Cl in 2 x $V_V$, with phosphate buffer to pH 8.0, (20 x 100 ul fractions),

5) constant 5 M KCl plus 0.8 M guanidinium Cl in 1.2 x $V_V$, with phosphate buffer to pH 8.0, (12 x 100 ul fractions).

Samples of 4 ul from each fraction are plated at suitable dilution on phage-sensitive Sup$^+$ cells (so that M13am429 will not grow). A sample of the column matrix is also used as inoculum for phage-sensitive Sup$^+$ cells. Plaques are transferred to ampicillin-containing LB agar, and Amp$^R$ colonies are tested for display of BPTI(K15) by use of trp* or AHTrp*.

By hypothesis, $V_{lim} = 4.0 \times 10^8$ is the largest value for which LG7 can be recovered. Thus $C_{sensi} = 4.0 \times 10^8$. Three cycles of chromatography are required to isolate LG7, so the first approximation to $C_{eff}$ is 740 ( = exp( log$_e$(4.0 x 10$^8$)/3 ) ).

We now determine the efficiency of the affinity separation (Sec. 10.2). This is done by: a) preparing mixtures of LG7 and LG11 in the ratio 1:Q, b) enriching the population for LG7 for one separation cycle, and c) determining the fraction of LG7 in the last phage-bearing fraction. When Q is 1.5 x $10^4$, 3% of colonies are BPTI positive. When Q is 1.5 x $10^3$, 60% of the colonies are BPTI positive. Thus we calculate $C_{eff}$ = .60 x 1.5 x $10^3$ = 900.

Our hypothetical LG7 should display one or more BPTI domains on each virion. The oso-ipbd gene is under control of the lacUV5 promoter so that expression levels of BPTI-M13 CP can be manipulated via [IPTG]. This construct may be used to develop many different binding proteins, all based on BPTI. An optimum level of induction and amount

of AfM(PBD) (= DOAMOM $_{optimum}$ = 2.0 mg/(ml of support)) should have been determined; target molecules will be applied to columns in this amount in the process disclosed in Sec. 15.1. These optimum levels may be adequate for all targets and all variegations of BPTI displayed on derivatives of M13 based on LG7, but some further optimization may be needed if other values of pH or temperatures are used.

Other <u>pbd</u> gene fragments may be substituted for the <u>bpti</u> gene fragment in pLG7 with a high likelihood that PBD will appear on the surface of the new LG7 derivative.

Example 1, Part III

HHMb is chosen as a typical protein target; an other protein could be used. HHMb satisfies all of the criteria for a target: 1) it is large enough to be applied to an affinity matrix, 2) after attachment it is not reactive, and 3) after attachment there is sufficient unaltered surface to allow specific binding by PBDs.

The essential information for HHMb is known: 1) HHMb is stable at least up to 70°C, between pH 4.4 and 9.3, 2) HHMb is stable up to 1.6 M Guanidinium Cl, 3) the pI of HHMb is 7.0, 4) for HHMb, $M_r$ = 16,000, 5) HHMb requires haem, 6) HHMb has no proteolytic activity.

In addition, the following information about HHMb and other myoglobins is available: 1) the sequence of HHMb, 2) the 3D structure of sperm whale myoglobin (HHMb has 19 amino acid differences and it is generally assumed that the 3D structures are almost identical), 3) its lack of enzymatic activity, 4) its lack of toxicity.

We set the specifications of an SBD as :

    1) T = 25°C

    2) pH = 8.0

    3) Acceptable solutes :

        A ) for binding :

            i) phosphate, as buffer, 0 to 20 mM, and
            ii) KCl, 10 mM,

        B ) for column elution :

            i) phosphate, as buffer, 0 to 30 mM,
            ii) KCl, up to 5 M, and
            iii) Guanidinium Cl, up to 0.8 M.

    4) Acceptable $K_d$ < 1.0 x $10^{-8}$ M.

We choose LG7 as GP(IPBD).

Residues to be varied are picked, in part, through the use of interactive computer graphics to visualize the structures. In this section, all residue numbers refer to BPTI. We pick a set of residues that forms a surface such that all residues can contact one target molecule. Information relevant to choosing BPTI residues to vary includes: 1) the 3D structure, 2) solvent accessibility of each residue (LEEB71), 3) a compilation of sequences of other proteins homologous to BPTI, and 4) knowledge of the structural nature of different amino acid types.

Tables 16 and 34 indicate which residues of BPTI: a) have substantial surface exposure, and b) are known to tolerate other amino acids in other closely related proteins. We use interactive computer graphics to pick sets of eight to twenty residues that are exposed and variable and such that all members of one set can touch a molecule of the target material at one time. If BPTI has a small amino acid at a given residue, that amino acid may not be able to contact the target simultaneously with all the other residues in the interaction set, but a larger amino acid might well make contact. A charged amino acid might affect binding without making direct contact. In such cases, the residue should be included in the interaction set, with a notation that larger residues might be useful. In a similar way, large amino acids near the geometric center of the interaction set may prevent residues on either side of the large central residue from making simultaneous contact. If a small amino acid, however, were substituted for the large amino acid, then the surface would become flatter and residues on either side could make simultaneous contact. Such a residue should be included in the interaction set with a notation that small amino acids may be useful.

Table 35 was prepared from standard model parts and shows the maximum span between $C_{beta}$ and the tip of each type of side group. $C_{beta}$ is used because it is rigidly attached to the protein main-chain; rotation about the $C_{al-}$

pha-C_beta bond is the most important degree of freedom for determining the location of the side group.

Table 34 indicates five surfaces that meet the given criteria. The first surface comprises the set of residues that contacts trypsin in the complex of trypsin with BPTI as reported in the Brookhaven Protein Data Bank entry "1TPA". This set is indicated by the number "1". The exposed surface of the residues in this set (taken from Table 16) totals 1148 A$^2$ and the approximates the area of contact between BPTI and trypsin.

Other surfaces, numbered 2 to 5, were picked by first picking one exposed, variable residue and then picking neighboring residues until a surface was defined. The choice of sets of residues shown in Table 34 is in no way exhaustive or unique; other sets of variable, surface residues can be picked. Hereinafter we refer to K15 as being at the top of the molecule, while the carboxy and amino termini are at the bottom.

Solvent accessibilities are useful, easily tabulated indicators of a residue's exposure. Solvent accessibilities must be used with some caution; small amino acids are under-represented and large amino acids over-represented. The user must consider what the solvent accessibility of a different amino acid would be when substituted into the structure of BPTI.

To create specific binding between a derivative of BPTI and HHMb, we will vary the residues in set #2. This set includes the twelve principal residues 17(R), 19(I), 21(Y), 27(A), 28(G), 29(L), 31(Q), 32(T), 34(V), 48(A), 49(E), and 52(M) (Sec. 13.1.1). None of the residues in set #2 is completely conserved in the sample of sequences reported in Table 34; thus we can vary them with a high probability of retaining the underlying structure. Independent substitution at each of these twelve residues of the amino acid types observed at that residue would produce approximately 4.4 x 10$^9$ amino acid sequences and the same number of surfaces.

BPTI is a very basic protein. This property has been used in isolating and purifying BPTI and its homologues so that the high frequency of arginine and lysine residues may reflect bias in isolation and is not necessarily required by the structure. Indeed, SCI-III from _Bombyx_ _mori_ contains seven more acidic than basic groups (SASA84).

Residue 17 is highly variable and fully exposed and can contain R, K, A, Y, H, F, L, M, T, G, Y, P, or S. All types of amino acids are seen: large, small, charged, neutral, and hydrophobic. That no acidic groups are observed may be due to bias in the sample.

Residue 19 is also variable and fully exposed, containing P, R, I, S, K, Q, and L.

Residue 21 is not very variable, containing F or Y in 31 of 33 cases and I and W in the remaining cases. The side group of Y21 fills the space between T32 and the main chain of residues 47 and 48. The OH at the tip of the Y side group projects into the solvent. Clearly one can vary the surface by substituting Y or F so that the surface is either hydrophobic or hydrophilic in that region. It is also possible that the other aromatic amino acid (_viz._ H) or the other hydrophobics (L, M, or V) might be tolerated.

Residue 27 most often contains A, but S, K, L, and T are also observed. On structural grounds, this residue will probably tolerate any hydrophilic amino acid and perhaps any amino acid.

Residue 28 is G in BPTI. This residue is in a turn, but is not in a conformation peculiar to glycine. Six other types of amino acids have been observed at this residue: K, N, Q, R, H, and N. Small side groups at this residue might not contact HHMb simultaneously with residues 17 and 34. Large side groups could interact with HHMb at the same time as residues 17 and 34. Charged side groups at this residue could affect binding of HHMb on the surface defined by the other residues of the principal set. Any amino acid, except perhaps P, should be tolerated.

Residue 29 is highly variable, most often containing L. This fully exposed position will probably tolerate almost any amino acid except, perhaps, P.

Residues 31, 32, and 34 are highly variable, exposed, and in extended conformations; any amino acid should be tolerated.

Residues 48 and 49 are also highly variable and fully exposed, any amino acid should be tolerated.

Residue 52 is in an alpha helix. Any amino acid, except perhaps P, might be tolerated.

Now we consider possible variation of the secondary set (Sec. 13.1.2) of residues that are in the neighborhood of the principal set. Neighboring residues that might be varied at later stages include 9(P), 11(T), 15(K), 16(A), 18(I), 20(R), 22(F), 24(N), 26(K), 35(Y), 47(S), 50(D), and 53(R).

Residue 9 is highly variable, extended, and exposed. Residue 9 and residues 48 and 49 are separated by a bulge caused by the ascending chain from residue 31 to 34. For residue 9 and residues 48 and 49 to contribute simultaneously to binding, either the target must have a groove into which the chain from 31 to 34 can fit, or all three residues (9, 48, and 49) must have large amino acids that effectively reduce the radius of curvature of the BPTI derivative.

Residue 11 is highly variable, extended, and exposed. Residue 11, like residue 9, is slightly far from the surface defined by the principal residues and will contribute to binding in the same circumstances.

Residue 15 is highly varied. The side group of residue 15 points away form the face defined by set #2. Changes of charge at residue 15 could affect binding on the surface defined by residue set #2.

Residue 16 is varied but points away from the surface defined by the principal set. Changes in charge at this residue could affect binding on the face defined by set #2.

Residue 18 is I in BPTI. This residue is in an extended conformation and is exposed. Five other amino acids have been observed at this residue: M, F, L, V, and T. Only T is hydrophilic. The side group points directly away from the surface defined by residue set #2. Substitution of charged amino acids at this residue could affect binding at surface defined by residue set #2.

Residue 20 is R in BPTI. This residue is in an extended conformation and is exposed. Four other amino acids have been observed at this residue: A, S, L, and Q. The side group points directly away from the surface defined by residue set #2. Alteration of the charge at this residue could affect binding at surface defined by residue set #2.

Residue 22 is only slightly varied, being Y, F, or H in 30 of 33 cases. Nevertheless, A, N, and S have been observed at this residue. Amino acids such as L, M, I, or Q could be tried here. Alterations at residue 22 may affect the mobility of residue 21; changes in charge at residue 22 could affect binding at the surface defined by residue set #2.

Residue 24 shows some variation, but probably can not interact with one molecule of the target simultaneously with all the residues in the principal set. Variation in charge at this residue might have an effect on binding at the surface defined by the principal set.

Residue 26 is highly varied and exposed. Changes in charge may affect binding at the surface defined by residue set #2; substitutions may affect the mobility of residue 27 that is in the principal set.

Residue 35 is most often Y, W has been observed. The side group of 35 is buried, but substitution of F or W could affect the mobility of residue 34.

Residue 47 is always T or S in the sequence sample used. The $O_{gamma}$ probably accepts a hydrogen bond from the NH of residue 50 in the alpha helix. Nevertheless, there is no overwhelming steric reason to preclude other amino acid types at this residue. In particular, other amino acids the side groups of which can accept hydrogen bonds, viz. N, D, Q, and E, may be acceptable here.

Residue 50 is often an acidic amino acid, but other amino acids are possible.

Residue 53 is often R, but other amino acids have been observed at this residue. Changes of charge may affect binding to the amino acids in interaction set #2.

From published models (HUBE77, WLOD84) one can see that R39 is on the opposite side of BPTI from the surface defined by the residues in set #2. Therefore, variation at residue 39 at the same time as variation of some residues in set #2 is much less likely to improve binding that occurs along surface #2 than is variation of the other residues in set #2.

In addition to the twelve principal residues and 13 secondary residues, there are two other residues, 30(C) and 33(F), involved in surface #2 that we will probably not vary, at least not until late in the procedure. These residues have their side groups buried inside BPTI and are conserved. Changing these residues does not change the surface nearly so much as does changing residues in the principal set. These buried, conserved residues do, however, contribute to the surface area of surface #2. The surface of residue set #2 is comparable to the area of the trypsin-binding surface. Principal residues 17, 19, 21, 27, 28, 29, 31, 32, 34, 48, 49, and 52 have a combined solvent-accessible area of 946.9 $Å^2$. Secondary residues 9, 11, 15, 16, 18, 20, 22, 24, 26, 35, 47, 50, and 53 have combined surface of 1041.7 Å2. Residues 30 and 33 have exposed surface totaling 38.2 $Å^2$. Thus the three groups' combined surface is 2026.8 $Å^2$.

Residue 30 is C in BPTI and is conserved in all homologous sequences. It should be noted, however, that C14/C38 is conserved in all natural sequences, yet Marks et al. (MARK87) showed that changing both C14 and C38 to A,A or T,T yields a functional trypsin inhibitor. Thus it is possible that BPTI-like molecules will fold if C30 is replaced.

Residue 33 is F in BPTI and in all homologous sequences. Visual inspection of the BPTI structure suggests that substitution of Y, M, H, or L might be tolerated.

Given our hypothetical affinity separation sensitivity, $C_{sensi}$, we decide to vary six residues leaving some margin for errors in the actual base composition of variegated bases. To obtain maximal recognition, we choose residues from the principal set that are as far apart as possible. Table 36 shows the distances between the beta carbons of residues in the principal and peripheral set. R17 and V34 are at one end of the principal surface. Residues A27, G28, L29, A48, E49, and M52 are at the other end, about twenty Angstroms away; of these, we will vary residues 17, 27, 29, 34, and 48. Residues 28, 49, and 52 will be varied at later rounds.

Of the remaining principal residues, 21 is left to later variations. Among residues 19, 31, and 32, we arbitrarily pick 19 to vary.

Unlimited variation of six residues produces $6.4 \times 10^7$ amino acid sequences. By hypothesis, $C_{sensi}$ is 1 in 4 $\times 10^8$. Table 37 shows the programmed variegation at the chosen residues. The parental sequence is present as 1 part in $5.5 \times 10^7$, but the least favored sequences are present at only 1 part in $4.2 \times 10^9$. Among single-amino-acid substitutions from the PPBD, the least favored is F17-I19-A27-L29-V34-A48 and has a calculated abundance of 1 part in $1.6 \times 10^8$. Using the optimal qfk codon, we can recover the parental sequence and all one-amino-acid substitutions to the PPBD if actual nt compositions come within 5% of programmed compositions. The number of transformants is $M_{ntv} = 1.0 \times 10^9$ (also by hypothesis), thus we will produce most of the programmed sequences.

The residue numbers above refer to mature BPTI. Since Table 25 refers to the pre-M13CP-BPTI protein, all mature BPTI sequence numbers have been increased by the length of the signal sequence, 23. Thus, we wish to vary residues 40, 42, 50, 52, 57, and 71. A DNA subsequence containing all these codons is found between the (ApaI) sites at base 191 and the SphI site at base 309 of the <u>osp-pbd</u> gene. Among <u>Apa</u>I, <u>Dra</u>II, and <u>Pss</u>I, <u>Apa</u>I is preferred because it recognizes six bases without any ambiguity and will cut fewer sequences in the vgDNA. Gratuitous restriction sites can be avoided in some cases by use of codon ambiguity: changing the codon for g51 from GGC to GGT makes it impossible to generate an <u>Apa</u>I site at codons 50, 51, and 6=52.

Each piece of dsDNA to be synthesized needs six to eight bases added at either end to allow cutting with restriction enzymes and is shown in Table 37. The first synthetic base (before cutting with <u>Apa</u>I and <u>Sph</u>I) is 184 and the last is 322. There are 142 bases to be synthesized. The center of the piece to the synthesized lies between Q54 and V57. The overlap can not include varied bases, so we choose bases 245 to 256 as the overlap that is 12 bases long. Note that the codon for F56 has been changed to TTC to increase the GC content of the overlap. The amino acids that are being varied are marked as <u>X</u> with a plus over them. Codons 57 and 71 are synthesized on the sense (bottom) strand. The design calls for "qfk" in the antisense strand, so that the sense strand contains (from 5' to 3') a) equal part C and A (<u>i.e.</u> the complement of k), b) (0.40 T, 0.22 A, 0.22 C, and 0.16 G) (<u>i.e.</u> the complement of f), and c) (0.26 T, 0.26 A, 0.30 C, and 0.18 G).

Each residue that is encoded by "qfk" has 21 possible outcomes, each of the amino acids plus stop. Table 12 gives the distribution of amino acids encoded by "qfk", assuming 5% errors. The abundance of the parental sequence is the product of the abundances of R x I x A x L x V x A. The abundance of the least-favored sequence is 1 in $4.2 \times 10^9$.

Olig#27 and olig#28 are annealed and extended with Klenow fragment and all four (nt)TPs. Both the ds synthetic DNA and RE pLG7 DNA are cut with both <u>Apa</u> I and <u>Sph</u> I. The cut DNA is purified and the appropriate pieces ligated (See Sec. 14.1) and used to transform competent PE383. (Sec. 14.2). In order to generate a sufficient number of transformants, we start with 5.0 l of cells.

1) culture <u>E. coli</u> in 5.0 l of LB broth at 37°C until cell density reaches $5 \times 10^7$ to $7 \times 10^7$ cells/ml,

2) chill on ice for 65 minutes, centrifuge the cell suspension at 4000g for 5 minutes at 4°C,

3) discard supernatant; resuspend the cells in 1667 ml of an ice-cold, sterile solution of 60 mM $CaCl_2$,

4) chill on ice for 15 minutes, and then centrifuge at 4000g for 5 minutes at 4°C,

5) resuspend cells in 2 x 400 ml of ice-cold, sterile 60 mM $CaCl_2$; store cells at 4°C for 24 hours,

6) add DNA (100 μg) in 20 ml of litigation or TE buffer; mix, inculafe on ice for minutes,

7) distribute into 200 μl aliquots and heat shock cells at 42°C for 20 seconds,

8) add 200 ml LB broth and incubate at 37°C for 1 hour,

9) add the culture to 2.0 l of LB broth containing ampicillin at 35-100 ug/ml and culture overnight at 37°C,

10) after 6 hours, remove 200 ml and plate 0.5 ml portions with log phase JM 107 on LB agar, using the soft-agar overlay technique. Phage are prepared from the soft agar,

11) centrifuge the overnight culture to remove cells, and pellet phage (MESS83),

12) harvest virions by method of Salivar, et al. (SALI64).

It is important to: a) use all or nearly all the vgDNA synthesized in ligation, b) use all or nearly all the ligation mixture to transform cells, and c) culture all or nearly all the transformants. These measures are directed at maintaining diversity.

It is important to collect virions in a way that samples all or nearly all the transformants. Because F⁻ cells are used in the transformation, multiple infections do not pose a problem in the overnight phage production. F' cells are used for phage production in agar.

HHMb has a pI of 7.0 and we carry out chromatography at pH 8.0 so that HHMb is slightly negative while BPTI and most of its mutants are positive. HHMb is fixed (Sec. 15.1) to a 2.0 ml column on Affi-Gel 10(™) or Affi-Gel 15(™) at 4.0 mg/ml support matrix, the same density that is optimal for a column supporting trp.

To remove variants of BPTI with strong, indiscriminate binding for any protein or for the support matrix (Sec. 15.2), we pass the variegated population of virions over a column that supports bovine serum albumin (BSA) before loading the population onto the {HHMb} column. Affi-Gel 10$^{(TM)}$ or Affi-Gel 15$^{(TM)}$ is used to immobilize BSA at the highest level the matrix will support. A 10.0 ml column is loaded with 5.0 ml of Affi-Gel-linked-BSA; this column, called {BSA}, has $V_V$ = 5.0 ml. The variegated population of virions containing $10^{12}$ pfu in 1 ml (0.2 x $V_V$) of 10 mM KCl, 1 mM phosphate, pH 8.0 buffer is applied to {BSA}. We wash {BSA} with 4.5 ml (0.9 x $V_V$) of 50 mM KCl, 1 mM phosphate, pH 8.0 buffer. The wash with 50 mM salt will elute virions that adhere slightly to BSA but not virions with strong binding. The pooled effluent of the {BSA} column is 5.5 ml of approximately 13 mM KCl.

The column {HHMb} is first blocked by treatment with $10^{11}$ virions of M13(am429) in 100 ul of 10 mM KCl buffered to pH 8.0 With phosphate; the column is washed with the same buffer until OD$_{260}$ returns to base line or 2 x $V_V$ have passed through the column, whichever comes first. The pooled effluent from {BSA} is added to {HHMb} in 5.5 ml of 13 mM KCl, 1 mM phosphate, pH 8.0 buffer. The column is eluted (Sec. 15.3) in the following way:

1) 10 mM KCl buffered to pH 8.0 with phosphate, until optical density at 280nm falls to base line or 2 x $V_V$, whichever is first, (effluent discarded),

2) a gradient of 10 mM to 2 M KCl in 3 x $V_V$, pH held at 8.0 with phosphate, (30 x 100 μl fractions),

3) a gradient of 2 M to 5 M KCl in 3 x $V_V$, phosphate buffer to pH 8.0 (30 x 100 μl fractions),

4) constant 5 M KCl plus 0 to 0.8 M guanidiniun Cl in 2 x $V_V$, with phosphate buffer to pH 8.0, (20 x 100 μl fractions), and

5) constant 5 M KCl plus 0.8 M guanidinium Cl in 1 x $V_V$, with phosphate buffer to pH 8.0, (10 x 100 μl fractions).

In addition to the elution fractions, a sample is removed from the column and used as an inoculum for phage-sensitive Sup$^+$ cells (Sec. 15.4). A sample of 4 μl from each fraction is plated on phage-sensitive Sup$^+$ cells. Fractions that yield too many colonies to count are replated at lower dilution. An approximate titre of each fraction is calculated. Starting with the last fraction and working toward the first fraction that was titered, we pool fractions until approximately $10^9$ phage are in the pool, i.e. about 1 part in 1000 of the phage applied to the column. This population is infected into 3 x $10^{11}$ phage-sensitive PE384 in 300 ml of LB broth. The low multiplicity of infection is chosen to reduce the possibility of multiple infection. After thirty minutes, viable phage have entered recipient cells but have not yet begun to produce new phage. Phage-born genes are expressed at this phase, and we can add ampicillin that will kill uninfected cells. These cells still carry F-pili and will absorb phage helping to prevent multiple infections.

If multiple infection should pose a problem that cannot be solved by growth at low multiple-of-infection on F$^+$ cells, the following procedure can be employed to obviate the problem. Virions obtained from the affinity separation are infected into F$^+$ E. coli and cultured to amplify the genetic messages (Sec. 15.5). CCC DNA is obtained either by harvesting RF DNA or by in vitro extension of primers annealed to ss phage DNA. The CCC DNA is used to transform F$^-$ cells at a high ratio of cells to DNA. Individual virions obtained in this way should bear proteins encoded only by the DNA within.

The variegation produces as many as 6.4 x $10^7$ different amino-acid sequences. C$_{eff}$ is 900. Thus, after two separation cycles, the probability of isolating a single SBD is less than 0.10; after three cycles, the probability rises above 0.10.

The phagemid population is grown and chromatographed three times and then examined for SBDs (Sec. 15.7). In each separation cycle, phage from the last three fractions that contain viable phage are pooled with phage obtained by removing some of the support matrix as an inoculum. At each cycle, about $10^{12}$ phage are loaded onto the column and about $10^9$ phage are cultured for the next separation cycle. After the third separation cycle, 32 colonies are picked from the last fraction that contained viable phage; phage from these colonies are denoted SBD1, SBD2,..., and SBD32.

Each of the SBDs is cultured and tested for retention on a Pep-Tie column supporting HHMb (Sec. 15.8). Phage LG7(SBD11) shows the greatest retention on the Pep-Tie {HHMb} column, eluting at 367 mM KCl while wtM13 elutes at 20 mM KCl. SBD11 becomes the parental amino-acid sequence to the second variegation cycle.

The result of this hypothetical experiment is shown in Table 38. R40 changed to D, I42 changed to Q, A50 changed to E, L52 remained L, and A71 changed to W.

The next round of variegation (Sec. 16) is illustrated in Table 39. The residues to be varied are chosen by: a) choosing some of the residues in the principal set that were not varied in the first round (viz. residues 42, 44, 51, 54, 55, 72, or 75 of the fusion), and b) choosing some residues in the secondary set. Residues 51, 54, 55, and 72 are varied through all twenty amino acids and, unavoidably, stop. Residue 44 is only varied between Y and F. Some residues in the secondary set are varied through a restricted range; primarily to allow different charges (+, 0, -) to appear. Residue 38 is varied through K, R, E, or G. Residue 41 is varied through I, V, K, or E. Residue 43 is varied through R, S, G, N, K, D, E, T, or A.

Olig#29 and olig#30 are synthesized, annealed, extended and cloned into pLG7 at the Apa I/Sph I sites. The ligation mixture is used to transform 5 l of competent PE383 cells so that $10^9$ transformants are obtained. A new {HHMb} is constructed using the same support matrix as was used in round 1. A sample of $10^{12}$ of the harvested LG7 are applied to {HHMb} and affinity separated. The last $10^9$ phage off the column and an inoculum are pooled and cultured. The cultured phagemids are re-chromatographed for three separation cycles. Thirty-two clonal isolates (denoted SBD11-1, SBD11-2,..., SBD11-32) are obtained from the effluent of the third separation cycle and tested for binding on a Pep-Tie {HHMb} column. Of this set, SBD11-23 shows the greatest retention on the Pep-Tie {HHMb} column, eluting at 692 mM KCl.

The results of this hypothetical selection is shown in Table 40. Residue 38 (K15 of BPTI) changed to E, 41 becomes V, 43 goes to N, 44 goes to F, 51 goes to F, 54 goes to S, 55 goes to A, and 72 goes to Q.

The sbd11-23 portion of the osp-pbd gene is cloned into an expression vector and BPTI(E15, D17, V18, Q19, N20, F21, E27, F28, L29, S31, A32, S34, W71, Q72) is expressed in the periplasm. This protein is isolated by standard methods and its binding to HHMb is tested. $K_d$ is found to be $4.5 \times 10^{-7}$ M.

A third round of variation, using SBD11-23 as PPBD, is illustrated in Table 41; eight amino acids are varied. Those in the principal set, residues 40, 55, and 57, are varied through all twenty amino acids. Residue 32 is varied through P, Q, T, K, A, or E. Residue 34 is varied through T, P, Q, K, A, or E. Residue 44 is varied through F, L, Y, C, W, or stop. Residue 50 is varied through E, K, or Q. Residue 52 is varied through L, F, I, M, or V.

The result of this variation is shown in Table 42. The selected SBD is denoted SBD11-23-5 and elutes from a Pep-Tie {HHMb} column at 980 mM KCl. The sbd11-23-5 segment is cloned into an expression vector and BPTI(E9, Q11, E15, A17, V18, Q19, N20, W21, Q27, F28, M29, S31, L32, H34, W71, Q72) is produced. This time the $K_d$ is $7.3 \times 10^{-9}$ M.

This example is hypothetical. It is anticipated that more variegation cycles will be needed to achieve dissociation constants of $10^{-8}$ M. It is also possible that more than three separation cycles will be needed in some variegation cycles. Real DNA chemistry and DNA synthesizers may have larger errors than our hypothetical 5%. If $S_{err} > 0.05$, then we may not be able to vary six residues at once. Variation of 5 residues at once is certainly possible.

Citations :

ACHT78:
Achtman, M, G Morelli, S Schwuchow,
J Bacteriol (1978), 135 (3) p1053-61.

AKOH72:
Ako, H, RJ Foster, and CA Ryan,
Biochem Biophys Res Commun (USA)(1972), 47(6) p1402-7.

ANFI73:
Anfinsen, CB,
Science (1973), 181(96)223-30.

ARGO87:
Argos, P,
J. Mol. Biol. (1987), 197:331-348.

AUDI84a:
Auditore-Hargreaves, K,
United States Patent 4,470,925, September 11, 1984.

AUDI84b:
Auditore-Hargreaves, K,
United States Patent 4,479,895, October 30, 1984.

AUER87:
Auerswald, E-A, W Schroeder, and M Kotick,
Biol. Chem. Hoppe-Seyler (1987), 368:1413-1425.

AUSU87:
Ausubel, FM, R Brent, RE Kingston, DD Moore, JG Seidman, JA Smith, and K Struhl, Editors
Current Protocols in Molecular Biology.
Greene Publishing Associates and Wiley-Interscience, Publishers
John Wiley & Sons, New York, 1987.

BANN81:
Banner, DW, C Nave, and DA Marvin,
Nature (1981),289:814-816.

BASH87:
Bash, PA, UC Singh, R Langridge, and PA Kollman,
Science (1987), 236 (4801) p564-8.

BECK83:
Beckwith, J, and TJ Silhavy,
Methods in Enzymology (1983), 97:3-11.

BECK88:
Beckwith, J, D Boyd, K McGovern, C. Manoil, JL San Milan,
S Froshauer, and N Green
Talk presented at "The Protein Folding Problem", a series of lectures and posters presented at the 1988 annual meeting of AAAS in Boston.

BENS84:
Benson, SA, E Bremer, and TJ Silhavy,
Proc Natl Acad Sci USA (1984), 81:3830-3834.

BENS86:
Benson, N, P Sugiono, S Bass, LV Mandelman, P Youderian,
Genetics (1986) 114(1)1-14.

BETT88:
Better, M, CP Chang, RR Robinson, and AH Horwitz,
Science (1988), 240:1041-1043.

BIRD67:
Birdsell, DC, and EH Cota-Robles,
J Bacteriol (1967), 93:427-437.

BLUN88:
Blundell, T, D Carney, S Gardner, F Hayes, B Howlin, T Hubbard, J Overington, DA Singh, BL Sibanda, and M Sutcliffe,
Eur J Biochem (15 March 1988), 172 (3) p513-20.

BOEK80:
Boeke, JD, M Russel, and P Model,
J. Mol. Biol. (1980), 144:103-116.

BONN85:
Bonnafous, JC, J Fornand, J Favero, and J-C Mani, Chapter 8 in Affinity Chromatography, a practical approach.,
Edited by PDG Dean, WSJohnson and FA Middle,
IRL Press, Oxford, UK 1985

BONO85:
Bonomi, F, S Pagani, DM Kurtz Jr,
Eur J Biochem (1985), 148(1)67-73.

BOQU87:
Boquet, PL, C Manoil, and J Beckwith,
J. Bacteriol. (1987), 169:1663-1669.

BOTS85:
Botstein, D, and D Shortle,
Science (1985), 229:1193-1201.

BRIG87:
Briggs, MR, JT Kadonaga, SP Bell, and R Tjian,
Science (Oct 3 1986), 234 (4772) 47-52.

CANT87:
Canters, GW,
FEBS Letters (1987), 212(1)168-172.

CARU83:
Caruthers, MH, SL Beaucage, JW Efcavitch, EF Fisher, RA Goldman, PL DeHaseth, W Mandecki, MD Matteucci, MS Rosendahl, and Y Stabinski,
Cold Spr. Harb. Symp. Quant. Biol. (1983), 47:411-418.

CARU85:
Caruthers, MH,
Science (1985), 230:281-285.

CARU87:
Caruthers, MH, P Gottlieb, LP Bracco, and L Cummings, in Protein Structure, Folding, and Design 2, 1987.
Ed. D Oxender (New York, AR Liss Inc.) p.9ff.

CHAM82:
Chambers, RW, I Kucan, and Z Kucan,
Nucleic Acids Res. (1982), 10(20)6465-73.

CHAN79:
Chang, CN, P Model, and G Blobel,
Proc. Natl. Acad. Sci. USA (1979), 76:1251-1255.

CHAR84:
Charbit, A, J-M Clement, and M Hofnung,
J. Mol. Biol. (1984), 175:395-401.

CHAR87:
Charbit, A, E Sobczak, ML Michel, A Molla, P Tiollais, M Hofnung,
J Immunol (1987), 139:1658-64.

CHAZ85:
Chazin, WJ, DP Goldenberg, TE Creighton, and K Wuthrich,
Eur J Biochem (1985), 152:(2)429-37.

CHEN88:
Chen, W, and K Struhl,
Proc Natl Acad Sci USA (1988), 85:2691-2695.

CHOT75:
Chothia, C, and J Janin,
Nature (1975), 256:705-708.

CHOT76:

Chothia, C, S Wodak, and J Janin,
Proc. Natl. Acad. Sci. USA (1976), <u>73</u>:3793-7.


CHOT86:
Chothia, C, and AM Lesk,
EMBO J (1986), <u>5</u>:823-826.


CHOU74:
Chou, PY, and GD Fasman,
Biochemistry (1974), <u>13</u>:(2)222-45.


CHOU78a:
Chou, PY, and GD Fasman,
Adv Enzymol (1978), <u>47</u>:45-148.


CHOU78b:
Chou, PY, and GD Fasman,
Annu Rev Biochem (1978), <u>47</u>:251-76.


CHUN86:
Chung, DW, K Fujikawa, BA McMullen, and EW Davie,
Biochemistry (1986), <u>25</u>:2410-2417.


CLEM81:
Clement, JM, and M Hofnung,
Cell (1981), <u>27</u>:507-514.


CLEM83:
Clement JM, E Lepouce, C Marchal, and M Hofnung,
EMBO J (1983), <u>2</u>:77-80.


CLOR87:
Clore, GM, AM Gronenborn, M Kjaer, and FM Poulsen,
Protein Engineering (1987), <u>1</u>:305-311.


CLUN84:
Clune, A, K-S Lee, and T Ferenci,
Biochem. and Biophys. Res. Comm. (1984), <u>121</u>:34-40.


CRAI85:
Craik, CS, C Largman, T Fletcher, S Roczniak, PJ Barr, R Fletterick, and WJ Rutter,
Science (1985), <u>228</u>:291-7.


CRAW87:
Crawford, IP, M Clarke, M van Cleemput, and C Yanofsky,
J Biol Chem (1987), <u>262</u>(1)239-244.


CREI84:
Creighton, TE,
<u>Proteins: Structures and Molecular Principles.</u>,
W. H. Freeman & Co., New York, 1984.


CRUZ88:
de la Cruz, VF, AA Lal, and TF McCutchan,
J Biol Chem (1988), <u>263</u>(9)4318-4322.


DAIR80:
Dairs, RW, D Botstein, and JR Roth,

Advanced Bacterial Genetics,
Cold Spring Harbor Laboratory Press, 1980.

DAWK86:
Dawkins, R,
The Blind Watchmaker
W. W. Norton & Co., New York, 1986.

DAYR86:
Dayringer, H, A Tramantano, and R Fletterick,
Computer Graphics and Molecular Modeling,
Cold Spring Harbor Laboratory, Cold Spring Harbor, NY, 1986.

DEBR86:
Debro, L, PC Fitz-James, and A Aronson,
J Bacteriol (1986), 165:258-68.

DENH78:
Denhardt, DT, D Dressler, and DS Ray editors,
The Spring-Stranded DNA Phages, Cold Spring Harbor Laboratory, 1978.

DEVO78:
DeVore, DP, and RJ Gruebel,
Biochem Biophys Res Commun (1978), 80(4)993-9.

DICK83:
Dickerson, RE, and I Geis,
Hemoglobin: Structure, Function, Evolution, and Pathology.,
The Bejamin/Cummings Publishing Co., Menlo Park, CA., 1983.

DILL87:
Dill, KA,
Protein Engineering (1987), 1:369-371.

DONO87
Donovan, W, Z Liangbiao, K Sandman, and R Losick,
J Mol Biol (1987), 196:1-10.

DUFT85:
Dufton, MJ,
Eur J Biochem (1985), 153:647-654.

EISE85:
Eisenbeis, SJ, MS Nasoff, SA Noble, LP Bracco, DR Dodds, MH Caruthers,
Proc. Natl. Acad. Sci. USA (1985), 82:1084-1088.

ENDE78:
Endermann, R, C Kramer, and U Henning,
FEBS Letters (1978), 86:21-24.

EPST63:
Epstein , CJ, RF Goldberger, and CB Anfinsen,
Cold Spr. Harb. Symp. Quant. Biol. (1963), 28:439ff.

ERIC86:
Erickson, BW, SB Daniels, PA Reddy, CG Unson, JS Richardson, and DC Richardson,
Current Communications in Molecular Biology: Computer Graphics and Molecular Modeling.,
Cold Spring Harbor Laboratory, Cold Spring Harbor, NY, 1986,

Fletterick, R and M Zoller, Editors.

ERRI88:
Errington, J, S Rong, MS Rosenkranz, and AL Sonenshein,
J Bacteriology (1988), 170:1162-1167.

FERE80a:
Ferenci, T, J Brass, and W Boos,
Biochem Soc Trans (1980), 8:680-1.

FERE80b:
Ferenci, T, and W Boos,
J Supramol Struct (1980), 13:101-16.

FERE80c:
Ferenci, T,
Eur J Biochem (1980), 108:631-6.

FERE82a:
Ferenci, T,
Ann. Microbiol. (Inst. Pasteur) (1982), 133A:167-169.

FERE82b:
Ferenci, T, and K-S Lee,
J. Mol. Biol. (1982), 160:431-444.

FERE83:
Ferenci, T, and KS Lee,
J Bacteriol. (1983), 154:984-987.

FERE86a:
Ferenci, T, and K-S Lee,
J. Bacteriol. (1986), 166:95-99.

FERE86b:
Ferencei, T, and K-S Lee,
J. Bacteriol. (1986), 167:1081-1082.

FERE86c:
Ferenci, T, M Muir, K-S Lee, and D Maris,
Biochimica et Biophysica Acta (1986), 860:44-50.

FERE87a:
Ferenci, T, and KS Lee,
Biochim Biophys Acta (1987), 896:319-22.

FERE87b:
Ferenci, T, TJ Silhavy,
J Bacteriol (1987), 169:5339-42.

FIOR85:
Fioretti, E, G Iacopino, M Angeletti, D Barra, F Bossa, and F Ascoli,
J Biol Chem (1985), 260:11451-11455.

FRIT85:
Fritz, H-J, in
DNA Cloning, Editor: DM Glover, IRL Press, Oxford, UK, 1985.
Volume I, Chapter 8, p151-163.

GABA82:
Gabay, J, and M Schwartz,
J Biol Chem (1982), 257(12)6627-6630.

GARA83:
Garavito, RM, J Jenkins, JN Jonsonius, R Karlsson, and JP Rosenbusch,
J Mol Biol (1983), 164:313-327.

GEHR87:
Gehring, K, A Charbit, E Brissaud, and M Hofnung,
J Bacteriol (1987), 169(5)2103-2106.

GOLD83:
Goldenberg, DP, and TE Creighton,
J Mol Biol (1983), 165: (2) p407-13.

GOLD87:
Gold, L, and G Stormo,
Volume 2, Chapter 78, p. 1302-1307, in
Escherichia coli and Salmonella typhimurium: Cellular and Molecular Biology,
Neidhardt, FC, Editor-in-Chief,
Amer. Soc. for Microbiology, Washington, DC, 1987.

GOTT87:
Gottesman, S,
Volume 2, Chapter 79, p. 1308-1312, in
Escherichia coli and Salmonella typhimurium: Cellular and Molecular Biology,
Neidhardt, FC, Editor-in-Chief,
Amer. Soc. for Microbiology, Washington, DC, 1987.

HAYA76:
Hayashi, K, M Takechi, N Kaneda, and T Sasaki,
FEBS Lett (1976), 66(2)210-4.

HEIN87:
Heine, HG, J Kyngdon, and T Ferenci,
Gene (1987), 53:287-92.

HEIN88:
Heine, HG, G Francis, KS Lee, and T Ferenci,
J Bacteriol (April 1988), 170:1730-8.

HERR78:
Herrmann, R, K Neugebauer, H Schaller, and H Zentgraf, in The Single-Stranded DNA Phages, Denhardt, DT, D Dressler, and DS Ray editors, Cold Spring Harbor Laboratory, 1978., p473-476.

HICK88:
Hickman, RK, and SB Levy,
J Bacteriol (1988), 170(4)1715-1720.

HINE80:
Hines, JC, and DS Ray,
Gene (1980), 11:(3-4)207-18.

HOGL83:
Hogle, J, T Kirchhausen, and SC Harrison,
J. Mol. Biol. (1983), 171:95-100.

HOLL83:
Hollecker, M, and TE Creighton,
J. Mol. Biol. (1983), 168:409-437.


HOOP87:
Hoopes, BC, and WR McClure,
Volume 2, Chapter 75, p 1231-1240, in
Escherichia coli and Salmonella typhimurium: Cellular and Molecular Biology,
Neidhardt, FC, Editor-in-Chief,
Amer. Soc. for Microbiology, Washington, DC, 1987.


HUBE77:
Huber, R, W Bode, D Kukla, U Kohl, CA Ryan,
Biophys Struct Mech (1975), 1(3)189-201


INOU86:
Inouye, M, and R Sarma, Editors,
Protein Engineering: Applications in Science, Medicine, and Industry.,
Academic Press, New York, 1986.


ITOK79:
Ito, K, G Mandel, and W Wickner,
Proc. Natl. Acad. Sci. USA (1979), 76:1199-1203.


JANI85:
Janin, J, and C Chothia,
Methods in Enzymology (1985), 115(28)420-430.


JAZW73a:
Jazwinski, SM, R Marco, and A Kornberg,
Proc Natl Acad Sci USA (1973), 70(1)205-9.


JAZW73b:
Jazwinski, SM, R Marco, and A Kornberg,
Virology (1975), 66(1)294-305.


JAZW74:
Marco, R, SM Jazwinski, and A Kornberg,
Virology (1974), 62:(1)209-23.


JONE85:
Jones, TA,
Methods Enzymol (1985), 115:157-71.


JONE87:
Jones, KA, JT Kadonaga, PJ Rosenfeld, TJ Kelly, and R Tjian,
Cell (Jan 16 1987), 48:79-89.


JOUB80:
Joubert, FJ, and N Taljaard,
Hoppe-Seyler's Z. Physiol. Chem. (1980), 361:661-674.


KABS84:
Kabsch, W, and C Sander,
Proc Natl Acad Sci USA (1984), 81(4)1075-8.


KADO86:
Kadonaga, JT, and R Tjian,

Proc Natl Acad Sci USA (Aug 1986), 83 (16) 5889-93.

KAIS87:
Kaiser, CA, D Preuss, P Grisafi, and D Botstein,
Science (1987), 235:312-317.

KANE76:
Kaneda, N, T Sasaki, and K Hayashi,
FEBS Lett (1976), 70(1)217-22.

KAPL78:
Kaplan, DA, L Greenfield, and G Wilcox,
in The Single-Stranded DNA Phages, Denhardt, DT, D Dressler, and DS Ray editors, Cold Spring Harbor Laboratory, 1978., p461-467.

KUHN85a:
Kuhn, A, and W Wickner,
J. Biol. Chem. (1985), 260:15914-15918.

KUHN85b:
Kuhn, A, and W Wickner,
J. Biol. Chem. (1985), 260:15907-15913.

KUHN87:
Kuhn, A,
Science (1987), 238:1413-1415.

LAND87:
Landick, R, and C Yanofsky,
Volume 2, Chapter 77, p 1276-1301,
Escherichia coli and Salmonella typhimurium: Cellular and Molecular Biology,
Neidhardt, FC, Editor-in-Chief,
Amer. Soc. for Microbiology, Washington, DC, 1987.

LEEB71:
Lee, B, and FM Richards,
J Mol Biol (1971), 55:(3)379-400,

LEEC86:
Lee, C, and J Beckwith,
Ann. Rev. Cell Biol. (1986), 2:315-336.

LOSI86:
Losick, R, P Youngman, and PJ Piggot,
Ann Rev Genet (1986), 20:625-669.

MAKE80:
Makela, O, H Sarvas, and I Seppala,
J. Immunol. Methods (1980), 37:213-223.

MAKO80:
Makowski, L, DLD Caspar, and DA Marvin,
J. Mol. Biol. (1980), 140:149-181.

MALA64:
Malamay, MH, and BL Horecker,
Biochem (1964), 3:1889-1893.

MANI82:
Maniatis, T, EF Fritsch, and J. Sambrook,
Molecular Cloning,
Cold Spring Harbor Laboratory, 1982.

MANO86:
Manoil, C, and J Beckwith,
Science (1986), 232:1403-1408.

MARC83:
Marchal, C, and M Hofnung,
EMBO J (1983), 2:81-86.

MARK86:
Marks, CB, M Vasser, P Ng, W Henzel, and S Anderson,
J. Biol. Chem. (1986), 261:7115-7118.

MARK87:
Marks, CB, H Naderi, PA Kosen, ID Kuntz, and S Anderson,
Science (1987), 235:1370-1373.

MARQ83:
Marquart, M, J Walter, J Deisinhoffer, W Bode, and R Huber,
Acta Cryst, B (1983), 39:480ff.

MARV78:
Marvin, DA,
in The Single-Stranded DNA Phages, Denhardt, DT, D Dressler, and DS Ray editors, Cold Spring Harbor Laboratory, 1978., p583-603.

MCPH86:
McPheeters, DS, A Christensen, ET Young, G Stormo, and L Gold,
Nucleic Acids Res (1986), 14:5813-26.

MESS77:
Messing, J, B Gronenborn, B Muller-Hill, and PH Hofschneider,
Proc Natl Acad Sci USA (1977), 74:3642-6.

MESS78:
Messing, J, and B Gronenborn,
in The Single-Stranded DNA Phages, Denhardt, DT, D Dressler, and DS Ray editors, Cold Spring Harbor Laboratory, 1978.,p449-453.

MICH86:
Michaelis, S, JF Hunt, and J Beckwith,
J. Bacteriol. (1986), 167:160-167.

MILL72:
Miller, JH,
Experiments in Molecular Genetics.
Cold Spring Harbor Laboratory, Cold Spring Harbor, NY. 1972

MILL87a:
Miller, S, J Janin, AM Lesk, and C Chothia,
J Mol Biol (1987), 196:641-656.

MILL87b:
Miller, ES, J Karam, M Dawson, M Trojanowska, P Gauss, and L Gold,

J Mol Biol (1987), 194:397-410.

MILL88:
Miller, J, JA Hatch, S Simonis, and SE Cullen,
Proc Natl Acad Sci USA (1988), 85:1359-1363.

MOSE83:
Moser, R, RM Thomas, and B Gutte,
FEBS Letters (1983), 157:247-251.

MOSE85:
Moser, R, S Klauser, T Leist, H Langen, T Epprecht, and B Gutte,
Angew. Chemie, Internatl Eng Ed. (1985), 24:719-798.

MOSE87:
Moser, R, S Frey, K Muenger, T Hehlgans, S Klauser, H Langen, E-L Winnacker, R Mertz, and B Gutte,
Protein Engineering (1987), 1:339-343.

NAKA86:
Nakae, T, J. Ishii, and T Ferenci,
J. Biol. Chem. (1986), 261:622-626.

NAKA87:
Nakamura, T, T Hirai, F Tokunaga, S Kawabata, and S Iwanaga,
J Biochem. (1987), 101:1297-1306.

NEID87:
Neidhardt, FC, Editor-in-Chief,
Escherichia coli and Salmonella typhimurium: Cellular and Molecular Biology,
Amer. Soc. for Microbiology, Washington, DC, 1987.

NEUH65:
Neu, HC, and LA Heppel,
J Biol Chem (1965), 240:3685-3692.

NIKA84:
Nikaido, H, and HCP Wu,
Proc Natl Acad Sci USA (1984), 81:1048-1052.

NIKA87:
Nikaido, H, and M Vaara,
Volume 1, Chapter 3, p7-22.
Escherichia coli and Salmonella typhimurium: Cellular and Molecular Biology,
Neidhardt, FC, Editor-in-Chief,
Amer. Soc. for Microbiology, Washington, DC, 1987.

NOMU78:
Nomura, N, A Oka, M Takanami, and H Yamagishi,
in The Single-Stranded DNA Phages, Denhardt, DT, D Dressler, and DS Ray editors, Cold Spring Harbor Laboratory, 1978., p467-472.

OHKA81:
Ohkawa, I, and RE Webster,
J. Biol. Chem. (1981), 256:9951-9958.

OHTA76:
Ohta, M, T Sasaki, and K Hayashi,
FEBS Lett (1976), 72(1)161-6.

OLIP86:
Oliphant, AR, AL Nussbaum, and K Struhl,
Gene (1986), <u>44</u>:177-183.

OLIP87:
Oliphant, AR, and K Struhl
<u>Methods in Enzymology</u> <u>155</u> (1987) p 568-582. Editor Wu, R; Academic Press, New York.

OLIV85:
Oliver, D,
Ann. Rev. Microbiol. (1985), <u>39</u>:615-648.

OLIV87:
Oliver, DB,
Volume 1, Chapter 6, p 56-69, in
<u>Escherichia coli and Salmonella typhimurium: Cellular and Molecular Biology,</u>
Neidhardt, FC, Editor-in-Chief,
Amer. Soc. for Microbiology, Washington, DC, 1987.

PABO79:
Pabo, CO, RT Sauer, JM Sturtevant, and M Ptashne,
Proc. Natl. Acad. Sci. USA (1979), <u>76</u>:1608-1612.

PADL85:
Padlan, EA, and WE Love,
J Biol Chem (1985), <u>260</u> (14) p8272-9.

PAKU86:
Pakula, AA, VB Young, and RT Sauer,
Proc. Natl. Acad. Sci. USA (1986), <u>83</u>:8829-8833.

PALV79:
Palva, ET, and P Westermann,
FEBS Letters (1979), <u>99</u>:77-80.

PAPA82:
Papamokos, E, E Weber, W Bode, R Huber, MW Empie, I Kato, and M Laskowski Jr.,
J Mol Biol (1982), <u>158</u>:515.

PARD81:
Pardoe, IU, and ATH Burness,
J Gen Virol (1981), <u>57</u>:239-243.

POTE83:
Poteete, AR,
J Mol Biol (1983), <u>171</u>:401-418.

PRIV86:
Privalov, PL, YV Griko, SY Venyaminov, and VP Kutyshenko,
J Mol Biol (1986), <u>190</u>(3)487-98.

QUIO87:
Quiocho, FA, NK Vyas, JS Sack and MA Storey,
in <u>Crystallography in Molecular Biology,</u> Moras, D. <u>et al.</u>, editors, Plenum Press, 1987.

RAOS87:
Rao SN, UC Singh, PA Bash, and PA Kollman
Nature (1987), <u>328</u> (6130) p551-4.

RASC86:
Rasched, I, and E Oberer,
Microbial. Rev. (1986) 50:401-427.

RASH84:
Rashin, A,
Biochemistry (1984), 23:5518.

RAYC87:
Ray, C, KM Tatti, CH Jones, and CP Moran Jr,
J Baceriol (1987), 169(5)1807-1811.

RAYG86:
Ray, GL, and WG Haldenwang,
J Bact (1986), 166:472-78.

REID88:
Reidhaar-Olson, JF, and RT Sauer,
Science (1988), 241:53-57.

RICH81:
Richardson, JS,
Adv. Protein Chemistry (1981), 34:167-339.

RICH86:
Richards, JH,
Nature (1986), 323:187.

ROAM80:
Roa, M, and JM Clement,
FEBS Letters (1980), 121:127-129.

ROBE86:
Roberts, S, and AR Rees
Protein Engineering (1986), 1:59-65.

RODR82:
Rodriguez, RL,
Gene (1982), 20:305-316.

ROSE85:
Rose, GD,
Methods in Enzymololgy (1985), 115(29)430-440.

ROSS81:
Rossman, M, and P Argos,
Ann. Rev. Biochem. (1981), 50:497ff.

RUSS81:
Russel, M, and P Model,
Proc. Natl. Acad. Sci. USA (1981), 78:1717-1721.

SABB88:
Subbarao, MN, and D Kennell,
J Bact (1988), 170:2860-2865.

SAIK85:
Saiki, RK, S Scharf, F Faloona, KB Mullis, GT Horn, HA Erlich, and N Arnheim,

Science (1985), 230:1350-1354.

SALI64:
Salivar, WO, H Tzagoloff, and D Pratt,
Virology (1964), 24:359-71.

SASA84:
Sasaki, T,
FEBS Lett. (1984), 168:227-230.

SCHA78:
Schaller, H, E Beck, and M Takanami,
The Single-Stranded DNA Phages, Denhardt, D.T., D. Dressler, and D.S. Ray editors, Cold Spring Harbor Laboratory, 1978., p139-163.

SCHA86:
Scharf, SJ, GT Horn, and HA Erlich,
Science (1986), 233:1076-1078.

SCHO84:
Schold, M, A Colombero, AA Reyes, and RB Wallace,
DNA (1984), 3(6)469-477.

SCHU79:
Schulz, GE, and RH Schirmer,
Principles of Protein Structure,
Springer-Verlag, New York, 1979.

SCHW87:
Schwarz, H, HJ Hinz, A Mehlich, H Tschesche, and HR Wenzel,
Biochemistry (1987), 26:(12)p3544-51.

SCOT87:
Scott, MJ, CS Huckaby, I Kato, WJ Kohr, M Laskowski Jr., M-J Tsai and BW O'Malley,
J Biol Chem (1987), 262(12)5899-5907.

SERW87:
Serwer, P,
J. Chromatography (1987), 418:345-357.

SHOR81:
Shortle, D, D DiMaio, and D Nathans,
Ann. Rev. Genet. (1981), 15:265-294.

SHOR85:
Shortle, D, and B Lin,
Genetics (1985), 110:539-555.

SMIT85:
Smith GP,
Science (1985), 228:1315-1317.

SMIT87a:
Smith M,
Protein Structure, Folding, and Design 2, 1987.
Ed. D Oxender (New York, AR Liss Inc.) p.395ff.

SMIT87b:

Smith, H, S Bron, J van Ee, and G Venema,
J Bacteriol. (1987), 169:3321-3328.


STAT87:
States, DJ, TE Creighton, CM Dobson, and M Karplus,
J Mol Biol (1987), 195: (3) p731-9.


STRY81:
Strydom, DJ, and FJ Joubert,
Hoppe-Seyler's Z. Physiol. Chem. (1981), 362:1377-1384.


SUDH85:
Sudhof, TC, JL Goldstein, MS Brown, and DW Russell,
Science (1985), 228:815-822.


SURE87:
Surewicz, WK, AG Szabo, HH Mantsch,
Eur J Biochem (1987), 167(3)519-523.


SUTC87a:
Sutcliffe, MJ, I Haneef, D Carney, and TL Blundell,
Protein Engineering (1987), 1:377-384.


SUTC87b:
Sutcliffe, MJ, FRF Hayes, and TL Blundell,
Protein Engineering (1987), 1:385-392.


SUZU83:
Suzuki, T and K Shikama,
Arch Biochem Biophys (1983), 224(2)695-9.


TAKA74:
Takahashi, H, S Iwanage, T Kitagawa, Y Hokama, and T Suzuki,
J Biochem (1974), 76:721-733.


TANK77:
Tan, NH, and ET Kaiser,
Biochemistry (1977), 16:1531-1541.


THER88:
Theriault, NY, JB Carter, and SP Pulaski,
BioTechniques (1988), 6(5)470-473.


THOR88:
Thornton, JM, BL Sibinda, MS Edwards, and DJ Barlow,
Bioessays Feb-Mar 1988, 8(2) 63-9.


TOTH86:
Toth MJ, and P Schimmel,
J Biol. Chem. (1986), 261:6643-6646.


TSCH87:
Tschesch, H, J Beckmann, A Mehlich, E Schnabel, E Truscheit, and HR Wenzel,
Biochimica et Biophysica Acta (1987), 913:97-101.


ULME83:
Ulmer, KM
Science (1983), 219(4585)666-71.

VITA84:
Vita, C, D Dalzoppo, and A Fontana,
Biochemistry (1984), 23:5512-5519.

WACH80:
Wachter, E, K Deppner, and K Hochstrasser,
FEBS Letters (1980), 119:58-62.

WAGN78:
Wagner, G, K Wuthrich, and H Tschesche,
Eur J Biochem (1978), 89:367-377.

WANG87:
Wagner, G, D Bruhwiler, and K Wuthrich,
J Mol Biol (1987), 196:(1) p227-31.

WAIT83:
Waite, JH,
J Biol Chem (1983), 258(5)2911-5.

WAIT85:
Waite, JH, TJ Housley, and ML Tanzer,
Biochemistry (1985), 24(19)5010-4.

WAIT86:
Waite, JH,
J Comp Physiol [B] (1986), 156(4)491-6.

WAND79:
Wandersman, C, M Schwartz, and T Ferenci,
J Bact (1979), 140 (1) p1-13.

WARD86:
Ward, WH, DH Jones, and AR Fersht,
J Biol Chem (1986), 261(21)9576-8.

WATS87:
Molecular Biology of the Gene, Fourth Edition,
Watson, JD, NH Hopkins, JW Roberts, JA Steitz, and AM Weiner,
Benjamin/Cummings Publishing Company, Inc., Menlo Park, CA., 1987.

WEBS78:
Webster, RE, and JS Cashman,
The Single-Stranded DNA Phages, Denhardt, DT,
D Dressler, and DS Ray editors, Cold Spring Harbor Laboratory, 1978., p557-569.

WELL87a:
Wells, JA, BC Cunningham, TP Graycar, and DA Estell,
Proc. Natl. Acad. Sci. USA (1987), 84:5167-5171.

WELL87b:
Wells, JA, DB Powers, RR Bott, TP Graycar, and DA Estell,
Proc. Natl. Acad. Sci. USA (1987), 84:1219-1223.

WETZ86:
Wetzel, R,
Protein Engineering (1986), 1:3-6.

WHAR86:
Wharton, RP,
The Binding Specificity Determinants of 434 Repressor.,
Harvard U. PhD Thesis, 1986,
University Microfilms, Ann Arbor, Michigan.

WILK84:
Wilkinson, AJ, AR Fersht, DM Blow, P Carter, and G Winter,
Nature (1984), 307:187-188.

WINT87:
Winter, RB, L Morrissey, P Gauss, L Gold, T Hsu, and J Karam,
Proc Natl Acad Sci USA (1987), 84:7822-6.

WISH75:
Wishner, BC, KB Ward, EE Lattman, and WE Love,
J Mol Biol (1975), 98:179-194.

WISH76:
Wishner, BC, JC Hanson, WM Ringle, and WE Love,
Proc. of the Symp. on Molecular Cellular Aspects of Sickle Cell Disease. DHEW Publication 76-1007, Natl Inst Health, Bethesda, Md., p1-31.

WLOD84:
Wlodawer, A, J Walter, R Huber, and L Sjolin,
J Mol Biol (1984), 180: (2) p301-29.

WLOD87a:
Wlodawer, A, J Nachman, GL Gilliland, W Gallagher, and C Woodward,
J Mol Biol (1987), 198 (3) p469-80.

WLOD87b:
Wlodawer, A, J Deisenhofer, and R Huber,
J Mol Biol (1987), 193: (1) p145-56.

YAGE87:
Yager, TD, and PH von Hippel,
Volume 2, chapter 76, p 1241-1275,
Escherichia coli and Salmonella typhimurium: Cellular and Molecular Biology,
Neidhardt, FC, Editor-in-Chief,
Amer. Soc. for Microbiology, Washington, DC, 1987.

ZIMM82:
Zimmermann, R, C Watts, and W Wickner,
J. Biol. Chem. (1982), 257:6529-6536.

ZOLL84:
Zoller, MJ, and M Smith,
DNA (1984), 3(6)479-488.

MESS83:
Messing, J,
Methods in Enzymology (1983), 101:20-78

YAMA70:
Yamamoto, KR, BM Alberts, R Benzinger, L Lawhorne, and G Treiber,
Virology (1970), 40:734-744

Table 2: Preferred Outer-Surface Proteins

| Genetic Package | Preferred Outer-Surface Protein | Reason for preference |
|---|---|---|
| M13 | coat protein (gpVIII) | a) exposed amino terminus, b) predictable post-translational processing, c) numerous copies in virion. |
| | gp III | a) fusion data available. |
| PhiX174 | G protein | a) known to be on virion exterior, b) small enough that the G-ipbd gene can replace H gene. |
| E. coli | LamB | a) fusion data available, b) non-essential. |
| B. subtilis spores | CotC | a) no post-translational processing, b) distinctive sdequence that causes protein to localize in spore coat, c) non-essential. |
| | CotD | Same as for CotC. |

Table 7

| Atomic radii Angstroms | |
|---|---|
| $C_{alpha}$ | 1.70 |
| $O_{carbonyl}$ | 1.52 |
| $N_{amide}$ | 1.55 |

Table 7 (continued)

| Atomic radii Angstroms | |
|---|---|
| Other atoms | 1.80 |

Table 8

| Fraction of DNA molecules having n non-parental bases when reagents that have fraction M of parental nt. | | | | | |
|---|---|---|---|---|---|
| M | .9965 | .97716 | .92612 | .8577 | .79433 | .63096 |
| f0 | .9000 | .5000 | .1000 | .0100 | .0010 | .000001 |
| f1 | .09499 | .35061 | .2393 | .04977 | .00777 | .0000175 |
| f2 | .00485 | .1188 | .2768 | .1197 | .0292 | .000149 |
| f3 | .00016 | .0259 | .2061 | .1854 | .0705 | .000812 |
| f4 | .000004 | .00409 | .1110 | .2077 | .1232 | .003207 |
| f8 | 0. | $2 \times 10^{-7}$ | .00096 | .0336 | .1182 | .080165 |
| f16 | 0. | 0. | 0. | $5 \times 10^{-7}$ | .00006 | .027281 |
| f23 | 0. | 0. | 0. | 0. | 0. | .0000089 |
| most | 0 | 0 | 2 | 5 | 7 | 12 |
| "most" is the value of n having the highest probability. | | | | | | |

Table 9: best vgCodon

```
Program "Find Optimum vgCodon."
INITIALIZE-MEMORY-OF-ABUNDANCES
DO ( t1 = 0.21 to 0.31 in steps of 0.01 )
. DO ( c1 = 0.13 to 0.23 in steps of 0.01 )
. . DO ( a1 = 0.23 to 0.33 in steps of 0.01 )
Comment        calculate g1 from other concentrations
. . . g1 = 1.0 - t1 - c1 - a1
. . . IF( g1 .ge. 0.15 )
. . . . DO ( a2 = 0.37 to 0.50 in steps of 0.01 )
. . . . . DO ( c2 = 0.12 to 0.20 in steps of 0.01 )
Comment        Force D+E = R + K
. . . . . . g2 = (g1*a2 -.5*a1*a2)/(c1+0.5*a1)
Comment        Calc t2 from other concentrations.
. . . . . . t2 = 1. - a2 - c2 - g2
. . . . . . IF(g2.gt. 0.1.and. t2.gt.0.1)
. . . . . . . CALCULATE-ABUNDANCES
. . . . . . . COMPARE-ABUNDANCES-TO-PREVIOUS-ONES
. . . . . . ..end_IF_block
. . . . . ..end_DO_loop ! c2
. . . . . ..end_DO_loop ! a2
. . . ..end_IF_block ! if g1 big enough
. . ..end_DO_loop ! a1
. ..end_DO_loop ! c1
..end_DO_loop ! t1
WRITE the best distribution and the abundances.
```

## Table 10: Abundances obtained
### from optimum vgCodon

| Amino acid | Abundance | Amino acid | Abundance |
|---|---|---|---|
| A | 4.80% | C | 2.86% |
| D | 6.00% | E | 6.00% |
| F | 2.86% | G | 6.60% |
| H | 3.60% | I | 2.86% |
| K | 5.20% | L | 6.82% |
| M | 2.86% | N | 5.20% |
| P | 2.88% | Q | 3.60% |
| R | 6.82% | S | 7.02% mfaa |
| T | 4.16% | V | 6.60% |
| W | 2.86% lfaa | Y | 5.20% |
| stop | 5.20% | | |

$$ratio = Abun(W)/Abun(S) = 0.4074$$

| j | $(1/ratio)^j$ | $(ratio)^j$ | stop-free |
|---|---|---|---|
| 1 | 2.454 | .4074 | .9480 |
| 2 | 6.025 | .1660 | .8987 |
| 3 | 14.788 | .0676 | .8520 |
| 4 | 36.298 | .0275 | .8077 |
| 5 | 89.095 | .0112 | .7657 |
| 6 | 218.7 | $4.57 \times 10^{-3}$ | .7258 |
| 7 | 536.8 | $1.86 \times 10^{-3}$ | .6881 |

lfaa = least - favored amino-acid

mfaa = most - favored amino-acid

Table 11: Calculate worst codon.

```
Program "Find worst vgCodon within Serr of given
              distribution."
INITIALIZE-MEMORY-OF-ABUNDANCES
Comment   Serr is % error level.
READ   Serr
Comment T1i,C1i,A1i,G1i, T2i,C2i,A2i,G2i, T3i,G3i
Comment are the intended nt-distribution.
READ   T1i, C1i, A1i, G1i
READ   T2i, C2i, A2i, G2i
READ   T3i, G3i
Fdwn = 1.-Serr
Fup  = 1.+Serr
DO ( t1 = T1i*Fdwn to T1i*Fup in 7 steps)
. DO ( c1 = C1i*Fdwn to C1i*Fup in 7 steps)
. . DO ( a1 = A1i*Fdwn to A1i*Fup in 7 steps)
. . . g1 = 1. - t1 - c1 - a1
. . . IF( (g1-G1i)/G1i .lt. -Serr)
Comment   g1 too far below G1i, push it back
. . . . g1 = G1i*Fdwn
. . . . factor = (1.-g1)/(t1 + c1 + a1)
. . . . t1 = t1*factor
. . . . c1 = c1*factor
. . . . a1 = a1*factor
. . . ..end_IF_block
. . . IF( (g1-G1i)/G1i .gt. Serr)
Comment   g1 too far above G1i, push it back
. . . . g1 = G1i*Fup
. . . . factor = (1.-g1)/(t1 + c1 + a1)
. . . . t1 = t1*factor
. . . . c1 = c1*factor
. . . . a1 = a1*factor
. . . ..end_IF_block
```

77

Table 11, continued.

. . . DO ( a2 = A2i*Fdwn to A2i*Fup in 7 steps)

Table 11, continued.


. . . . DO ( c2 = C2i*Fdwn to C2i*Fup in 7 steps)

. . . . . DO (g2=G2i*Fdwn to G2i*Fup in 7 steps)

Comment     Calc t2 from other concentrations.

. . . . . . t2 = 1. - a2 - c2 - g2

. . . . . . IF( (t2-T2i)/T2i .lt. -Serr)

Comment  t2 too far below T2i, push it back

. . . . . . . t2 = T2i*Fdwn

. . . . . . . factor = (1.-t2)/(a2 + c2 + g2)

. . . . . . . a2 = a2*factor

. . . . . . . c2 = c2*factor

. . . . . . . g2 = g2*factor

. . . . . . ..end_IF_block

. . . . . . IF( (t2-T2i)/T2i .gt. Serr)

Comment  t2 too far above T2i, push it back

. . . . . . . t2 = T2i*Fup

. . . . . . . factor = (1.-t2)/(a2 + c2 + g2)

-                Table 11, continued.


. . . . . . . a2 = a2*factor

. . . . . . . c2 = c2*factor

. . . . . . . g2 = g2*factor

. . . . . . ..end_IF_block

. . . . . . IF(g2.gt. 0.0 .and. t2.gt.0.0)

. . . . . . . t3 = 0.5*(1.-Serr)

. . . . . . . g3 = 1. - t3

. . . . . . . CALCULATE-ABUNDANCES

. . . . . . . COMPARE-ABUNDANCES-TO-PREVIOUS-ONES

. . . . . . . t3 = 0.5

. . . . . . . g3 = 1. - t3

Table 11, continued.

```
. . . . . . . . CALCULATE-ABUNDANCES
. . . . . . . . COMPARE-ABUNDANCES-TO-PREVIOUS-ONES
. . . . . . . t3 = 0.5*(1.+Serr)
. . . . . . . . g3 = 1. - t3
. . . . . . . CALCULATE-ABUNDANCES
```
                   Table 11, continued.

```
. . . . . . . . COMPARE-ABUNDANCES-TO-PREVIOUS-ONES
. . . . . . . ..end_IF_block
. . . . . ...end_DO_loop ! g2
. . . . ...end_DO_loop ! c2
. . . ...end_DO_loop ! a2
. . ...end_DO_loop ! a1
. ...end_DO_loop ! c1
..end_DO_loop ! t1
WRITE the WORST distribution and the abundances.
```

## Table 12: Abundances obtained using optimum vgCodon assuming 5% errors

| Amino acid | Abundance | | Amino acid | Abundance |
|------------|-----------|------|------------|-----------|
| A | 4.59% | | C | 2.76% |
| D | 5.45% | | E | 6.02% |
| F | 2.49% | lfaa | G | 6.63% |
| H | 3.59% | | I | 2.71% |
| K | 5.73% | | L | 6.71% |
| M | 3.00% | | N | 5.19% |
| P | 3.02% | | Q | 3.97% |
| R | 7.68% | mfaa | S | 7.01% |
| T | 4.37% | | V | 6.00% |
| W | 3.05% | | Y | 4.77% |
| stop | 5.27% | | | |

$$\text{ratio} = \text{Abun}(F)/\text{Abun}(R) = 0.3248$$

| i | $(1/\text{ratio})^j$ | $(\text{ratio})^j$ | stop-free |
|---|---------|---------|-----------|
| 1 | 3.079 | .3248 | .9473 |
| 2 | 9.481 | .1055 | .8973 |
| 3 | 29.193 | .03425 | .8500 |
| 4 | 89.888 | .01112 | .8052 |
| 5 | 276.78 | $3.61 \times 10^{-3}$ | .7627 |
| 6 | 852.22 | $1.17 \times 10^{-3}$ | .7225 |
| 7 | 2624.1 | $3.81 \times 10^{-4}$ | .6844 |

## Table 13: BPTI Homologues

| R # | 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|---|
| -3 | - | - | - | F | - | - | - | - | - | - | - | - | - | - | - | - | Z | - | - |
| -2 | - | - | - | Q | T | - | - | - | - | - | - | Q | - | - | - | H | G | Z | - |
| -1 | - | - | - | T | E | - | - | - | - | - | - | P | - | - | - | D | D | G | - |
| 1 | R | R | R | P | R | R | R | R | R | R | R | L | A | R | R | R | K | R | A |
| 2 | P | P | P | P | P | P | P | P | P | P | P | R | A | P | P | P | R | P | A |
| 3 | D | D | D | D | D | D | D | D | D | D | D | K | K | D | R | T | D | S | K |
| 4 | F | F | F | L | F | F | F | F | F | F | F | L | Y | F | F | F | I | F | Y |
| 5 | C | C | C | C | C | C | C | C | C | C | C | C | C | C | C | C | C | C | C |
| 6 | L | L | L | Q | L | L | L | L | L | L | L | I | K | E | E | N | R | N | K |
| 7 | E | E | E | L | E | E | E | E | E | E | E | L | L | L | L | L | L | L | L |
| 8 | P | P | P | P | P | P | P | P | P | P | P | H | P | P | P | P | P | P | P |
| 9 | P | P | P | Q | P | P | P | P | P | P | P | R | L | A | A | P | P | A | V |
| 10 | Y | Y | Y | A | Y | Y | Y | Y | Y | Y | Y | N | R | E | E | E | E | E | R |
| 11 | T | T | T | R | T | T | T | T | T | T | T | P | I | T | T | S | Q | T | Y |
| 12 | G | G | G | G | G | G | G | G | G | G | G | G | G | G | G | G | G | G | G |
| 13 | P | P | P | P | P | P | P | P | P | P | P | R | P | L | L | R | P | P | P |
| 14 | C | T | A | C | C | C | C | C | C | C | C | C | C | C | C | C | C | C | C |
| 15 | K | K | K | K | K | V | G | A | L | I | K | Y | K | K | R | K | K | K | K |
| 16 | A | A | A | A | A | A | A | A | A | A | A | Q | R | A | A | G | G | A | K |
| 17 | R | R | R | A | A | R | R | R | R | R | R | K | K | Y | R | H | R | S | K |
| 18 | I | I | I | L | M | I | I | I | I | I | I | I | I | I | I | I | L | I | F |
| 19 | I | I | I | L | I | I | I | I | I | I | I | P | P | R | R | R | P | R | P |
| 20 | R | R | R | R | R | R | R | R | R | R | R | A | S | S | S | R | R | Q | S |
| 21 | Y | Y | Y | Y | Y | Y | Y | Y | Y | Y | Y | F | F | F | F | I | Y | Y | F |
| 22 | F | F | F | F | F | F | F | F | F | F | F | Y | Y | H | H | Y | F | Y | Y |
| 23 | Y | Y | Y | Y | Y | Y | Y | Y | Y | Y | Y | Y | Y | Y | Y | Y | Y | Y | Y |
| 24 | N | N | N | N | N | N | N | N | N | N | N | N | K | N | N | N | N | N | N |
| 25 | A | A | A | S | - | A | A | A | A | A | A | Q | W | L | R | L | P | S | W |
| 26 | K | K | K | T | K | K | K | K | K | K | K | K | A | A | E | A | K | K | K |
| 27 | A | A | A | S | A | A | A | A | A | A | A | K | A | A | A | S | S | S | A |
| 28 | G | G | G | N | G | G | G | G | G | G | G | K | K | Q | Q | N | R | G | K |
| 29 | L | L | L | A | F | L | L | L | L | L | L | Q | Q | Q | Q | K | M | G | Q |
| 30 | C | C | C | C | C | C | C | C | C | C | C | C | C | C | C | C | C | C | C |
| 31 | Q | Q | Q | E | E | Q | Q | Q | Q | Q | Q | E | L | L | L | K | E | Q | L |
| 32 | T | T | T | P | T | T | T | T | T | T | T | G | P | Q | E | V | S | Q | F |
| 33 | F | F | F | F | F | F | F | F | F | F | F | F | F | F | F | F | F | F | F |
| 34 | V | V | V | T | V | V | V | V | V | V | V | T | D | I | I | F | I | I | N |
| 35 | Y | Y | Y | Y | Y | Y | Y | Y | Y | Y | Y | W | Y | Y | Y | Y | Y | Y | Y |
| 36 | G | G | G | G | G | G | G | G | G | G | G | S | S | G | G | G | G | G | S |
| 37 | G | G | G | G | G | G | G | G | G | G | G | G | G | G | G | G | G | G | G |
| 38 | C | T | A | C | C | C | C | C | C | C | C | C | C | C | C | C | C | C | C |
| 39 | R | R | R | Q | R | R | R | R | R | R | R | G | G | G | G | G | K | R | G |
| 40 | A | A | A | G | A | A | A | A | A | A | A | G | G | G | G | G | G | G | G |
| 41 | K | K | K | N | K | K | K | K | K | K | K | N | N | N | N | N | N | N | N |
| 42 | R | R | R | N | S | R | R | R | R | R | R | S | A | A | A | A | K | Q | A |
| 43 | N | N | N | N | N | N | N | N | N | N | N | N | N | N | N | N | N | N | N |

Table 13, continued.

| R # | 1 | 2 | 3 | 4 | 5 | 6 | 7 | 8 | 9 | 10 | 11 | 12 | 13 | 14 | 15 | 16 | 17 | 18 | 19 |
|-----|---|---|---|---|---|---|---|---|---|----|----|----|----|----|----|----|----|----|----|
| 44 | N | N | N | N | N | N | N | N | N | N | N | R | R | R | R | N | N | R | R |
| 45 | F | F | F | F | F | F | F | F | F | F | F | F | F | F | F | F | F | F | F |
| 46 | K | K | K | E | K | K | K | K | K | K | K | K | K | K | K | E | K | D | K |
| 47 | S | S | S | T | S | S | S | S | S | S | S | T | T | T | T | T | T | T | T |
| 48 | A | A | A | T | A | A | A | A | A | A | A | I | I | I | I | R | K | T | I |
| 49 | E | E | E | E | E | E | E | E | E | E | E | E | E | D | D | D | A | Q | E |
| 50 | D | D | D | M | D | D | D | D | D | D | D | E | E | E | E | E | E | Q | E |
| 51 | C | C | C | C | C | C | C | C | C | C | C | C | C | C | C | C | C | C | C |
| 52 | M | M | M | L | M | M | M | M | M | M | E | R | R | R | H | R | V | Q | R |
| 53 | R | R | R | R | R | R | R | R | R | R | R | R | R | R | R | E | R | G | R |
| 54 | T | T | T | I | T | T | T | T | T | T | T | T | T | T | T | T | A | V | T |
| 55 | C | C | C | C | C | C | C | C | C | C | C | C | C | C | C | C | C | C | C |
| 56 | G | G | G | E | G | G | G | G | G | G | G | I | V | V | V | G | R | V | V |
| 57 | G | G | G | P | G | G | G | G | G | G | G | R | G | G | G | G | P | – | G |
| 58 | A | A | A | P | A | A | A | A | A | A | A | K | – | – | – | K | P | – | – |
| 59 | – | – | – | Q | – | – | – | – | – | – | – | – | – | – | – | – | E | – | – |
| 60 | – | – | – | Q | – | – | – | – | – | – | – | – | – | – | – | – | R | – | – |
| 61 | – | – | – | T | – | – | – | – | – | – | – | – | – | – | – | – | P | – | – |
| 62 | – | – | – | D | – | – | – | – | – | – | – | – | – | – | – | – | – | – | – |
| 63 | – | – | – | K | – | – | – | – | – | – | – | – | – | – | – | – | – | – | – |
| 64 | – | – | – | S | – | – | – | – | – | – | – | – | – | – | – | – | – | – | – |

R # = residue number

1 BPTI
2 Engineered BPTI From MARK87
3 Engineered BPTI From MARK87
4 Bovine Colostrum (DUFT85)
5 Bovine Serum (DUFT85)
6 Semisynthetic BPTI, TSCH87
7 Semisynthetic BPTI, TSCH87
8 Semisynthetic BPTI, TSCH87
9 Semisynthetic BPTI, TSCH87
10 Semisynthetic BPTI, TSCH87
11 Engineered BPTI, AUER87
12 Dendroaspis polylepis polylepis (Black mamba) venom I
    (DUFT85)
13 Dendroaspis polylepis polylepis (Black Mamba) venom K
    (DUFT85)
14 Hemachatus hemachates (Ringhals Cobra) HHV II
    (DUFT85)
15 Naja nivea (Cape cobra) NNV II (DUFT85)
16 Vipera russelli (Russel's viper) RVV II (TAKA74)
17 Red sea turtle egg white (DUFT85)
18 Snail mucus (Helix pomania) (WAGN78)
19 Dendroaspis angusticeps (Eastern green mamba)
C13 S1 C3 toxin    (DUFT85)

Table 13, continued.

| R # | 20 | 21 | 22 | 23 | 24 | 25 | 26 | 27 | 28 | 29 | 30 | 31 | 32 | 33 |
|---|---|---|---|---|---|---|---|---|---|---|---|---|---|---|
| -5 | - | - | - | - | - | - | - | - | - | - | - | - | - | D |
| -4 | - | - | - | - | - | - | - | - | - | - | - | - | - | E |
| -3 | - | - | - | - | - | - | - | - | - | - | - | - | T | P |
| -2 | Z | - | L | Z | R | K | - | - | - | R | R | - | E | T |
| -1 | P | - | Q | D | D | N | - | - | - | Q | K | - | R | T |
| 1 | R | R | H | H | R | R | I | K | T | R | R | R | G | D |
| 2 | R | P | R | P | P | P | N | E | V | H | H | P | F | L |
| 3 | K | Y | T | K | K | T | G | D | A | R | P | D | L | P |
| 4 | L | A | F | F | F | F | D | S | A | D | D | F | D | I |
| 5 | C | C | C | C | C | C | C | C | C | C | C | C | C | C |
| 6 | I | E | K | Y | Y | N | E | Q | N | D | D | L | T | E |
| 7 | L | L | L | L | L | L | L | L | L | K | K | E | S | Q |
| 8 | H | I | P | P | P | L | P | G | P | P | P | P | P | A |
| 9 | R | V | A | A | A | P | K | Y | V | P | P | P | P | FG |
| 10 | N | A | E | D | D | E | V | S | I | D | D | Y | V | D |
| 11 | P | A | P | P | P | T | V | A | R | K | T | T | T | A |
| 12 | G | G | G | G | G | G | G | G | G | G | K | G | G | G |
| 13 | R | P | P | R | R | R | P | P | P | N | I | P | P | L |
| 14 | C | C | C | C | C | C | C | C | C | C | C | C | C | C |
| 15 | Y | M | K | K | L | N | R | M | R | - | - | K | R | F |
| 16 | D | F | A | A | A | A | A | G | A | G | Q | A | A | G |
| 17 | K | F | S | H | Y | L | R | M | F | P | T | K | G | Y |
| 18 | I | I | I | I | M | I | F | T | I | V | V | M | F | M |
| 19 | P | S | P | P | P | P | P | S | Q | R | R | I | K | K |
| 20 | A | A | A | R | R | A | R | R | L | A | A | R | R | L |
| 21 | F | F | F | F | F | F | Y | Y | W | F | F | Y | Y | Y |
| 22 | Y | Y | Y | Y | Y | Y | Y | F | A | Y | Y | F | N | S |
| 23 | Y | Y | Y | Y | Y | Y | Y | Y | F | Y | Y | Y | Y | Y |
| 24 | N | S | N | D | N | N | N | N | D | D | K | N | N | N |
| 25 | Q | K | W | S | P | S | S | G | A | T | P | A | T | Q |
| 26 | K | G | A | A | A | H | S | T | V | R | S | K | R | E |
| 27 | K | A | A | S | S | L | S | S | K | L | A | A | T | T |
| 28 | K | N | K | N | N | H | K | M | G | K | K | G | K | K |
| 29 | Q | K | K | K | K | K | R | A | K | T | R | F | Q | N |
| 30 | C | C | C | C | C | C | C | C | C | C | C | C | C | C |
| 31 | E | Y | Q | N | E | Q | E | E | V | K | V | E | E | E |
| 32 | R | P | L | K | K | K | K | T | L | A | Q | T | P | E |
| 33 | F | F | F | F | F | F | F | F | F | F | F | F | F | F |
| 34 | D | T | H | I | I | N | I | Q | P | Q | R | V | K | I |
| 35 | W | Y | Y | Y | Y | Y | Y | Y | Y | Y | Y | Y | Y | Y |
| 36 | S | S | G | G | G | G | G | G | G | R | G | G | G | G |
| 37 | G | G | G | G | G | G | G | G | G | G | G | G | G | G |
| 38 | C | C | C | C | C | C | C | C | C | C | C | C | C | C |
| 39 | G | R | K | P | R | G | G | M | Q | D | D | K | K | Q |
| 40 | G | G | G | G | G | G | G | G | G | G | G | A | G | G |
| 41 | N | N | N | N | N | N | N | N | N | D | D | K | N | N |
| 42 | S | A | A | A | A | A | A | G | G | H | H | S | G | D |
| 43 | N | N | N | N | N | N | N | N | N | G | G | N | N | N |

## Table 13, continued.

| R # | 20 | 21 | 22 | 23 | 24 | 25 | 26 | 27 | 28 | 29 | 30 | 31 | 32 | 33 |
|-----|----|----|----|----|----|----|----|----|----|----|----|----|----|----|
| 44 | R | R | R | N | N | N | N | N | K | N | N | N | R | R |
| 45 | F | F | F | F | F | F | F | F | F | F | F | F | Y | F |
| 46 | K | K | S | K | K | K | H | V | Y | K | K | R | K | S |
| 47 | T | T | T | T | T | T | T | T | S | T | S | S | S | T |
| 48 | I | I | I | W | W | I | L | E | E | E | D | A | E | L |
| 49 | E | E | E | D | D | D | E | K | K | T | H | E | Q | A |
| 50 | E | E | K | E | E | E | E | E | E | L | L | D | D | E |
| 51 | C | C | C | C | C | C | C | C | C | C | C | C | C | C |
| 52 | R | R | R | R | R | Q | E | L | R | R | R | M | L | E |
| 53 | R | R | H | Q | H | R | K | Q | E | C | C | R | D | Q |
| 54 | T | T | A | T | T | T | V | T | Y | E | E | T | A | K |
| 55 | C | C | C | C | C | C | C | C | C | C | C | C | C | C |
| 56 | I | V | V | G | V | A | G | R | G | L | E | G | S | I |
| 57 | G | V | G | A | A | A | V | – | V | V | L | G | G | N |
| 58 | – | – | – | S | S | K | R | – | P | Y | Y | A | F | – |
| 59 | – | – | – | A | G | Y | S | – | G | P | R | – | – | – |
| 60 | – | – | – | – | I | G | – | – | D | – | – | – | – | – |

20 <u>Dendroaspis angusticeps</u> (Eastern Green Mamba) C13 S2 C3 toxin (DUFT85)
21 <u>Dendroaspis polylepis polylepes</u> (Black mamba) B toxin (DUFT85)
22 <u>Dendroaspis polylepis polylepes</u> (Black Mamba) E toxin (DUFT85)
23 <u>Vipera ammodytes</u> TI toxin (DUFT85)
24 <u>Vipera ammodytes</u> CTI toxin (DUFT85)
25 <u>Bungarus fasciatus</u> VIII B toxin (DUFT85)
26 <u>Anemonia sulcata</u> (sea anemone) 5 II (DUFT85)
27 <u>Homo sapiens</u> HI-14 "inactive" domain (DUFT85)
28 <u>Homo sapiens</u> HI-14 "active" domain (DUFT85)
29 beta bungarotoxin B1 (DUFT85)
30 beta bungarotoxin B2 (DUFT85)
31 Bovine spleen TI II (FIOR85)
32 <u>Tachypleus tridentatus</u> (Horseshoe crab) hemocyte inhibitor (NAKA87)
33 <u>Bombyx mori</u> (silkworm) SCI-III (SASA84)

Notes :
  a) both beta bungarotoxins have residue 15 deleted.
  b) <u>B. mori</u> has an extra residue between C5 and C14; we have assigned F and G to residue 9.
  c) all natural proteins have C at 5, 14, 30, 38, 50, & 55.
  d) all homologues have F33 and G37.
  e) extra C's in bungarotoxins form interchain cystine bridges

Table 14

| Tally of Ionizable Groups. BPTI homologues. | | | | | | | | | | |
|---|---|---|---|---|---|---|---|---|---|---|
| Sequence Identifier | D | E | K | R | Y | H | NH | CO2 | + | # |
| 1 | 2 | 2 | 4 | 6 | 4 | 0 | 1 | 1 | 6 | 16 |
| 2 | 2 | 2 | 4 | 6 | 4 | 0 | 1 | 1 | 6 | 16 |
| 3 | 2 | 2 | 4 | 6 | 4 | 0 | 1 | 1 | 6 | 16 |
| 4 | 2 | 4 | 2 | 3 | 3 | 0 | 1 | 1 | -1 | 13 |
| 5 | 2 | 4 | 4 | 4 | 4 | 0 | 1 | 1 | 2 | 16 |
| 6 | 2 | 2 | 3 | 6 | 4 | 0 | 1 | 1 | 5 | 15 |
| 7 | 2 | 2 | 3 | 6 | 4 | 0 | 1 | 1 | 5 | 15 |
| 8 | 2 | 2 | 3 | 6 | 4 | 0 | 1 | 1 | 5 | 15 |
| 9 | 2 | 2 | 3 | 6 | 4 | 0 | 1 | 1 | 5 | 15 |
| 10 | 2 | 2 | 3 | 6 | 4 | 0 | 1 | 1 | 5 | 15 |
| 11 | 2 | 3 | 4 | 6 | 4 | 0 | 1 | 1 | 5 | 19 |
| 12 | 0 | 3 | 7 | 7 | 3 | 1 | 1 | 1 | 11 | 19 |
| 13 | 1 | 2 | 8 | 5 | 4 | 0 | 1 | 1 | 10 | 18 |
| 14 | 2 | 3 | 2 | 5 | 3 | 1 | 1 | 1 | 2 | 14 |
| 15 | 1 | 4 | 2 | 7 | 2 | 2 | 1 | 1 | 4 | 16 |
| 16 | 2 | 5 | 3 | 7 | 3 | 2 | 1 | 1 | 3 | 19 |
| 17 | 2 | 4 | 6 | 7 | 3 | 0 | 1 | 1 | 7 | 21 |
| 18 | 1 | 1 | 2 | 4 | 4 | 0 | 1 | 1 | 4 | 8 |
| 19 | 0 | 2 | 9 | 4 | 4 | 0 | 1 | 1 | 11 | 17 |
| 20 | 2 | 3 | 6 | 7 | 3 | 1 | 1 | 1 | 8 | 20 |
| 21 | 0 | 3 | 3 | 5 | 5 | 0 | 1 | 1 | 5 | 13 |
| 22 | 0 | 2 | 6 | 3 | 3 | 2 | 1 | 1 | 7 | 13 |
| 23 | 4 | 1 | 5 | 3 | 4 | 2 | 1 | 1 | 3 | 15 |
| 24 | 3 | 2 | 4 | 6 | 5 | 1 | 1 | 1 | 5 | 17 |
| 25 | 1 | 2 | 5 | 3 | 3 | 1 | 1 | 1 | 5 | 13 |
| 26 | 1 | 5 | 4 | 4 | 4 | 1 | 1 | 1 | 2 | 16 |
| 27 | 1 | 4 | 2 | 2 | 4 | 0 | 1 | 1 | -1 | 11 |
| 28 | 2 | 3 | 4 | 3 | 3 | 0 | 1 | 1 | 2 | 14 |
| 29 | 6 | 2 | 5 | 7 | 4 | 2 | 1 | 1 | 4 | 22 |
| 30 | 6 | 2 | 6 | 7 | 4 | 2 | 1 | 1 | 5 | 23 |
| 31 | 2 | 3 | 5 | 4 | 4 | 0 | 1 | 1 | 4 | 16 |
| 32 | 3 | 3 | 5 | 5 | 4 | 0 | 1 | 1 | 4 | 18 |
| 33 | 4 | 7 | 3 | 1 | 4 | 0 | 1 | 1 | -7 | 17 |
| Sequences given in Table 10. | | | | | | | | | | |

+ is sum of K + R + NH - D - E - CO2, approximate charge on molecule at pH 7.0

# is sum of K + R + NH + D + E + CO2, <u>i.e.</u> number of ionized groups at pH 7.0

### Table 15: Amino acids observed at each Residue
### BPTI homologues

| Res. # | Number Different AAs | Contents | BPTI |
|---|---|---|---|
| -5 | 2 | D -32 | - |
| -4 | 2 | E -32 | - |
| -3 | 5 | T P F Z -29 | - |
| -2 | 10 | Z3 R3 Q2 T2 H G L K E -18 | - |
| -1 | 10 | D4 T2 P2 Q2 E G N K R -18 | - |
| 1 | 10 | R21 A2 K2 H2 P L I T G D | R |
| 2 | 9 | P20 R4 A2 H2 N E V F L | P |
| 3 | 10 | D15 K6 T3 R2 P2 S Y G A L | D |
| 4 | 7 | F19 D4 L3 Y2 I2 A2 S | F |
| 5 | 1 | C33 | C |
| 6 | 10 | L11 E5 N4 K3 Q2 I2 Y2 D2 T R | L |
| 7 | 5 | L18 E11 K2 S Q | E |
| 8 | 7 | P26 H2 A2 I L G F | P |
| 9 | 9 | P17 A6 V3 R2 Q L K Y F | P |
| 10 | 10 | Y11 E7 D4 A2 N2 R2 V2 S I D | Y |
| 11 | 10 | T17 P5 A3 R2 I S Q Y V K | T |
| 12 | 2 | G32 K | G |
| 13 | 5 | P22 R6 L3 N I | P |
| 14 | 3 | C31 T A | C |
| 15 | 12 | K15 R4 Y2 M2 L2 -2 V G A I N F | K |
| 16 | 7 | A22 G5 Q2 R K D F | A |
| 17 | 12 | R12 K5 A2 Y3 H2 S2 F2 L M T G P | R |
| 18 | 6 | I21 M4 F3 L2 V2 T | I |
| 19 | 7 | I11 P10 R6 S2 K2 L Q | I |
| 20 | 5 | R19 A7 S4 L2 Q | R |
| 21 | 4 | Y18 F13 W I | Y |
| 22 | 6 | F14 Y14 H2 A N S | F |
| 23 | 2 | Y32 F | Y |
| 24 | 4 | N26 K3 D3 S | N |
| 25 | 10 | A12 S5 Q3 P3 W3 L2 T2 K G R | A |
| 26 | 9 | K16 A6 T2 E2 S2 R2 G H V | K |
| 27 | 5 | A18 S8 K3 L2 T2 | A |
| 28 | 7 | G13 K10 N5 Q2 R H M | G |
| 29 | 10 | L9 Q7 K7 A2 F2 R2 M G T N | L |
| 30 | 1 | C33 | C |
| 31 | 7 | Q12 E11 L4 K2 V2 Y N | Q |
| 32 | 11 | T12 P5 K4 Q3 E2 L2 G V S R A | T |
| 33 | 1 | F33 | F |
| 34 | 11 | V11 I8 T3 D2 N2 Q2 F H P R K | V |
| 35 | 2 | Y31 W2 | Y |
| 36 | 3 | G27 S5 R | G |
| 37 | 1 | G33 | G |
| 38 | 3 | C31 T A | C |
| 39 | 7 | R13 G9 K4 Q3 D2 P M | R |

## Table 15: continued.

| Res. # | Number Different AAs | Contents | |
|---|---|---|---|
| 40 | 2 | G22 A11 | A |
| 41 | 3 | N20 K11 D2 | K |
| 42 | 9 | A11 R9 S4 G3 H2 D Q K N | R |
| 43 | 2 | N31 G2 | N |
| 44 | 3 | N21 R11 K | N |
| 45 | 2 | F32 Y | F |
| 46 | 8 | K24 E2 S2 D H V Y R | K |
| 47 | 2 | T19 S14 | S |
| 48 | 9 | A11 I9 E4 T2 W2 L2 R K D | A |
| 49 | 7 | E19 D6 A2 Q2 K2 T H | E |
| 50 | 6 | E16 D12 L2 M Q K | D |
| 51 | 1 | C33 | C |
| 52 | 7 | R13 M10 L3 E3 Q2 H V | M |
| 53 | 8 | R21 Q3 E2 H2 C2 G K D | R |
| 54 | 7 | T23 A3 V2 E2 I Y K | T |
| 55 | 1 | C33 | C |
| 56 | 8 | G15 V8 I3 E2 R2 A L S | G |
| 57 | 8 | G19 V4 A3 P2 -2 R L N | G |
| 58 | 8 | A11 -10 P3 K3 S2 Y2 R F | A |
| 59 | 9 | -24 G2 Q E A Y S P R | - |
| 60 | 6 | -28 Q R I G D | - |
| 61 | 3 | -31 T P | - |
| 62 | 2 | -32 D | - |
| 63 | 2 | -32 K | - |
| 64 | 2 | -32 S | - |

## Table 16: Exposure in BPTI

Coordinates taken from
Brookhaven Protein Data Bank entry 6PTI.

```
HEADER     PROTEINASE INHIBITOR (TRYPSIN) 13-MAY-87   6PTI
COMPND     BOVINE PANCREATIC TRYPSIN INHIBITOR
COMPND     2(/BPTI$,CRYSTAL FORM /III$)
AUTHOR     A.WLODAWER
```

Solvent radius =   1.40
Atomic radii given in Table 7

Areas in Angstroms-squared.

| Residue | | Total area | Not Covered by M/C | fraction | Not covered at all | fraction |
|---|---|---|---|---|---|---|
| ARG | 1 | 342.45 | 205.09 | 0.5989 | 152.49 | 0.4453 |
| PRO | 2 | 239.12 | 92.65 | 0.3875 | 47.56 | 0.1989 |
| ASP | 3 | 272.39 | 158.77 | 0.5829 | 143.23 | 0.5258 |
| PHE | 4 | 311.33 | 137.82 | 0.4427 | 43.21 | 0.1388 |
| CYS | 5 | 241.06 | 48.36 | 0.2006 | 0.23 | 0.0010 |
| LEU | 6 | 280.98 | 151.45 | 0.5390 | 115.87 | 0.4124 |
| GLU | 7 | 291.39 | 128.91 | 0.4424 | 90.39 | 0.3102 |
| PRO | 8 | 236.12 | 128.71 | 0.5451 | 99.98 | 0.4234 |
| PRO | 9 | 236.09 | 109.82 | 0.4652 | 45.80 | 0.1940 |
| TYR | 10 | 330.97 | 153.63 | 0.4642 | 79.49 | 0.2402 |
| THR | 11 | 249.20 | 80.10 | 0.3214 | 64.99 | 0.2608 |
| GLY | 12 | 184.21 | 56.75 | 0.3081 | 23.05 | 0.1252 |
| PRO | 13 | 240.07 | 130.25 | 0.5426 | 75.27 | 0.3136 |
| CYS | 14 | 237.10 | 75.55 | 0.3186 | 53.52 | 0.2257 |
| LYS | 15 | 310.77 | 200.25 | 0.6444 | 192.00 | 0.6178 |
| ALA | 16 | 209.41 | 66.63 | 0.3182 | 45.59 | 0.2177 |
| ARG | 17 | 351.09 | 243.67 | 0.6940 | 201.48 | 0.5739 |
| ILE | 18 | 277.10 | 100.51 | 0.3627 | 58.95 | 0.2127 |
| ILE | 19 | 278.03 | 146.06 | 0.5254 | 96.05 | 0.3455 |
| ARG | 20 | 339.11 | 144.65 | 0.4266 | 43.81 | 0.1292 |
| TYR | 21 | 333.60 | 102.24 | 0.3065 | 69.67 | 0.2089 |
| PHE | 22 | 306.08 | 70.64 | 0.2308 | 23.01 | 0.0752 |
| TYR | 23 | 338.66 | 77.05 | 0.2275 | 17.34 | 0.0512 |
| ASN | 24 | 264.88 | 99.03 | 0.3739 | 38.69 | 0.1461 |
| ALA | 25 | 211.15 | 85.13 | 0.4032 | 48.20 | 0.2283 |
| LYS | 26 | 313.29 | 216.14 | 0.6899 | 202.84 | 0.6474 |
| ALA | 27 | 210.66 | 96.05 | 0.4560 | 54.78 | 0.2601 |
| GLY | 28 | 186.83 | 71.52 | 0.3828 | 32.09 | 0.1718 |
| LEU | 29 | 280.70 | 132.42 | 0.4718 | 93.61 | 0.3335 |
| CYS | 30 | 238.15 | 57.27 | 0.2405 | 19.33 | 0.0812 |
| GLN | 31 | 301.15 | 141.80 | 0.4709 | 82.64 | 0.2744 |
| THR | 32 | 251.26 | 138.17 | 0.5499 | 76.47 | 0.3043 |

Table 16, continued.

| | | | | | | |
|---|---|---|---|---|---|---|
| PHE | 33 | 304.27 | 59.79 | 0.1965 | 18.91 | 0.0622 |
| VAL | 34 | 251.56 | 109.78 | 0.4364 | 42.36 | 0.1684 |
| TYR | 35 | 332.64 | 80.52 | 0.2421 | 15.05 | 0.0452 |
| GLY | 36 | 187.06 | 11.90 | 0.0636 | 1.97 | 0.0105 |
| GLY | 37 | 185.28 | 84.26 | 0.4548 | 39.17 | 0.2114 |
| CYS | 38 | 234.56 | 73.64 | 0.3139 | 26.40 | 0.1125 |
| ARG | 39 | 417.13 | 304.62 | 0.7303 | 250.73 | 0.6011 |
| ALA | 40 | 209.53 | 94.01 | 0.4487 | 52.95 | 0.2527 |
| LYS | 41 | 314.60 | 166.23 | 0.5284 | 108.77 | 0.3457 |
| ARG | 42 | 349.06 | 232.83 | 0.6670 | 179.59 | 0.5145 |
| ASN | 43 | 266.47 | 38.53 | 0.1446 | 5.32 | 0.0200 |
| ASN | 44 | 269.65 | 91.08 | 0.3378 | 23.39 | 0.0867 |
| PHE | 45 | 313.22 | 69.73 | 0.2226 | 14.79 | 0.0472 |
| LYS | 46 | 309.83 | 217.18 | 0.7010 | 155.73 | 0.5026 |
| SER | 47 | 224.78 | 69.11 | 0.3075 | 24.80 | 0.1103 |
| ALA | 48 | 211.01 | 82.06 | 0.3889 | 31.07 | 0.1473 |
| GLU | 49 | 286.62 | 161.00 | 0.5617 | 100.01 | 0.3489 |
| ASP | 50 | 299.53 | 156.42 | 0.5222 | 95.96 | 0.3204 |
| CYS | 51 | 238.68 | 24.51 | 0.1027 | 0.00 | 0.0000 |
| MET | 52 | 293.05 | 89.48 | 0.3054 | 66.70 | 0.2276 |
| ARG | 53 | 356.20 | 224.61 | 0.6306 | 189.75 | 0.5327 |
| THR | 54 | 251.53 | 116.43 | 0.4629 | 51.64 | 0.2053 |
| CYS | 55 | 240.40 | 69.95 | 0.2910 | 0.00 | 0.0000 |
| GLY | 56 | 184.66 | 60.79 | 0.3292 | 32.78 | 0.1775 |
| GLY | 57 | 106.58 | 49.71 | 0.4664 | 38.28 | 0.3592 |
| ALA | 58 | no position given in Protein Data Bank | | | | |

"Total area"        is the area measured by a rolling sphere of radius 1.4 A, where only the atoms within the residue are considered. This takes account of conformation.

"Not covered by M/C"        is the area measured by a rolling sphere of radius 1.4 A where all main-chain atoms are considered, fraction is the exposed area divided by the total area. Surface buried by main-chain atoms is more definitely covered than is surface covered by side group atoms.

"Not covered at all"        is the area measured by a rolling sphere of radius 1.4 A where all atoms of the protein are considered.

Table 17

| Plasmids used in Detailed Example | |
|---|---|
| Phage | Contents |
| LG1 | M13mp18 with Ava II/Aat II/Acc I/Rsr II/Sau I adaptor |
| pLG2 | LG1 with amp$^R$ and ColE1 of pBR322 cloned into Aat II/Acc I sites |
| pLG3 | pLG2 with Acc I site removed |
| pLG4 | pLG3 with first part of osp-pbd gene cloned into Rsr II/Sau I sites, Avr II/Asu II sites created |
| pLG5 | pLG4 with second part of osp-pbd gene cloned into Avr II/Asu II sites, BssH I site created |
| pLG6 | pLG5 with third part of osp-pbd gene cloned into Asu II/BssH I sites, Bbe I site created |
| pLG7 | pLG6 with last part of osp-pbd gene cloned into Bbe I/Asu II sites |
| pLG8 | pLG7 with disabled osp-pbd gene, same length DNA. |
| pLG9 | pLG7 mutated to display BPTI(V15$_{BPTI}$) |
| pLG10 | pLG8 + tet$^R$ gene - amp$^R$ gene |
| pLG11 | pLG9 + tet$^R$ gene - amp$^R$ gene |

## Table 25: Annotated Sequence of ipbd gene

```
5'- C|GGA|CCG|TAT|CCA|GGC|TTT|ACA|CTT|TAT|
28
        | Rsr II |              |  -35  |


|GCT|TCC|GGC|TCG|TAT|AAT|GTG|TGG|              52
              |  -10  |


      |AAT|TGT|GAG|CGG|ATA|ACA|ATT|            73
      |   lac operator          |


      |CCT|AGG|AGG|CTC|ACT|                     88
      | Avr II|
        | S. D. |


| m | k | k | s | l | v | l | k | a | s |
| 1 | 2 | 3 | 4 | 5 | 6 | 7 | 8 | 9 | 10|
|ATG|AAG|AAA|TCT|CTG|GTT|CTT|AAG|GCT|AGC|      118
                      | Afl II| Nhe I |


| v | a | v | a | t | l | v | p | m | l |
| 11| 12| 13| 14| 15| 16| 17| 18| 19| 20|
|GTT|GCT|GTC|GCG|ACC|CTG|GTA|CCG|ATG|CTG|      148
      | Nru I|        | Kpn I|


| s | f | a | r | p | d | f | c | l | e |
| 21| 22| 23| 24| 25| 26| 27| 28| 29| 30|
|TCT|TTT|GCT|CGT|CCG|GAT|TTC|TGT|CTC|GAG|      178
```

Table 25, continued.

|AccIII|       | Ava I |

| Xho I |

| p | p | y | t | g | p | c | k | a | r |
| 31| 32| 33| 34| 35| 36| 37| 38| 39| 40|
|CCG|CCA|TAT|ACT|GGG|CCC|TGC|AAA|GCG|CGC|     208
| PflM I . |         |BssH II|
| Apa I |
| Dra II |
| Pss I |  -

| i | i | r | y | f | y | n | a | k |
| 41| 42| 43| 44| 45| 46| 47| 48| 49|
|ATC|ATC|CGT|TAT|TTC|TAC|AAC|GCT|AAA|     235

Table 25, continued.

| a | g | l | c | q | t | f | v | y | g | g |
|---|---|---|---|---|---|---|---|---|---|---|
| 50| 51| 52| 53| 54| 55| 56| 57| 58| 59| 60|
|GCA|GGC|CTG|TGC|CAG|ACC|TTT|GTA|TAC|GGT|GGT|

|    Stu I|                                    |    Acc I  |
                                               |    Xca I  |

268


| c | r | a | k | r | n | n | f | k |
|---|---|---|---|---|---|---|---|---|
| 61| 62| 63| 64| 65| 66| 67| 68| 69|
|TGC|CGT|GCT|AAG|CGT|AAC|AAC|TTT|AAA|

|    Esp I   |

295


| s | a | e | d | c | m | r | t | c | g |
|---|---|---|---|---|---|---|---|---|---|
| 70| 71| 72| 73| 74| 75| 76| 77| 78| 79|
|TCG|GCC|GAA|GAT|TGC|ATG|CGT|ACC|TGC|GGT|

|XmaIII|                |    Sph I|

325


| g | a | a | e | g | d | d |
|---|---|---|---|---|---|---|
| 80| 81| 82| 83| 84| 85| 86|
|GGC|GCC|GCT|GAA|GGT|GAT|GAT|

|    Bbe I  |
|    Nar I  |

346


| p | a | k | a | a |
|---|---|---|---|---|
| 87| 88| 89| 90| 91|
|CCG|GCC|AAA|GCG|GCC|

|     Sfi I             |

361

```
| f | n | s | l | q | a | s | a | t |
```

Table 25, continued.

```
| 92| 93| 94| 95| 96| 97| 98| 99|100|
|TTT|AAC|TCT|CTG|CAA|GCT|TCT|GCT|ACC|        388
                |Hind 3|
```

```
| e | y | i | g | y | a | w |
|101|102|103|104|105|106|107|
|GAA|TAT|ATC|GGT|TAC|GCG|TGG|                 409
            | Mlu I|
```

```
| a | m | v | v | v |
|108|109|110|111|112|
|GCC|ATG|GTG|GTG|GTT|                         424
   |   BstX I        |
   | Nco I|
```

## Table 25, continued.

```
| i | v | g | a | t | i | g | i |
|113|114|115|116|117|118|119|120|
|ATC|GTT|GGT|GCT|ACC|ATC|GGT|ATC|                    448


| k | l | f | k | k | f | t | s | k | a |
|121|122|123|124|125|126|127|128|129|130|
|AAA|CTG|TTT|AAG|AAA|TTT|ACT|TCG|AAA|GCG|            478
                                |Asu II|  -


| s | . | . | . |
|131|132|133|134|
|TCT|TAA|TAG|TGA|GGT|TAC|CAG|TCT|                    502
                | BstE II|


|AAG|CCC|GCC|TAA|TGA|GCG|GGC|TTT|TTT|TTT|            532
        |   Trp terminator                    |


|CCT|GAG|G -3'                                       539
| Sau I  |
```

Note the following enzyme equivalences,

Xma III   = Eag I
Acc III   = BspM II
Dra II    = EcoO109 I
Asu II    = BstB I
Sau I     = Bsu36 I

## Table 27: DNA_synth1

5′ |CCG|TCC|GTC|GGA|CCG|TAT|CCA|GGC|TTT|ACA|CTT|TAT|

|GCT|TCC|GGC|TCG|TAT|AAT|GTG|TGG|

|AAT|TGT|GAG|CGG|ATA|ACA|ATT|
olig#4 = 3′- gt taa

|CCT|AGG|
gga tcc

/ 3′ = olig#3
|GCC|GCT|CCT|TCG|AAA|GCG|
cgg cga gga agc ttt cgc

|TCT|TAA|TAG|TGA|GGT|TAC|CAG|TCT|
aga att atc act cca atg gtc aga

|AAG|CCC|GCC|TAA|TGA|GCG|GGC|TTT|TTT|TTT|
ttc ggg cgg att act cgc ccg aaa aaa aaa

|CCT|GAG|GCA|GGT|GAG|CG
gga ctc cgt cca ctc gc - 5′

Table 27, continued.

"Top" strand          99
"Bottom" strand       100
Overlap               23   (14 c/g and 9 a/t)
Net length            158

## Table 28: DNA_seq2

```
5'- |gca|cca|acg|
    | spacer    |


|CCT|AGG|AGG|CTC|ACT|
| Avr II|
    | S. D. |


| m | k | k | s | l | v | l | k | a | s |
| 1 | 2 | 3 | 4 | 5 | 6 | 7 | 8 | 9 | 10|
|ATG|AAG|AAA|TCT|CTG|GTT|CTT|AAG|GCT|AGC|
                            | Afl II| Nhe I |


| v | a | v | a | t | l | v | p | m | l |
| 11| 12| 13| 14| 15| 16| 17| 18| 19| 20|
|GTT|GCT|GTC|GCG|ACC|CTG|GTA|CCG|ATG|CTG|
        | Nru I|  -    | Kpn I|  - -- -


| s | f | a | r | p | d | f | c | l | e |
| 21| 22| 23| 24| 25| 26| 27| 28| 29| 30|
|TCT|TTT|GCT|CGT|CCG|GAT|TTC|TGT|CTC|GAG|
            |AccIII|            | Ava I |
                                | Xho I |


| p | p | y | t | g | p | c | k | a | r |
| 31| 32| 33| 34| 35| 36| 37| 38| 39| 40|
|CCG|CCA|TAT|ACT|GGG|CCC|TGC|AAA|GCG|CGC|
    | PflM I      |              |BssH II|
```

Table 28, continued.

```
| Apa I |
| Dra II |
| Pss I  |
```

```
| i | i | r |
| 41| 42| 43|
|atc|atc|cgt|
```

```
| t | s | k |
|127|128|129|
|ACT|TCG|AAa|gcg|gct|gcg| - 3'
   |Asu II|  spacer     |
```

## Table 30: DNA_seq3

```
                                          | a | r |
                                          | 39| 40|
                       5'- |ccc|tgc|aca|GCG|CGC|
                           |   spacer    |BssH II|
```

```
| i | i | r | y | f | y | n | a | k |
| 41| 42| 43| 44| 45| 46| 47| 48| 49|
|ATC|ATC|CGT|TAT|TTC|TAC|AAC|GCT|AAA|
```

```
| a | g | l | c | q | t | f | v | y | g | g |
| 50| 51| 52| 53| 54| 55| 56| 57| 58| 59| 60|
|GCA|GGC|CTG|TGC|CAG|ACC|TTT|GTA|TAC|GGT|GGT|
      | Stu I|                 | Acc I |
                               | Xca I |
```

```
| c | r | a | k | r | n | n | f | k |
| 61| 62| 63| 64| 65| 66| 67| 68| 69|
|TGC|CGT|GCT|AAG|CGT|AAC|AAC|TTT|AAA|
          | Esp I  |
```

```
| s | a | e | d | c | m | r | t | c | g |
| 70| 71| 72| 73| 74| 75| 76| 77| 78| 79|
|TCG|GCC|GAA|GAT|TGC|ATG|CGT|ACC|TGC|GGT|
    |XmaIII|              | Sph I|
```

```
| g | a |
| 80| 81|
```

Table 30, continued.

```
|GGC|GCC|gct|gaa|
| Bbe I | spacer
| Nar I |
```

```
                    | t | s | k |
                    |127|128|129|
         |ttt|acT|TCG|AAa|gcg|tcg|ccg|  - 3'
                    |Asu II|
```

Table 32: DNA_seq4

```
                | g | a | a | e | g | d | d |
5'              | 80| 81| 82| 83| 84| 85| 86|
|cct|cgc|cct|GGC|GCC|GCT|GAA|GGT|GAT|GAT|
|   spacer   | Bbe I |
              | Nar I |
```

```
| p | a | k | a | a |
| 87| 88| 89| 90| 91|
|CCG|GCC|AAA|GCG|GCC|
   | Sfi I          |
```

```
| f | n | s | l | q | a | s | a | t |
| 92| 93| 94| 95| 96| 97| 98| 99|100|
|TTT|AAC|TCT|CTG|CAA|GCT|TCT|GCT|ACC|
              |Hind 3|
```

```
| e | y | i | g | y | a | w |
|101|102|103|104|105|106|107|
|GAA|TAT|ATC|GGT|TAC|GCG|TGG|
              | Mlu I|
```

```
| a | m | v | v | v |
|108|109|110|111|112|
|GCC|ATG|GTG|GTG|GTT|
   | BstX I        |
   | Nco I|
```

Table 32, continued.

```
| i | v | g | a | t | i | g | i |
|113|114|115|116|117|118|119|120|
|ATC|GTT|GGT|GCT|ACC|ATC|GGT|ATC|


| k | l | f | k | k | f | t | s | k |
|121|122|123|124|125|126|127|128|129|
|AAA|CTG|TTT|AAG|AAA|TTT|ACT|TCG|AAa|gcg|tcg|ggc| - 3'
                                        |Asu II| spacer        |
```

Table 34: Some interaction sets in BPTI

| Res. # | Number Diff. AAs | Contents | BPTI | 1 | 2 | 3 | 4 | 5 |
|---|---|---|---|---|---|---|---|---|
| -5 | 2 | D -32 | — | | | | | |
| -4 | 2 | E -32 | — | | | | | |
| -3 | 5 | T P F Z -29 | — | | | | | |
| -2 | 10 | Z3 R3 Q2 T2 H G L K E -18 | — | | | | | |
| -1 | 10 | D4 T2 P2 Q2 E G N K R -18 | — | | | | | |
| 1 | 10 | R21 A2 K2 H2 P L I T G D | R | | | | | 5 |
| 2 | 9 | P20 R4 A2 H2 N E V F L | P | | | | s | 5 |
| 3 | 10 | D15 K6 T3 R2 P2 S Y G A L | D | | | | 4 | s |
| 4 | 7 | F19 D4 L3 Y2 I2 A2 S | F | | | | s | 5 |
| 5 | 1 | C33 | C | | | | x | x |
| 6 | 10 | L11 E5 N4 K3 Q2 I2 Y2 D2 T R | L | | | | 4 | |
| 7 | 5 | L18 E11 K2 S Q | E | | | s | 4 | |
| 8 | 7 | P26 H2 A2 I L G F | P | | | 3 | 4 | |
| 9 | 9 | P17 A6 V3 R2 Q L K Y F | P | | s | 3 | 4 | |
| 10 | 10 | Y11 E7 D4 A2 N2 R2 V2 S I D | Y | s | | s | 4 | |
| 11 | 10 | T17 P5 A3 R2 I S Q Y V K | T | l | s | 3 | 4 | |
| 12 | 2 | G32 K | G | x | | x | x | |

Table 34, continued.

| | | | | | | | | |
|---|---|---|---|---|---|---|---|---|
| 13 | 5 | P22 R6 L3 N I | P | 1 | | s | 4 | s |
| 14 | 3 | C31 T A | C | 1 | | s | s | 5 |
| 15 | 12 | K15 R4 Y2 M2 L2 -2 V G A I N F | K | 1 | s | 3 | 4 | s |
| 16 | 7 | A22 G5 Q2 R K D F | A | 1 | s | s | s | 5 |
| 17 | 12 | R12 K5 A2 Y3 H2 S2 F2 L M T G P | R | 1 | 2 | 3 | | s |
| 18 | 6 | I21 M4 F3 L2 V2 T | I | 1 | s | s | | 5 |
| 19 | 7 | I11 P10 R6 S2 K2 L Q | I | 1 | 2 | 3 | | s |
| 20 | 5 | R19 A7 S4 L2 Q | R | | s | s | s | 5 |
| 21 | 4 | Y18 F13 W I | Y | | 2 | s | s | s |
| 22 | 6 | F14 Y14 H2 A N S | F | | s | 3 | 4 | |
| 23 | 2 | Y32 F | Y | | | s | s | |
| 24 | 4 | N26 K3 D3 S | N | | s | 3 | | |
| 25 | 10 | A12 S5 Q3 P3 W3 L2 T2 K G R | A | | s | s | | |
| 26 | 9 | K16 A6 T2 E2 S2 R2 G H V | K | | s | 3 | 4 | |
| 27 | 5 | A18 S8 K3 L2 T2 | A | | 2 | 3 | 4 | |
| 28 | 7 | G13 K10 N5 Q2 R H M | G | | 2 | s | s | |
| 29 | 10 | L9 Q7 K7 A2 F2 R2 M G T N | L | | 2 | 3 | | |
| 30 | 1 | C33 | C | | x | x | x | |
| 31 | 7 | Q12 E11 L4 K2 V2 Y N | Q | | 2 | 3 | 4 | |
| 32 | 11 | T12 P5 K4 Q3 E2 L2 G V S R A | T | | 2 | 3 | s | |
| 33 | 1 | F33 | F | x | x | x | x | |
| 34 | 11 | V11 I8 T3 D2 N2 Q2 F H P R K | V | 1 | 2 | 3 | s | |
| 35 | 2 | Y31 W2 | Y | s | s | s | | 5 |
| 36 | 3 | G27 S5 R | G | 1 | | | | |
| 37 | 1 | G33 | G | x | | | | x |
| 38 | 3 | C31 T A | C | 1 | | | s | 5 |
| 39 | 7 | R13 G9 K4 Q3 D2 P M | R | 1 | | | 4 | s |

Table 34, continued.

| 40 | 2 | G22 A11 | | | | | | | | A | s | | s | 5 |
| 41 | 3 | N20 K11 D2 | | | | | | | | K | | | 4 | s |
| 42 | 9 | A11 R9 S4 G3 H2 D Q K N | | | | | | | | R | | | s | 5 |
| 43 | 2 | N31 G2 | | | | | | | | N | | | s | |
| 44 | 3 | N21 R11 K | | | | | | | | N | | | s | |
| 45 | 2 | F32 Y | | | | | | | | F | | | s | |
| 46 | 8 | K24 E2 S2 D H V Y R | | | | | | | | K | | | | 5 |
| 47 | 2 | T19 S14 | | | | | | | | S | s | | | 5 |
| 48 | 9 | A11 I9 E4 T2 W2 L2 R K D | | | | | | | | A | | 2 s | s | |
| 49 | 7 | E19 D6 A2 Q2 K2 T H | | | | | | | | E | | 2 | s | |
| 50 | 6 | E16 D12 L2 M Q K | | | | | | | | D | s | | | 5 |
| 51 | 1 | C33 | | | | | | | | C | x | | x | |
| 52 | 7 | R13 M10 L3 E3 Q2 H V | | | | | | | | M | | 2 | s | |
| 53 | 8 | R21 Q3 E2 H2 C2 G K D | | | | | | | | R | s | | | 5 |
| 54 | 7 | T23 A3 V2 E2 I Y K | | | | | | | | T | | | | 5 |
| 55 | 1 | C33 | | | | | | | | C | | | x | |
| 56 | 8 | G15 V8 I3 E2 R2 A L S | | | | | | | | G | | | | |
| 57 | 8 | G19 V4 A3 P2 -2 R L N | | | | | | | | G | | | | |
| 58 | 8 | A11 -10 P3 K3 S2 Y2 R F | | | | | | | | A | | | | |
| 59 | 9 | -24 G2 Q E A Y S P R | | | | | | | | - | | | | |
| 60 | 6 | -28 Q R I G D | | | | | | | | - | | | | |
| 61 | 3 | -31 T P | | | | | | | | - | | | | |
| 62 | 2 | -32 D | | | | | | | | - | | | | |
| 63 | 2 | -32 K | | | | | | | | - | | | | |
| 64 | 2 | -32 S | | | | | | | | - | | | | |

s   indicates secondary set

x   indicates in or close to surface but buried and/or highly
    conserved.

Table 35

| Distances from $C_{beta}$ to Tip of Side Group in Angstroms | |
| --- | --- |
| Amino Acid type | Distance |
| A | 0.0 |
| C (reduced) | 1.8 |
| D | 2.4 |

Table 35 (continued)

| Distances from $C_{beta}$ to Tip of Side Group in Angstroms | |
|---|---|
| Amino Acid type | Distance |
| E | 3.5 |
| F | 4.3 |
| G | - |
| H | 4.0 |
| I | 2.5 |
| K | 5.1 |
| L | 2.6 |
| M | 3.8 |
| N | 2.4 |
| P | 2.4 |
| Q | 3.5 |
| R | 6.0 |
| S | 1.5 |
| T | 1.5 |
| V | 1.5 |
| W | 5.3 |
| Y | 5.7 |
| Notes: These distances were calculated for standard model parts with all side groups fully extended. | |

## Table 36: Distances, BPTI residue set #2
Distances in Angstroms between $C_{beta}s$.
Hypothetical $C_{beta}$ was added to each Glycine.

| | R17 | I19 | Y21 | A27 | G28 | L29 | Q31 | T32 | V34 | A48 |
|-----|------|------|------|------|------|------|------|------|------|------|
| I19 | 7.7 | | | | | | | | | |
| Y21 | 15.1 | 8.4 | | | | | | | | |
| A27 | 22.6 | 17.1 | 12.2 | | | | | | | |
| G28 | 26.6 | 20.4 | 13.8 | 5.3 | | | | | | |
| L29 | 22.5 | 15.8 | 9.6 | 5.1 | 5.2 | | | | | |
| Q31 | 16.1 | 10.4 | 6.8 | 6.8 | 10.6 | 6.8 | | | | |
| T32 | 11.7 | 5.2 | 6.1 | 12.0 | 15.5 | 10.9 | 5.4 | | | |
| V34 | 5.6 | 6.5 | 11.6 | 17.6 | 21.7 | 18.0 | 11.4 | 8.2 | | |
| A48 | 18.5 | 11.0 | 5.4 | 12.6 | 13.3 | 8.4 | 8.8 | 8.3 | 15.7 | |
| E49 | 22.0 | 14.7 | 8.9 | 16.9 | 16.1 | 12.2 | 13.9 | 13.3 | 19.8 | 5.5 |
| M52 | 23.6 | 16.3 | 8.6 | 12.2 | 10.3 | 7.6 | 11.3 | 13.2 | 20.0 | 6.2 |
| P9 | 14.0 | 11.3 | 9.0 | 12.2 | 15.4 | 13.3 | 7.9 | 9.2 | 8.7 | 13.9 |
| T11 | 9.5 | 11.2 | 13.5 | 18.8 | 22.5 | 19.8 | 13.5 | 12.1 | 5.7 | 18.5 |
| K15 | 7.9 | 14.6 | 20.1 | 27.4 | 31.3 | 27.9 | 21.4 | 18.1 | 10.3 | 24.6 |
| A16 | 5.5 | 10.1 | 15.9 | 25.2 | 28.5 | 24.6 | 18.6 | 14.5 | 8.6 | 19.8 |
| I18 | 6.1 | 6.0 | 11.2 | 21.3 | 24.4 | 20.2 | 14.7 | 10.4 | 7.0 | 15.0 |
| R20 | 10.6 | 5.9 | 5.4 | 16.0 | 18.5 | 14.6 | 9.8 | 6.9 | 7.8 | 10.2 |
| F22 | 15.6 | 10.9 | 5.6 | 10.5 | 12.8 | 10.3 | 6.2 | 8.1 | 10.8 | 10.3 |
| N24 | 19.9 | 14.7 | 9.4 | 4.1 | 7.3 | 6.1 | 4.8 | 10.0 | 14.7 | 11.4 |
| K26 | 24.4 | 20.1 | 15.2 | 5.4 | 7.7 | 9.8 | 10.1 | 15.3 | 19.0 | 17.0 |
| C30 | 18.9 | 12.1 | 4.6 | 8.8 | 9.5 | 5.3 | 5.9 | 8.2 | 14.9 | 4.9 |
| F33 | 10.8 | 7.4 | 7.7 | 12.6 | 16.4 | 13.0 | 6.6 | 5.6 | 5.5 | 12.2 |
| Y35 | 8.4 | 7.4 | 9.4 | 18.4 | 21.4 | 17.9 | 12.2 | 9.5 | 5.8 | 14.4 |
| S47 | 17.6 | 10.6 | 6.6 | 17.3 | 17.9 | 13.4 | 12.6 | 10.4 | 15.9 | 5.3 |
| D50 | 20.0 | 13.6 | 7.2 | 17.2 | 16.8 | 13.5 | 13.5 | 12.9 | 17.6 | 7.6 |
| C51 | 18.9 | 12.2 | 4.0 | 12.1 | 12.2 | 8.8 | 8.8 | 9.7 | 15.3 | 5.4 |
| R53 | 25.4 | 18.6 | 11.0 | 17.2 | 15.0 | 13.0 | 15.7 | 16.7 | 22.3 | 9.7 |
| R39 | 15.4 | 16.9 | 17.1 | 24.9 | 27.2 | 24.9 | 20.1 | 18.7 | 13.8 | 22.3 |

Table 36, continued.
Distances in Angstroms between $C_{beta}$s.
Hypothetical $C_{beta}$ was added to each Glycine.

|      | E49  | M52  | P9   | T11  | K15  | A16  | I18  | R20  | F22  | N24  |
|------|------|------|------|------|------|------|------|------|------|------|
| M52  | 6.1  |      |      |      |      |      |      |      |      |      |
| P9   | 17.7 | 15.5 |      |      |      |      |      |      |      |      |
| T11  | 22.1 | 21.5 | 7.2  |      |      |      |      |      |      |      |
| K15  | 27.5 | 28.7 | 16.4 | 9.5  |      |      |      |      |      |      |
| A16  | 22.2 | 24.2 | 14.9 | ,9.8 | 6.2  |      |      |      |      |      |
| I18  | 17.4 | 19.5 | 12.2 | 9.5  | 10.4 | 4.9  |      |      |      |      |
| R20  | 13.0 | 13.8 | 8.0  | 9.4  | 14.9 | 10.6 | 6.2  |      |      |      |
| F22  | 13.8 | 11.4 | 4.1  | 10.6 | 19.1 | 16.3 | 12.7 | 6.9  |      |      |
| N24  | 15.6 | 11.2 | 8.4  | 15.3 | 24.1 | 21.9 | 18.2 | 12.7 | 6.6  |      |
| K26  | 20.9 | 15.7 | 12.1 | 18.6 | 27.9 | 26.6 | 23.3 | 18.1 | 11.6 | 5.9  |
| C30  | 8.7  | 5.6  | 10.6 | 16.6 | 24.1 | 20.2 | 15.7 | 9.8  | 6.8  | 6.9  |
| F33  | 16.5 | 15.4 | 4.2  | 7.1  | 15.0 | 12.8 | 9.6  | 6.1  | 5.6  | 9.3  |
| Y35  | 17.2 | 17.8 | 7.8  | 5.8  | 11.0 | 7.6  | 4.9  | 4.3  | 8.8  | 14.8 |
| S47  | 4.7  | 9.1  | 15.3 | 18.5 | 23.1 | 17.6 | 12.8 | 9.1  | 12.0 | 15.3 |
| D50  | 5.5  | 7.7  | 14.7 | 18.6 | 24.2 | 19.2 | 14.7 | 9.9  | 11.0 | 14.7 |
| C51  | 7.1  | 5.4  | 11.0 | 16.4 | 23.5 | 19.2 | 14.6 | 8.7  | 6.9  | 9.6  |
| R53  | 6.3  | 5.6  | 17.9 | 23.1 | 29.6 | 24.8 | 20.3 | 15.0 | 13.8 | 15.5 |
| R39  | 23.9 | 24.0 | 13.0 | 9.5  | 12.0 | 11.8 | 12.5 | 12.8 | 14.7 | 20.8 |

|      | K26  | C30  | F33  | Y35  | S47  | D50  | C51  | R53  |
|------|------|------|------|------|------|------|------|------|
| C30  | 12.4 |      |      |      |      |      |      |      |
| F33  | 13.9 | 10.1 |      |      |      |      |      |      |
| Y35  | 19.5 | 13.5 | 6.4  |      |      |      |      |      |
| S47  | 21.0 | 8.8  | 13.5 | 13.2 |      |      |      |      |
| D50  | 20.1 | 8.6  | 14.3 | 13.7 | 5.0  |      |      |      |
| C51  | 15.0 | 3.7  | 10.9 | 12.5 | 6.9  | 5.2  |      |      |
| R53  | 19.9 | 9.9  | 18.2 | 18.8 | 9.4  | 5.8  | 7.4  |      |
| R39  | 24.3 | 20.6 | 14.4 | 9.6  | 20.4 | 19.0 | 18.8 | 23.4 |

### Table 37: vgDNA to vary BPTI set #2.1

|  |  |  | g | p | c | k | a | +<br>X |  |
|---|---|---|---|---|---|---|---|---|---|
|  |  |  | 35 | 36 | 37 | 38 | 39 | 40 |  |
| 5'- | CAC | CCT | GGG | CCC | TGC | AAA | GCG | qfk | 208 |
|  | \|spacer | Apa I \| |  |  |  |  |  |  |  |

|  | + |  |  |  |  |  |  |  |  |
|---|---|---|---|---|---|---|---|---|---|
| i | X | r | y | f | y | n | a | k |  |
| 41 | 42 | 43 | 44 | 45 | 46 | 47 | 48 | 49 |  |
| ATC | qfk | CGT | TAT | TTC | TAC | AAC | GCT | AAA | 235 |

/ 3' = olig#27  72 nts

|  |  |  |  |  |  |  |  |  |  |  |  |
|---|---|---|---|---|---|---|---|---|---|---|---|
| + | ! | + |  |  |  |  | \| | + |  |  |  |
| X | g | X | c | q | t | f | X | y | g | g |  |
| 50 | 51 | 52 | 53 | 54 | 55 | 56 | 57 | 58 | 59 | 60 |  |
| qfk | GGt | qfk | TGC | CAG | ACC | TTc | qfk | TAC | GGT | GGT | 268 |

olig#28= 3'- acg gtc tgg aag **m atg cca cca
78 nts

Overlap = 12 (7 CG, 5 AT)

|  |  |  |  |  |  |  |  |  |  |
|---|---|---|---|---|---|---|---|---|---|
| c | r | a | k | r | n | n | f | k |  |
| 61 | 62 | 63 | 64 | 65 | 66 | 67 | 68 | 69 |  |
| TGC | CGT | GCT | AAG | CGT | AAC | AAC | TTT | AAA | 295 |
| acg | gca | cga | ttc | gca | ttg | ttg | aaa | ttt |  |
|  | \| Esp I | \| |  |  |  |  |  |  |  |

|  | + |  |  |  |  |  |
|---|---|---|---|---|---|---|
| s | X | e | d | c | m |  |
| 70 | 71 | 72 | 73 | 74 | 75 |  |
| TCT | qfk | GAG | GAT | TGC | ATG | C  322 |
| agc | **m | ctc | cta | acg | tac | gca ccc acc  -5' |
|  | \| Sph I\| | spacer  \| |  |  |  |  |

k = equal parts of T and G; m = equal parts of C and A;
q = (.26 T, .18 C, .26 A, and .30 G);
f = (.22 T, .16 C, .40 A, and .22 G);
* = complement of symbol above

Residue              40    42    50    52    57    71
Possibilities    21 x 21 x 21 x 21 x 21 x 21  = $8.6 \times 10^7$
Abundance x 10:
of PPBD          .768 .271 .459 .671 .600 .459
Produce = $1.77 \times 10^{-8}$

Parent = $1/(5.5 \times 10^7)$    least favored = $1/(4.2 \times 10^9)$
Least favored one-amino-acid substitution from PPBD present
at 1 in $1.6 \times 10^7$

## Table 38: Result of varying set#2 of BPTI 2.1

| | l | e |
|---|---|---|
| | 29 | 30 |
| | CTC | GAG |
| | Ava | I |
| | Xho | I |

178

| p | p | y | t | g | p | c | k | a | D |
|---|---|---|---|---|---|---|---|---|---|
| 31 | 32 | 33 | 34 | 35 | 36 | 37 | 38 | 39 | 40 |
| CCG | CCA | TAT | ACT | GGG | CCC | TGC | AAA | GCG | GAT |

PflM I
Apa I
Dra II
Pss I

208

| i | Q | r | y | f | y | n | a | k |
|---|---|---|---|---|---|---|---|---|
| 41 | 42 | 43 | 44 | 45 | 46 | 47 | 48 | 49 |
| ATC | CAG | CGT | TAT | TTC | TAC | AAC | GCT | AAA |

235

| E | g | L | c | q | t | f | S | y | g | g |
|---|---|---|---|---|---|---|---|---|---|---|
| 50 | 51 | 52 | 53 | 54 | 55 | 56 | 57 | 58 | 59 | 60 |
| GAG | GGC | CTG | TGC | CAG | ACC | TTT | TCG | TAC | GGT | GGT |

268

| c | r | a | k | r | n | n | f | k |
|---|---|---|---|---|---|---|---|---|
| 61 | 62 | 63 | 64 | 65 | 66 | 67 | 68 | 69 |
| TGC | CGT | GCT | AAG | CGT | AAC | AAC | TTT | AAA |

Esp I

295

| s | W | e | d | c | m | r | t | c | g |
|---|---|---|---|---|---|---|---|---|---|
| 70 | 71 | 72 | 73 | 74 | 75 | 76 | 77 | 78 | 79 |
| TCG | TGG | GAA | GAT | TGC | ATG | CGT | ACC | TGC | GGT |

Sph I

325

| g | a |
|---|---|
| 80 | 81 |
| GGC | GCC |
| Bbe | I |
| Nar | I |

Table 39: vgDNA to vary set#2 BPTI 2.2

|  |  |  | + |  |  |  |
|---|---|---|---|---|---|---|
| g | p | c | X | a | D |  |
| 35 | 36 | 37 | 38 | 39 | 40 |  |
| 5' - cg gca cgc GGG | CCC | TGC | mrA | GCG | GAT | 208 |
| \| spacer \| | Apa I \| |  |  |  |  |  |

| + |  | + | + |  |  |  |  |  |
|---|---|---|---|---|---|---|---|---|
| X | Q | X | X | f | y | n | a | k |
| 41 | 42 | 43 | 44 | 45 | 46 | 47 | 48 | 49 |
| rwA | CAG | rvk | TwT | TTC | TAC | AAC | GCT | AAA |
| 235 |  |  |  |  |  |  |  |  |

|  | + |  |  | + | + |  |  |  |  |  |
|---|---|---|---|---|---|---|---|---|---|---|
| E | X | L | c | X | X | f | S | y | g | g |
| 50 | 51 | 52 | 53 | 54 | 55 | 56 | 57 | 58 | 59 | 60 |
| GAG | qfk | CTG | TGC | qfk | qfk | TTT | TCG | TAC | GGT | GGT |
| 268 |  |  |  |  |  |  |  |  |  |  |

91 nts olig#30 3'- g cca cca

Overlap = 15 (11 CG, 4 AT)

/- 3' olig#29 94 nts

| c | r | a | k | r | n | n | f | k |
|---|---|---|---|---|---|---|---|---|
| 61 | 62 | 63 | 64 | 65 | 66 | 67 | 68 | 69 |
| TGC | CGT | GCT | AAG | CGT | AAC | AAC | TTT | AAA |
| acg | gca | cga | ttc | gca | ttg | ttg | aaa | ttt |
| \| Esp I \| |  |  |  |  |  |  |  |  |

295

|  |  | + |  |  |  |
|---|---|---|---|---|---|
| s | W | X | d | c | m |
| 70 | 71 | 72 | 73 | 74 | 75 |
| TCG | TGG | qfk | GAT | TGC | ATG | C |
| agc | acc | **m | cta | acg | tac gcg acc tgc -5' |
| \| Sph I\| spacer \| |  |  |  |  |  |

k = equal parts of T and G; v = equal parts of C, A, and G;
m = equal parts of C and A; r = equal parts of A and G;
w = equal parts of A and T;
q = (.26 T, .18 C, .26 A, and .30 G);
f = (.22 T, .16 C, .40 A, and .22 G);
* = complement of symbol above

| Residue | 38 | 41 | 43 | 44 | 51 | 54 | 55 | 72 |
|---|---|---|---|---|---|---|---|---|
| Possibilities | 4 x | 4 x | 9 x | 2 x | 21 x | 21 x | 21 x | 21 |

$$= 6.2 \times 10^7$$

Abundance x 10 2.5 2.5 .833 5. .663 .397 .437 .602
Product = $2.3 \times 10^{-8}$

Parent = $1/(4.4 \times 10^7)$ least favored = $1/(1.25 \times 10^9)$
Least favored one-amino-acid substitution from PPBD present
at 1 in $1.2 \times 10^7$

## Table 40: Result of varying set#2 of BPTI 2.2

```
                                          |  l  |  e  |
                                          | 29  | 30  |
                                          | CTC | GAG |                 178
                                          |   Xho  I  |

| p  | p  | y  | t  | g  | p  | c  | E  | a  | D  |
| 31 | 32 | 33 | 34 | 35 | 36 | 37 | 38 | 39 | 40 |
|CCG |CCA |TAT |ACT |GGG |CCC |TGC |GAG |GCG |GAT |                      208
|    PflM  I      |
                 |  Apa  I  |

| V  | Q  | N  | F  | f  | y  | n  | a  | k  |
| 41 | 42 | 43 | 44 | 45 | 46 | 47 | 48 | 49 |
|GTT |CAG |AAT |TTT |TTC |TAC |AAC |GCT |AAA |                          235

| E  | F  | L  | c  | S  | A  | f  | S  | y  | g  | g  |
| 50 | 51 | 52 | 53 | 54 | 55 | 56 | 57 | 58 | 59 | 60 |
|GAG |TTT |CTG |TGC |TCT |GCT |TTT |TCG |TAC |GGT |GGT |                 268

| c  | r  | a  | k  | r  | n  | n  | f  | k  |
| 61 | 62 | 63 | 64 | 65 | 66 | 67 | 68 | 69 |
|TGC |CGT |GCT |AAG |CGT |AAC |AAC |TTT |AAA |                          295
|   Esp  I   |

| s  | W  | Q  | d  | c  | m  | r  | t  | c  | g  |
| 70 | 71 | 72 | 73 | 74 | 75 | 76 | 77 | 78 | 79 |
|TCG |TGG |CAG |GAT |TGC |ATG |CGT |ACC |TGC |GGT |                      325
                      |  Sph  I|

|  g  |  a  |
| 80  | 81  |
| GGC | GCC |
|   Bbe  I  |
|  Nar  I   |
```

## Table 41: vg DNA set#2 of BPTI 2.3

|  |  | l | e |
|---|---|---|---|
|  |  | 29 | 30 |

5'- cg agc ctg | CTC | GAG |       178
    | spacer | Xho I |

|  | + |  | + |  |  |  |  |  | + |
|---|---|---|---|---|---|---|---|---|---|
| p | X | y | X | g | p | c | E | a | X |
| 31 | 32 | 33 | 34 | 35 | 36 | 37 | 38 | 39 | 40 |
| CCG | vmq | TAT | vmq | GGG | CCC | TGC | GAG | GCG | qfk |

      208

|  |  |  | + | ' |  |  |  |  |
|---|---|---|---|---|---|---|---|---|
| V | Q | N | X | f | y | n | a | k |
| 41 | 42 | 43 | 44 | 45 | 46 | 47 | 48 | 49 |
| GTT | CAG | AAT | Tdk | TTC | TAC | AAC | GCc | AAq |

-3' olig#33  71 nts

67 nts olig#34 3'- g atg ttg cgg ttc

Overlap = 13 (7 CG, 6 AT)

| + |  | + |  |  | + |  | + |  |  |  |
|---|---|---|---|---|---|---|---|---|---|---|
| X | F | X | c | S | X | f | X | y | g | g |
| 50 | 51 | 52 | 53 | 54 | 55 | 56 | 57 | 58 | 59 | 60 |
| vAG | TTT | nTk | TGC | TCT | qfk | TTT | qfk | TAC | GGT | GGT |
| btc | aaa | nam | acg | aga | **m | aaa | **m | atg | cca | cca |

      268

| c | r | a | k |  |
|---|---|---|---|---|
| 61 | 62 | 63 | 64 |  |
| TGC | CGT | GCT | AAG | C |
| acg | gca | cga | ttc | gcg acc ggc |

   | Esp I | spacer |

k = equal parts of T and G; m = equal parts of C and A;
w = equal parts of A and T; n = equal parts of A,C,G,T;
d = equal parts A,G,T;      v = equal parts A,C,G;
q = (.26 T, .18 C, .26 A, and .30 G);
f = (.22 T, .16 C, .40 A, and .22 G);
* = complement of symbol above

Residue        32   34   40   44   50   52   55   57
Possibilities   6 x  6 x 21 x  6 x  3 x  5 x 21 x 21 =
                                              $3 \times 10^7$

Abundance x 10
of PPBD        10/6 10/6 .545  10/6 10/3 30/8 .459 .701
product = $1.01 \times 10^{-7}$

parent = $1/(1 \times 10^7)$      least favored = $1/(4 \times 10^8)$
Least favored one-amino-acid substitution from PPBD present
at 1 in $3 \times 10^7$

## Table 42: Result of varying set#2 of BPTI 2.3

| l | e |
|---|---|
| 29 | 30 |
| CTC | GAG |
| Ava I | |
| Xho I | |

178

| p | E | y | Q | g | p | c | E | a | A |
|---|---|---|---|---|---|---|---|---|---|
| 31 | 32 | 33 | 34 | 35 | 36 | 37 | 38 | 39 | 40 |
| CCG | GAG | TAT | CAG | GGG | CCC | TGC | GAG | GCG | GCT |
| | | | | Apa I | | | | | |

208

| V | Q | N | W | f | y | n | a | k |
|---|---|---|---|---|---|---|---|---|
| 41 | 42 | 43 | 44 | 45 | 46 | 47 | 48 | 49 |
| GTT | CAG | AAT | TGG | TTC | TAC | AAC | GCT | AAA |

235

| Q | F | M | c | S | L | f | H | y | g | g |
|---|---|---|---|---|---|---|---|---|---|---|
| 50 | 51 | 52 | 53 | 54 | 55 | 56 | 57 | 58 | 59 | 60 |
| CAG | TTT | ATG | TGC | TCT | CTT | TTT | CAT | TAC | GGT | GGT |

268

| c | r | a | k | r | n | n | f | k |
|---|---|---|---|---|---|---|---|---|
| 61 | 62 | 63 | 64 | 65 | 66 | 67 | 68 | 69 |
| TGC | CGT | GCT | AAG | CGT | AAC | AAC | TTT | AAA |
| | Esp I | | | | | | | |

295

| s | W | Q | d | c | m | r | t | c | g |
|---|---|---|---|---|---|---|---|---|---|
| 70 | 71 | 72 | 73 | 74 | 75 | 76 | 77 | 78 | 79 |
| TCG | TGG | CAG | GAT | TGC | ATG | CGT | ACC | TGC | GGT |
| | | | | Sph I | | | | | |

325

| g | a |
|---|---|
| 80 | 81 |
| GGC | GCC |
| Bbe I | |
| Nar I | |

**Claims**

1. A method of obtaining a nucleic acid encoding a proteinaceous binding domain that binds a predetermined target which comprises

   (a) providing a population of amplifiable genetic packages, said genetic packages being selected from the group consisting of cells, spores and viruses, each said genetic package being genetically alterable and having an outer surface, said outer surface including a genetically determined outer surface protein, each said genetic package including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or

114

more identifiable surface residues, and for which both an affinity molecule and an amino acid sequence are either available or obtainable, and (ii) an outer surface transport signal for obtaining the display of the potential binding domain on the outer surface of the genetic package, expression of which construct results in the display of said chimeric potential binding protein and its potential binding domain on the outer surface of said genetic package; and wherein said population of genetic packages collectively display the genetic message encapsulated by said genetic packages being amplifiable *in vitro* or by cell culture of said genetic packages or host cells transformed therewith and separable on the basis of the potential binding domain displayed thereon,

(b) causing the expression of said chimeric potential binding proteins and the display of said potential binding domains on the outer surface of said packages;

(c) contacting said packages with predetermined target material such that said potential binding domains and the target material may interact;

(d) separating packages displaying a potential binding domain that binds the target material from packages that do not so bind on the basis of their ability to bind with the target material in step (c), and

(e) recovering at least one package displaying on its outer surface a chimeric binding protein comprising a stable successful binding domain (SBD) which bound said target, said package comprising nucleic acid encoding said successful binding domain, and amplifying said SBD-encoding nucleic acid *in vivo* or *in vitro;*

with the proviso that when the parental protein is a single chain antibody, (I) the genetic package is a filamentous phage, and/or (II) the outer surface of the genetic package presents, not only said chimeric protein, but also the cognate wild type outer surface protein.

2. The method of claim 1, wherein said population is a variegated population of amplifiable genetic packages, which collectively display a plurality of different potential binding domains, the differentiation among said plurality of different potential binding domains occurring through the at least partially random variation of one or more predetermined amino acid positions of said parental binding domain to randomly obtain at each said position an amino acid belonging to a predetermined set of two or more amino acids, the amino acids of said set occurring at said position in statistically predetermined expected proportions.

3. A chimeric protein comprising (i) at least a segment of an outer surface protein of a virus, said segment providing an outer surface transport signal recognized by a cell infected by said virus such that the chimeric protein is assembled into the coat of the virus particles produced by said cell, and (ii) a stable, proteinaceous binding domain, other than a single chain antibody, said domain comprising one or more identifiable surface residues, which is sufficiently stable in structure to have a melting point of at least 40°C, and which domain binds a predetermined target material, the target being bound sufficiently strongly so that the dissociation constant of the binding domain: target complex is less than $10^{-6}$ moles/liter, and that is heterologous to said virus.

4. A virus bearing on its outer surface a chimeric binding protein according to claim 3, said binding domain being capable of binding to a target material to which the native outer surface (coat) protein does not preferentially bind, said binding domain being foreign to the native coat proteins of said virus.

5. The method of claim 1 or 2, or the virus of claim 3 or 4, wherein the amplifiable genetic package or virus is a bacteriophage.

6. The method or virus of claim 5 wherein the amplifiable genetic package or virus is a filamentous phage.

7. A method of obtaining a nucleic acid encoding a proteinaceous binding domain that binds a predetermined target material, comprising:

(a) preparing a variegated population of amplifiable genetic packages, said genetic packages being filamentous phage, each said phage including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or more identifiable surface residues, and for which both an affinity molecule and an amino acid sequence are either available or obtainable, and (ii) an outer surface transport signal for obtaining the display of the potential binding domain on the outer surface of the phage, expression of which construct results in the display of said chimeric potential binding protein and its potential binding domain on the outer surface of said phage; and wherein said variegated population of filamentous phage collectively display a plurality of different potential binding domains, differentiation among said plurality of different potential binding domains occurring

115

through the at least partially random variation of one or more predetermined amino acid positions of said parental binding domain to randomly obtain at each said position an amino acid belonging to a predetermined set of two or more amino acids, the amino acids of said set occurring at said position in statistically predetermined expected proportions, the genetic message encapsulated by said filamentous phage being amplifiable *in vitro* or by cell culture and separable on the basis of the potential binding domain displayed thereon,

(b) causing the expression of said chimeric potential binding proteins and the display of said potential binding domains on the outer surface of said phage;

(c) contacting said phage with the predetermined target material such that said potential binding domains and the target material may interact;

(d) separating phage displaying a potential binding domain that binds the target material from phage that do not so bind on the basis of their ability to bind with the target material in step (c), and

(e) recovering at least one phage displaying on its outer surface a chimeric binding protein comprising a stable successful binding domain (SBD) which bound said target, said phage comprising nucleic acid encoding said successful binding domain, and amplifying said SBD-encoding nucleic acid *in vivo* or *in vitro*.

8. A method of obtaining a nucleic acid encoding a proteinaceous binding domain that binds a predetermined target which comprises

(a) providing a population of amplifiable genetic packages, said genetic packages being filamentous phage, each said phage including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or more identifiable surface residues, and for which both an affinity molecule and an amino acid sequence are either available or obtainable, and (ii) an outer surface transport signal for obtaining the display of the potential binding domain on the outer surface of the filamentous phage, expression of which construct results in the display of said chimeric potential binding protein and its potential binding domain on the outer surface of said filamentous phage; and wherein said population of filamentous phage collectively display the genetic message encapsulated by said filamentous phage being amplifiable *in vitro* or by cell culture and separable on the basis of the potential binding domain displayed thereon,

(b) causing the expression of said chimeric potential binding proteins and the display of said potential binding domains on the outer surface of said phage;

(c) contacting said phage with the predetermined target material such that said potential binding domains and the target material may interact;

(d) separating phage displaying a potential binding domain that binds the target material from phage that do not so bind on the basis of their ability to bind with the target material in step (c), and

(e) recovering at least one phage displaying on its outer surface a chimeric binding protein comprising a stable successful binding domain (SBD) which bound said target, said phage comprising nucleic acid encoding said successful binding domain, and amplifying said SBD-encoding nucleic acid *in vivo* or *in vitro*.

9. A chimeric protein comprising (i) at least a segment of an outer surface protein of a virus, said virus being a filamentous phage, said segment providing an outer surface transport signal recognized by a cell infected by said filamentous phage such that the chimeric protein is assembled into the coat of filamentous phage particles produced by said cell, and (ii) a stable, proteinaceous binding domain, said domain comprising one or more identifiable surface residues, which is sufficiently stable in structure to have a melting point of at least 40°C, and which domain binds a predetermined target material, the target being bound sufficiently strongly so that the dissociation constant of the binding domain: target complex is less than $10^{-6}$ moles/liter, and that is heterologous to said filamentous phage.

10. A method of obtaining a nucleic acid encoding a proteinaceous binding domain that binds a predetermined target which comprises

(a) providing a population of amplifiable genetic packages, said genetic packages being selected from the group consisting of cells, spores and viruses, each said genetic package being genetically alterable and having an outer surface, said outer surface including a genetically determined outer surface protein, each said geneitc package including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or more identifiable surface residues, and for which both an affinity molecule and an amino acid sequence are either available or obtainable, and (ii) an outer surface transport signal for obtaining the display of the potential binding domain on the outer surface of the genetic package, expression of which construct results in the display of said chimeric potential binding protein and its potential binding domain on the outer surface of said genetic package; and wherein said population of genetic packages collectively display the genetic message encapsulated by said genetic packages being amplifiable *in vitro* or by cell culture of said genetic packages or host cells transformed therewith and separable on the basis of the potential binding domain displayed thereon,

(b) causing the expression of said chimeric potential binding proteins and the display of said potential binding domains on the outer surface of said packages;

(c) contacting said packages with the predetermined target material such that said potential binding domains and the target material may interact;

(d) separating packages displaying a potential binding domain that binds the target material from packages that do not so bind on the basis of their ability to bind with the target material in step (c), and

(e) recovering at least one package displaying on its outer surface a chimeric binding protein comprising a stable successful binding domain (SBD) which bound said target, said package comprising nucleic acid encoding said successful binding domain, and amplifying said SBD-encoding nucleic acid *in vivo* or *in vitro;*

wherein the parental protein is a single chain antibody, and (i) the genetic package is a filamentous phage, and/or (ii) the outer surface of the genetic package presents, not only said chimeric protein, but also cognate wild type outer surface protein.

11. The method, protein or virus of any of claims 1-10, wherein the outer surface transport signal is provided by the gene VIII protein of a filamentous phage, or a segment thereof embodying such a signal.

12. The method, protein or virus of any of claims 1-10, wherein the outer surface transport signal is provided by the gene III protein of a filamentous phage, or a segment thereof embodying such a signal.

13. The method, protein or virus of any of claims 1-12, wherein the outer surface of the genetic package presents, not only said chimeric protein, but also cognate wild type outer surface protein.

14. The method, protein or virus of claim 13 wherein at least one of the genes encoding said chimeric protein and said wild type outer surface protein is under the control of a regulatable promoter, allowing the ratio between chimeric and wild type protein to be controlled.

15. The method, protein or virus of any of claims 1-14 wherein the potential binding domain or proteinaceous binding domain is coupled essentially to the amino terminal of a mature coat protein.

16. The method, protein or virus of any of claims 1-15 wherein the potential binding domain or proteinaceous binding domain is initially chosen to be one which is over 50% homologous with a domain of a known protein, the domain of the known protein having a melting point of at least about 60°C.

17. The method, protein or virus of any of claims 1-16 wherein, when the target is an antibody, the predetermined parental protein or proteinaceous binding domain is not an antigen specifically bound by that antibody.

18. A fusion protein consisting of (a) an amino terminal moiety which is a foreign peptide or protein comprising an amino acid sequence foreign to a mature gene III protein of a filamentous phage, and (b) a carboxy terminal carrier protein moiety which comprises essentially all of said mature gene III protein, said fusion protein optionally including (c) a linker between said amino and carboxy terminal moieties, said carrier protein moiety acting, when the fusion protein is produced in a suitable host cell infected by the phage, to cause the display of the fusion protein or a processed form thereof on the surface of phage particles produced by the cell.

19. A fusion protein comprising (a) at least a functional portion of a mature gene VIII protein of a filamentous phage, said portion acting, when the fusion protein is produced in a suitable host cell infected by the phage, to cause display of the fusion protein or a processed form thereof on the surface of the phage, and (b) a foreign peptide or protein coupled directly or indirectly to said functional portion of said mature gene VIII protein.

20. The fusion protein of claim 18 or 19 where the complex of the foreign peptide or protein with a target other than an antibody has a dissociation constant less than or equal to $10^{-6}$ moles/liter.

**21.** The fusion protein of any of claims 18-20 wherein the foreign peptide or protein is not found naturally in untransformed *E. coli.*

**22.** The fusion protein of any of claims 18-20 wherein the foreign peptide or protein is not found naturally in untransformed bacteria.

**23.** A fusion protein consisting of (a) a foreign peptide or protein moiety comprising an amino acid sequence foreign to a mature gene III protein of a filamentous phage, and (b) a carrier protein moiety which is a functional portion of said mature gene III protein, said fusion protein optionally including (c) a linker foreign to said gene III protein between said amino and carboxy terminal moieties, said carrier protein corresponding to a carboxy portion of mature gene III protein obtainable by truncating the gene III protein at a domain boundary, where the complex of the foreign peptide or protein with a target other than an antibody has a dissociation constant less than or equal to $10^{-6}$ moles/liter.

**24.** A method of separating a predetermined target material from a predetermined contaminating material which comprises

(a) obtaining a nucleic acid encoding a binding protein having a proteinaceous binding domain that binds to said predetermined target material substantially more strongly than to said predetermined contaminating material, by the method of claim 1;
(b) producing a binding protein which is either said chimeric binding protein or a secondary binding protein having essentially the same binding domain;
(c) immobilizing or labeling said binding protein of (b), so as to form an affinity separation reagent; and
(d) contacting a mixture comprising said target material and said contaminating material with said reagent such that said target material and said reagent can interact, and separating said target material from said contaminating material on the basis of their affinity for said affinity separation reagent.

**25.** A method of preparing a population of variegated DNA encoding a potential binding domain which is a mutant of a stable predetermined domain of a predetermined parental protein, other than a single chain antibody, comprising one or more identifiable surface residues, and for which both an affinity molecule and an amino acid sequence are either available or obtainable, wherein, for at least one codon, a desired mix of amino acids is obtained by use of a nonequimolar mixture of nucleotides in synthesizing at least one base position of that codon, the distribution of nucleotides incorporated at said variegated codon being chosen to yield (a) a more equal abundance of acidic and basic amino acids, and/or (b) a lower ratio of abundance of most-favored amino acid to least-favored amino acid, relative to that yielded by use of an equimolar ratio of the same nucleotides at said base positions.

**26.** Variegated DNA prepared by the method of claim 25, and suitable for use in the method of claim 1.

**27.** A method of obtaining a nucleic acid encoding a proteinaceous binding domain that binds a predetermined target which comprises

(a) providing a population of amplifiable genetic packages, said genetic packages being selected from the group consisting of cells, spores and viruses, each said genetic package being genetically alterable and having an outer surface, said outer surface including a genetically determined outer surface protein, each said genetic package including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or more identifiable surface residues, and for which both an affinity molecule and an amino acid sequence are either available or obtainable, and (ii) an outer surface transport signal for obtaining the display of the potential binding domain on the outer surface of the genetic package, expression of which construct results in the display of said chimeric potential binding protein and its potential binding domain on the outer surface of said genetic package; and wherein said population of genetic packages collectively display the genetic message encapsulated by said genetic packages being amplifiable *in vitro* or by cell culture of said genetic packages or host cells transformed therewith and separable on the basis of the potential binding domain displayed thereon,
(b) causing the expression of said chimeric potential binding proteins and the display of said potential binding domains on the outer surface of said packages;
(c) contacting said packages with the predetermined target material such that said potential binding domains and the target material may interact;

(d) separating packages displaying a potential binding domain that binds the target material from packages that do not so bind on the basis of their ability to bind with the target material in step (c), and

(e) recovering at least one package displaying on its outer surface a chimeric binding protein comprising a stable successful binding domain (SBD) which bound said target, said package comprising nucleic acid encoding said successful binding domain, and amplifying said SBD-encoding nucleic acid *in vivo* or *in vitro*;

wherein the target is the antigen combining site of an antibody.

28. A method of obtaining a nucleic acid encoding a proteinaceous binding domain that binds a predetermined target which comprises

(a) providing a population of amplifiable genetic packages, said genetic packages being selected from the group consisting of cells, spores and viruses, each said genetic package being genetically alterable and having an outer surface, said outer surface including a genetically determined outer surface protein, each said genetic package including a first nucleic acid construct coding for a chimeric potential binding protein, each said chimeric protein comprising and each said construct comprising DNA encoding (i) a potential binding domain which is a mutant of a stable predetermined domain of a predetermined parental protein, comprising one or more identifiable surface residues, and for which both an affinity molecule and an amino acid sequence are either available or obtainable, and (ii) an outer surface transport signal for obtaining the display of the potential binding domain on the outer surface of the genetic package, expression of which construct results in the display of said chimeric potential binding protein and its potential binding domain on the outer surface of said genetic package; and wherein said population of genetic packages collectively display the genetic message encapsulated by said genetic packages being amplifiable *in vitro* or by cell culture of said genetic packages or host cells transformed therewith and separable on the basis of the potential binding domain displayed thereon,

(b) causing the expression of said chimeric potential binding proteins and the display of said potential binding domains on the outer surface of said packages;

(c) contacting said packages with the predetermined target material such that said potential binding domains and the target material may interact;

(d) separating packages displaying a potential binding domain that binds the target material from packages that do not so bind on the basis of their ability to bind with the target material in step (c), and

(e) recovering at least one package displaying on its outer surface a chimeric binding protein comprising a stable successful binding domain (SBD) which bound said target, said package comprising nucleic acid encoding said successful binding domain, and amplifying said SBD-encoding nucleic acid *in vivo* or *in vitro*;

wherein the potential binding domains differ at one or more, but not all, amino acid positions, and wherein the positions of variation are not synthetically predetermined but are known or determinable.

29. A chimeric protein comprising (i) at least a segment of an outer surface protein of a virus, said virus being a filamentous phage, said segment providing an outer surface transport signal recognized by a cell infected by said filamentous phage such that the chimeric protein is assembled into the coat of filamentous phage particles produced by said cell, and (ii) a stable, proteinaceous binding domain which is a single chain antibody, said domain comprising one or more identifiable surface residues, which is sufficiently stable in structure to have a melting point of at least 40°C, and which domain binds a predetermined target material, the target being bound sufficiently strongly so that the dissociation constant of the binding domain: target complex is less than $10^{-6}$ moles/liter, and that is heterologous to said filamentous phage.

30. A chimeric protein comprising (i) at least a segment of an outer surface protein of a virus, said virus being a filamentous phage, said segment providing an outer surface transport signal recognized by a cell infected by said filamentous phage such that the chimeric protein is assembled into the coat of filamentous phage particles produced by said cell, and (ii) a stable, proteinaceous binding domain, said domain comprising one or more identifiable surface residues, which is sufficiently stable in structure to have a melting point of at least 40°C, and which domain binds a predetermined target material which is the antigen combining site of an antibody, the target being bound sufficiently strongly so that the dissociation constant of the binding domain: target complex is less than $10^{-6}$ moles/liter, and that is heterologous to said filamentous phage.

31. A chimeric protein comprising (i) at least a segment of an outer surface protein of a virus which is not a filamentous phage, said segment providing an outer surface transport signal recognized by a cell infected by said virus such that the chimeric protein is assembled into the coat of the virus particles produced by said cell, and (ii) a stable, proteinaceous binding domain, other than a single chain antibody, said domain comprising one or more identifiable surface residues, which is sufficiently stable in structure to have a melting point of at least 40°C, and which domain binds a predetermined target material, the target being bound sufficiently strongly so that the dissociation constant of the binding domain: target complex is less than $10^{-6}$ moles/liter, and that is heterologous to said virus.

32. A chimeric protein comprising (i) at least a segment of an outer surface protein of a virus, said segment providing an outer surface transport signal recognized by a cell infected by said virus such that the chimeric protein is assembled into the coat of the virus particles produced by said cell, and (ii) a stable, proteinaceous binding domain, other than a single chain antibody, said domain comprising one or more identifiable surface residues, which is sufficiently stable in structure to have a melting point of at least 40°C, and which domain binds a predetermined target material which is the antigen combining site of an antibody, the target being bound sufficiently strongly so that the dissociation constant of the binding domain: target complex is less than $10^{-6}$ moles/liter, and that is heterologous to said virus.

33. The protein of claim 3 or the virus of claim 4, wherein the outer surface of the virus presents, not only said chimeric protein, but also cognate wild type outer surface protein.

34. The protein of claim 3 or the virus of claim 4, wherein at least one of the genes encoding said chimeric protein and said wild type outer surface protein is under the control of a regulatable promoter, allowing the ratio between chimeric and wild type protein to be controlled.

INITIAL POTENTIAL BINDING DOMAIN   IPBD    SINGLE SEQUENCE

| HUMAN CHOICE

PARENTAL POTENTIAL BINDING       PPBD      SINGLE SEQUENCE
DOMAIN

VARIEGATION

$10^6 - 10^9$ SEQUENCES

POTENTIAL
BINDING          PBD1, PBD2,......PBD12073625
DOMAINS

SELECTION

SUCCESSFUL       SBD1, SBD2,...SBD30
BINDING DOMAINS

ANALYSIS

PARENTAL POTENTIAL                          PARENT TO NEXT
BINDING DOMAIN           PPBD               VARIEGATION CYCLE

## FIG. 1.

CHOOSE GENETIC PACKAGE,
OUTER SURFACE PROTEIN,
OCV AND IPBD

SET PPBD = IPBD

CHOOSE RESIDUES TO VARY

SYNTHESIZE VG DNA, CLONE INTO OCV, &
INTRODUCE INTO wt GP TO OBTAIN GP (pbd)

CAUSE GPs TO EXPRESS AND DISPLAY PBDs

USE AFFINITY SEPARATION TO ISOLATE
GP(SBD)s FROM OTHER GP (PBD)s

ENRICHMENT
CYCLE

YES

FUTHER ENRICHMENT NEEDED

NO

RECOVER AND AMPLIFY GP (SBD)s

CHARACTERIZE ISOLATED PBDs

YES — BINDING GOOD ENOUGH

NO

SET PPBD = SBD
OR, IF NO SBD,
SET PPBD = IPBD

VARIEGATION CYCLE

DONE

# FIG. 2.

FIG. 3.

EP 1 026 240 A2

FIG. 4.

FIG. 5a.

FIG.5b.

FIG. 5C.

FIG. 5d.